# EUROPEAN PATENT APPLICATION

(11) **EP 3 785 726 A1**
(43) Date of publication of application: **03.03.2021**
(21) Application number: 19194964.3
(22) Date of filing: 02.09.2019
(51) Int. Cl.: A61K 38/37

(54) **FACTOR VIII PROTEIN WITH INCREASED HALF-LIFE**

(71) Applicant: Biotest AG, 63303 Dreieich (DE)
(72) Inventor: Kistner, Steffen, 60599 Frankfurt/Main (DE); Schüttrumpf, Jörg, 63303 Dreieich (DE); Daufenbach, Jens, 55120, Mainz (DE); Herbener, Peter, 35274 Kirchhain (DE); Ungerer, Christopher, 63225 Langen (DE)
(74) Representative: Moré, Solveig Helga

(57) **Abstract**

The present invention relates to recombinant coagulation factors, in particular, recombinant Factor VIII (FVIII) proteins having an increased half-life. They comprise a heavy chain portion and a light chain portion of Factor VIII and at least two albumin binding domains, wherein at least one albumin binding domain is C-terminal to the heavy chain portion and at least one albumin binding domain is C-terminal to the light chain portion. If the protein is a single chain protein, the albumin binding domain(s) C-terminal to the heavy chain portion is/are N-terminal to the light chain portion. The invention also relates to nucleic acids encoding the proteins of the invention, methods of producing them and pharmaceutical compositions comprising any of these, wherein the pharmaceutical composition preferably is for use in treatment of hemophilia A.

SEQUENCE LISTING

<110> Biotest AG

<120> Factor VIII protein with increased half-life

<130> 11851 P 6689 EP

<160> 59

<170> BiSSAP 1.3.6

<210> 1
<211> 2351
<212> PRT
<213> Homo sapiens

<220>
<221> SIGNAL
<222> 1..19
<223> signal

<220>
<223> wt human FVIII

<400> 1
Met Gln Ile Glu Leu Ser Thr Cys Phe Phe Leu Cys Leu Leu Arg Phe
1               5                   10                  15
Cys Phe Ser Ala Thr Arg Arg Tyr Tyr Leu Gly Ala Val Glu Leu Ser
            20                  25                  30
Trp Asp Tyr Met Gln Ser Asp Leu Gly Glu Leu Pro Val Asp Ala Arg
        35                  40                  45
Phe Pro Pro Arg Val Pro Lys Ser Phe Pro Phe Asn Thr Ser Val Val
    50                  55                  60
Tyr Lys Lys Thr Leu Phe Val Glu Phe Thr Asp His Leu Phe Asn Ile
65                  70                  75                  80
Ala Lys Pro Arg Pro Pro Trp Met Gly Leu Leu Gly Pro Thr Ile Gln
                85                  90                  95
Ala Glu Val Tyr Asp Thr Val Val Ile Thr Leu Lys Asn Met Ala Ser
            100                 105                 110
His Pro Val Ser Leu His Ala Val Gly Val Ser Tyr Trp Lys Ala Ser
        115                 120                 125
Glu Gly Ala Glu Tyr Asp Asp Gln Thr Ser Gln Arg Glu Lys Glu Asp
    130                 135                 140
Asp Lys Val Phe Pro Gly Gly Ser His Thr Tyr Val Trp Gln Val Leu
145                 150                 155                 160
Lys Glu Asn Gly Pro Met Ala Ser Asp Pro Leu Cys Leu Thr Tyr Ser
                165                 170                 175
Tyr Leu Ser His Val Asp Leu Val Lys Asp Leu Asn Ser Gly Leu Ile
            180                 185                 190
Gly Ala Leu Leu Val Cys Arg Glu Gly Ser Leu Ala Lys Glu Lys Thr
        195                 200                 205
Gln Thr Leu His Lys Phe Ile Leu Leu Phe Ala Val Phe Asp Glu Gly
    210                 215                 220
Lys Ser Trp His Ser Glu Thr Lys Asn Ser Leu Met Gln Asp Arg Asp
225                 230                 235                 240
Ala Ala Ser Ala Arg Ala Trp Pro Lys Met His Thr Val Asn Gly Tyr
                245                 250                 255
Val Asn Arg Ser Leu Pro Gly Leu Ile Gly Cys His Arg Lys Ser Val
            260                 265                 270
Tyr Trp His Val Ile Gly Met Gly Thr Thr Pro Glu Val His Ser Ile
        275                 280                 285

Phe Leu Glu Gly His Thr Phe Leu Val Arg Asn His Arg Gln Ala Ser
    290                 295                 300
Leu Glu Ile Ser Pro Ile Thr Phe Leu Thr Ala Gln Thr Leu Leu Met
305                 310                 315                 320
Asp Leu Gly Gln Phe Leu Leu Phe Cys His Ile Ser Ser His Gln His
                325                 330                 335
Asp Gly Met Glu Ala Tyr Val Lys Val Asp Ser Cys Pro Glu Glu Pro
            340                 345                 350
Gln Leu Arg Met Lys Asn Asn Glu Glu Ala Glu Asp Tyr Asp Asp Asp
        355                 360                 365
Leu Thr Asp Ser Glu Met Asp Val Val Arg Phe Asp Asp Asp Asn Ser
    370                 375                 380
Pro Ser Phe Ile Gln Ile Arg Ser Val Ala Lys Lys His Pro Lys Thr
385                 390                 395                 400
Trp Val His Tyr Ile Ala Ala Glu Glu Glu Asp Trp Asp Tyr Ala Pro
                405                 410                 415
Leu Val Leu Ala Pro Asp Asp Arg Ser Tyr Lys Ser Gln Tyr Leu Asn
            420                 425                 430
Asn Gly Pro Gln Arg Ile Gly Arg Lys Tyr Lys Lys Val Arg Phe Met
        435                 440                 445
Ala Tyr Thr Asp Glu Thr Phe Lys Thr Arg Glu Ala Ile Gln His Glu
    450                 455                 460
Ser Gly Ile Leu Gly Pro Leu Leu Tyr Gly Glu Val Gly Asp Thr Leu
465                 470                 475                 480
Leu Ile Ile Phe Lys Asn Gln Ala Ser Arg Pro Tyr Asn Ile Tyr Pro
                485                 490                 495
His Gly Ile Thr Asp Val Arg Pro Leu Tyr Ser Arg Arg Leu Pro Lys
            500                 505                 510
Gly Val Lys His Leu Lys Asp Phe Pro Ile Leu Pro Gly Glu Ile Phe
        515                 520                 525
Lys Tyr Lys Trp Thr Val Thr Val Glu Asp Gly Pro Thr Lys Ser Asp
    530                 535                 540
Pro Arg Cys Leu Thr Arg Tyr Tyr Ser Ser Phe Val Asn Met Glu Arg
545                 550                 555                 560
Asp Leu Ala Ser Gly Leu Ile Gly Pro Leu Leu Ile Cys Tyr Lys Glu
                565                 570                 575
Ser Val Asp Gln Arg Gly Asn Gln Ile Met Ser Asp Lys Arg Asn Val
            580                 585                 590
Ile Leu Phe Ser Val Phe Asp Glu Asn Arg Ser Trp Tyr Leu Thr Glu
        595                 600                 605
Asn Ile Gln Arg Phe Leu Pro Asn Pro Ala Gly Val Gln Leu Glu Asp
    610                 615                 620
Pro Glu Phe Gln Ala Ser Asn Ile Met His Ser Ile Asn Gly Tyr Val
625                 630                 635                 640
Phe Asp Ser Leu Gln Leu Ser Val Cys Leu His Glu Val Ala Tyr Trp
                645                 650                 655
Tyr Ile Leu Ser Ile Gly Ala Gln Thr Asp Phe Leu Ser Val Phe Phe
            660                 665                 670
Ser Gly Tyr Thr Phe Lys His Lys Met Val Tyr Glu Asp Thr Leu Thr
        675                 680                 685
Leu Phe Pro Phe Ser Gly Glu Thr Val Phe Met Ser Met Glu Asn Pro
    690                 695                 700
Gly Leu Trp Ile Leu Gly Cys His Asn Ser Asp Phe Arg Asn Arg Gly
705                 710                 715                 720
Met Thr Ala Leu Leu Lys Val Ser Ser Cys Asp Lys Asn Thr Gly Asp
                725                 730                 735
Tyr Tyr Glu Asp Ser Tyr Glu Asp Ile Ser Ala Tyr Leu Leu Ser Lys
            740                 745                 750
Asn Asn Ala Ile Glu Pro Arg Ser Phe Ser Gln Asn Ser Arg His Pro
        755                 760                 765
Ser Thr Arg Gln Lys Gln Phe Asn Ala Thr Thr Ile Pro Glu Asn Asp
    770                 775                 780
Ile Glu Lys Thr Asp Pro Trp Phe Ala His Arg Thr Pro Met Pro Lys

785                 790                 795                 800
Ile Gln Asn Val Ser Ser Ser Asp Leu Leu Met Leu Leu Arg Gln Ser
                805                 810                 815
Pro Thr Pro His Gly Leu Ser Leu Ser Asp Leu Gln Glu Ala Lys Tyr
            820                 825                 830
Glu Thr Phe Ser Asp Asp Pro Ser Pro Gly Ala Ile Asp Ser Asn Asn
        835                 840                 845
Ser Leu Ser Glu Met Thr His Phe Arg Pro Gln Leu His His Ser Gly
    850                 855                 860
Asp Met Val Phe Thr Pro Glu Ser Gly Leu Gln Leu Arg Leu Asn Glu
865                 870                 875                 880
Lys Leu Gly Thr Thr Ala Ala Thr Glu Leu Lys Lys Leu Asp Phe Lys
                885                 890                 895
Val Ser Ser Thr Ser Asn Asn Leu Ile Ser Thr Ile Pro Ser Asp Asn
            900                 905                 910
Leu Ala Ala Gly Thr Asp Asn Thr Ser Ser Leu Gly Pro Pro Ser Met
        915                 920                 925
Pro Val His Tyr Asp Ser Gln Leu Asp Thr Thr Leu Phe Gly Lys Lys
    930                 935                 940
Ser Ser Pro Leu Thr Glu Ser Gly Gly Pro Leu Ser Leu Ser Glu Glu
945                 950                 955                 960
Asn Asn Asp Ser Lys Leu Leu Glu Ser Gly Leu Met Asn Ser Gln Glu
                965                 970                 975
Ser Ser Trp Gly Lys Asn Val Ser Ser Thr Glu Ser Gly Arg Leu Phe
            980                 985                 990
Lys Gly Lys Arg Ala His Gly Pro Ala Leu Leu Thr Lys Asp Asn Ala
        995                 1000                1005
Leu Phe Lys Val Ser Ile Ser Leu Leu Lys Thr Asn Lys Thr Ser Asn
    1010                1015                1020
Asn Ser Ala Thr Asn Arg Lys Thr His Ile Asp Gly Pro Ser Leu Leu
1025                1030                1035                1040
Ile Glu Asn Ser Pro Ser Val Trp Gln Asn Ile Leu Glu Ser Asp Thr
                1045                1050                1055
Glu Phe Lys Lys Val Thr Pro Leu Ile His Asp Arg Met Leu Met Asp
            1060                1065                1070
Lys Asn Ala Thr Ala Leu Arg Leu Asn His Met Ser Asn Lys Thr Thr
        1075                1080                1085
Ser Ser Lys Asn Met Glu Met Val Gln Gln Lys Lys Glu Gly Pro Ile
    1090                1095                1100
Pro Pro Asp Ala Gln Asn Pro Asp Met Ser Phe Phe Lys Met Leu Phe
1105                1110                1115                1120
Leu Pro Glu Ser Ala Arg Trp Ile Gln Arg Thr His Gly Lys Asn Ser
                1125                1130                1135
Leu Asn Ser Gly Gln Gly Pro Ser Pro Lys Gln Leu Val Ser Leu Gly
            1140                1145                1150
Pro Glu Lys Ser Val Glu Gly Gln Asn Phe Leu Ser Glu Lys Asn Lys
        1155                1160                1165
Val Val Val Gly Lys Gly Glu Phe Thr Lys Asp Val Gly Leu Lys Glu
    1170                1175                1180
Met Val Phe Pro Ser Ser Arg Asn Leu Phe Leu Thr Asn Leu Asp Asn
1185                1190                1195                1200
Leu His Glu Asn Asn Thr His Asn Gln Glu Lys Lys Ile Gln Glu Glu
                1205                1210                1215
Ile Glu Lys Lys Glu Thr Leu Ile Gln Glu Asn Val Val Leu Pro Gln
            1220                1225                1230
Ile His Thr Val Thr Gly Thr Lys Asn Phe Met Lys Asn Leu Phe Leu
        1235                1240                1245
Leu Ser Thr Arg Gln Asn Val Glu Gly Ser Tyr Asp Gly Ala Tyr Ala
    1250                1255                1260
Pro Val Leu Gln Asp Phe Arg Ser Leu Asn Asp Ser Thr Asn Arg Thr
1265                1270                1275                1280
Lys Lys His Thr Ala His Phe Ser Lys Lys Gly Glu Glu Glu Asn Leu
                1285                1290                1295

Glu Gly Leu Gly Asn Gln Thr Lys Gln Ile Val Glu Lys Tyr Ala Cys
            1300                1305                1310
Thr Thr Arg Ile Ser Pro Asn Thr Ser Gln Gln Asn Phe Val Thr Gln
        1315                1320                1325
Arg Ser Lys Arg Ala Leu Lys Gln Phe Arg Leu Pro Leu Glu Glu Thr
    1330                1335                1340
Glu Leu Glu Lys Arg Ile Ile Val Asp Asp Thr Ser Thr Gln Trp Ser
1345                1350                1355                1360
Lys Asn Met Lys His Leu Thr Pro Ser Thr Leu Thr Gln Ile Asp Tyr
                1365                1370                1375
Asn Glu Lys Glu Lys Gly Ala Ile Thr Gln Ser Pro Leu Ser Asp Cys
            1380                1385                1390
Leu Thr Arg Ser His Ser Ile Pro Gln Ala Asn Arg Ser Pro Leu Pro
        1395                1400                1405
Ile Ala Lys Val Ser Ser Phe Pro Ser Ile Arg Pro Ile Tyr Leu Thr
    1410                1415                1420
Arg Val Leu Phe Gln Asp Asn Ser Ser His Leu Pro Ala Ala Ser Tyr
1425                1430                1435                1440
Arg Lys Lys Asp Ser Gly Val Gln Glu Ser Ser His Phe Leu Gln Gly
                1445                1450                1455
Ala Lys Lys Asn Asn Leu Ser Leu Ala Ile Leu Thr Leu Glu Met Thr
            1460                1465                1470
Gly Asp Gln Arg Glu Val Gly Ser Leu Gly Thr Ser Ala Thr Asn Ser
        1475                1480                1485
Val Thr Tyr Lys Lys Val Glu Asn Thr Val Leu Pro Lys Pro Asp Leu
    1490                1495                1500
Pro Lys Thr Ser Gly Lys Val Glu Leu Leu Pro Lys Val His Ile Tyr
1505                1510                1515                1520
Gln Lys Asp Leu Phe Pro Thr Glu Thr Ser Asn Gly Ser Pro Gly His
                1525                1530                1535
Leu Asp Leu Val Glu Gly Ser Leu Leu Gln Gly Thr Glu Gly Ala Ile
            1540                1545                1550
Lys Trp Asn Glu Ala Asn Arg Pro Gly Lys Val Pro Phe Leu Arg Val
        1555                1560                1565
Ala Thr Glu Ser Ser Ala Lys Thr Pro Ser Lys Leu Leu Asp Pro Leu
    1570                1575                1580
Ala Trp Asp Asn His Tyr Gly Thr Gln Ile Pro Lys Glu Glu Trp Lys
1585                1590                1595                1600
Ser Gln Glu Lys Ser Pro Glu Lys Thr Ala Phe Lys Lys Lys Asp Thr
                1605                1610                1615
Ile Leu Ser Leu Asn Ala Cys Glu Ser Asn His Ala Ile Ala Ala Ile
            1620                1625                1630
Asn Glu Gly Gln Asn Lys Pro Glu Ile Glu Val Thr Trp Ala Lys Gln
        1635                1640                1645
Gly Arg Thr Glu Arg Leu Cys Ser Gln Asn Pro Pro Val Leu Lys Arg
    1650                1655                1660
His Gln Arg Glu Ile Thr Arg Thr Thr Leu Gln Ser Asp Gln Glu Glu
1665                1670                1675                1680
Ile Asp Tyr Asp Asp Thr Ile Ser Val Glu Met Lys Lys Glu Asp Phe
                1685                1690                1695
Asp Ile Tyr Asp Glu Asp Glu Asn Gln Ser Pro Arg Ser Phe Gln Lys
            1700                1705                1710
Lys Thr Arg His Tyr Phe Ile Ala Ala Val Glu Arg Leu Trp Asp Tyr
        1715                1720                1725
Gly Met Ser Ser Ser Pro His Val Leu Arg Asn Arg Ala Gln Ser Gly
    1730                1735                1740
Ser Val Pro Gln Phe Lys Lys Val Val Phe Gln Glu Phe Thr Asp Gly
1745                1750                1755                1760
Ser Phe Thr Gln Pro Leu Tyr Arg Gly Glu Leu Asn Glu His Leu Gly
                1765                1770                1775
Leu Leu Gly Pro Tyr Ile Arg Ala Glu Val Glu Asp Asn Ile Met Val
            1780                1785                1790
Thr Phe Arg Asn Gln Ala Ser Arg Pro Tyr Ser Phe Tyr Ser Ser Leu

1795                1800                1805
Ile Ser Tyr Glu Glu Asp Gln Arg Gln Gly Ala Glu Pro Arg Lys Asn
    1810                1815                1820
Phe Val Lys Pro Asn Glu Thr Lys Thr Tyr Phe Trp Lys Val Gln His
1825                1830                1835                1840
His Met Ala Pro Thr Lys Asp Glu Phe Asp Cys Lys Ala Trp Ala Tyr
                1845                1850                1855
Phe Ser Asp Val Asp Leu Glu Lys Asp Val His Ser Gly Leu Ile Gly
            1860                1865                1870
Pro Leu Leu Val Cys His Thr Asn Thr Leu Asn Pro Ala His Gly Arg
        1875                1880                1885
Gln Val Thr Val Gln Glu Phe Ala Leu Phe Phe Thr Ile Phe Asp Glu
    1890                1895                1900
Thr Lys Ser Trp Tyr Phe Thr Glu Asn Met Glu Arg Asn Cys Arg Ala
1905                1910                1915                1920
Pro Cys Asn Ile Gln Met Glu Asp Pro Thr Phe Lys Glu Asn Tyr Arg
                1925                1930                1935
Phe His Ala Ile Asn Gly Tyr Ile Met Asp Thr Leu Pro Gly Leu Val
            1940                1945                1950
Met Ala Gln Asp Gln Arg Ile Arg Trp Tyr Leu Leu Ser Met Gly Ser
        1955                1960                1965
Asn Glu Asn Ile His Ser Ile His Phe Ser Gly His Val Phe Thr Val
    1970                1975                1980
Arg Lys Lys Glu Glu Tyr Lys Met Ala Leu Tyr Asn Leu Tyr Pro Gly
1985                1990                1995                2000
Val Phe Glu Thr Val Glu Met Leu Pro Ser Lys Ala Gly Ile Trp Arg
                2005                2010                2015
Val Glu Cys Leu Ile Gly Glu His Leu His Ala Gly Met Ser Thr Leu
            2020                2025                2030
Phe Leu Val Tyr Ser Asn Lys Cys Gln Thr Pro Leu Gly Met Ala Ser
        2035                2040                2045
Gly His Ile Arg Asp Phe Gln Ile Thr Ala Ser Gly Gln Tyr Gly Gln
    2050                2055                2060
Trp Ala Pro Lys Leu Ala Arg Leu His Tyr Ser Gly Ser Ile Asn Ala
2065                2070                2075                2080
Trp Ser Thr Lys Glu Pro Phe Ser Trp Ile Lys Val Asp Leu Leu Ala
                2085                2090                2095
Pro Met Ile Ile His Gly Ile Lys Thr Gln Gly Ala Arg Gln Lys Phe
            2100                2105                2110
Ser Ser Leu Tyr Ile Ser Gln Phe Ile Ile Met Tyr Ser Leu Asp Gly
        2115                2120                2125
Lys Lys Trp Gln Thr Tyr Arg Gly Asn Ser Thr Gly Thr Leu Met Val
    2130                2135                2140
Phe Phe Gly Asn Val Asp Ser Ser Gly Ile Lys His Asn Ile Phe Asn
2145                2150                2155                2160
Pro Pro Ile Ile Ala Arg Tyr Ile Arg Leu His Pro Thr His Tyr Ser
                2165                2170                2175
Ile Arg Ser Thr Leu Arg Met Glu Leu Met Gly Cys Asp Leu Asn Ser
            2180                2185                2190
Cys Ser Met Pro Leu Gly Met Glu Ser Lys Ala Ile Ser Asp Ala Gln
        2195                2200                2205
Ile Thr Ala Ser Ser Tyr Phe Thr Asn Met Phe Ala Thr Trp Ser Pro
    2210                2215                2220
Ser Lys Ala Arg Leu His Leu Gln Gly Arg Ser Asn Ala Trp Arg Pro
2225                2230                2235                2240
Gln Val Asn Asn Pro Lys Glu Trp Leu Gln Val Asp Phe Gln Lys Thr
                2245                2250                2255
Met Lys Val Thr Gly Val Thr Thr Gln Gly Val Lys Ser Leu Leu Thr
            2260                2265                2270
Ser Met Tyr Val Lys Glu Phe Leu Ile Ser Ser Ser Gln Asp Gly His
        2275                2280                2285
Gln Trp Thr Leu Phe Phe Gln Asn Gly Lys Val Lys Val Phe Gln Gly
    2290                2295                2300

Asn Gln Asp Ser Phe Thr Pro Val Val Asn Ser Leu Asp Pro Pro Leu
2305                2310                2315                2320
Leu Thr Arg Tyr Leu Arg Ile His Pro Gln Ser Trp Val His Gln Ile
                2325                2330                2335
Ala Leu Arg Met Glu Val Leu Gly Cys Glu Ala Gln Asp Leu Tyr
            2340                2345                2350

<210> 2
<211> 9
<212> PRT
<213> Artificial Sequence

<220>
<223> minimal processing sequence

<400> 2
Pro Arg Ser Phe Ser Gln Asn Pro Pro
1               5

<210> 3
<400> 3
000

<210> 4
<211> 11
<212> PRT
<213> Artificial Sequence

<220>
<223> Processing sequence

<400> 4
Pro Arg Ser Phe Ser Gln Asn Pro Pro Val Leu
1               5                   10

<210> 5
<211> 11
<212> PRT
<213> Artificial Sequence

<220>
<223> Processing sequence

<220>
<221> VARIANT
<222> 4
<223> can be varied for de-immunization

<400> 5
Pro Arg Ser Xaa Ser Gln Asn Pro Pro Val Leu
1               5                   10

<210> 6
<211> 11
<212> PRT
<213> Artificial Sequence

<220>
<223> Processing sequence

<220>
<221> VARIANT
<222> 5
<223> can be varied for de-immunization

<400> 6
Pro Arg Ser Phe Xaa Gln Asn Pro Pro Val Leu
1               5                   10

<210> 7
<211> 11
<212> PRT
<213> Artificial Sequence


<220>
<223> Processing sequence

<220>
<221> VARIANT
<222> 6
<223> can be varied for de-immunization

<400> 7
Pro Arg Ser Phe Ser Xaa Asn Pro Pro Val Leu
1               5                   10

<210> 8
<211> 11
<212> PRT
<213> Artificial Sequence


<220>
<223> Processing sequence

<220>
<221> VARIANT
<222> 7
<223> can be varied for de-immunization

<400> 8
Pro Arg Ser Phe Ser Gln Xaa Pro Pro Val Leu
1               5                   10

<210> 9
<211> 10
<212> PRT
<213> Artificial Sequence


<220>
<223> Merging sequence, e.g., in V0

<400> 9
Gln Ser Asp Gln Glu Glu Ile Asp Tyr Asp
1               5                   10

<210> 10
<400> 10
000

<210> 11
<400> 11
000

<210> 12
<400> 12
000

<210> 13
<400> 13
000

<210> 14
<400> 14
000

<210> 15
<400> 15
000

<210> 16
<211> 1445
<212> PRT
<213> Artificial Sequence

<220>
<221> SIGNAL
<222> 1..19

<220>
<223> V0 aa incl. signal sequence

<400> 16
Met Gln Ile Glu Leu Ser Thr Cys Phe Phe Leu Cys Leu Leu Arg Phe
1               5                   10                  15
Cys Phe Ser Ala Thr Arg Arg Tyr Tyr Leu Gly Ala Val Glu Leu Ser
            20                  25                  30
Trp Asp Tyr Met Gln Ser Asp Leu Gly Glu Leu Pro Val Asp Ala Arg
        35                  40                  45
Phe Pro Pro Arg Val Pro Lys Ser Phe Pro Phe Asn Thr Ser Val Val
    50                  55                  60
Tyr Lys Lys Thr Leu Phe Val Glu Phe Thr Asp His Leu Phe Asn Ile
65                  70                  75                  80
Ala Lys Pro Arg Pro Pro Trp Met Gly Leu Leu Gly Pro Thr Ile Gln
                85                  90                  95
Ala Glu Val Tyr Asp Thr Val Val Ile Thr Leu Lys Asn Met Ala Ser
            100                 105                 110
His Pro Val Ser Leu His Ala Val Gly Val Ser Tyr Trp Lys Ala Ser
        115                 120                 125
Glu Gly Ala Glu Tyr Asp Asp Gln Thr Ser Gln Arg Glu Lys Glu Asp
    130                 135                 140
Asp Lys Val Phe Pro Gly Gly Ser His Thr Tyr Val Trp Gln Val Leu
145                 150                 155                 160
Lys Glu Asn Gly Pro Met Ala Ser Asp Pro Leu Cys Leu Thr Tyr Ser
                165                 170                 175
Tyr Leu Ser His Val Asp Leu Val Lys Asp Leu Asn Ser Gly Leu Ile
            180                 185                 190
Gly Ala Leu Leu Val Cys Arg Glu Gly Ser Leu Ala Lys Glu Lys Thr
        195                 200                 205
Gln Thr Leu His Lys Phe Ile Leu Leu Phe Ala Val Phe Asp Glu Gly
    210                 215                 220
Lys Ser Trp His Ser Glu Thr Lys Asn Ser Leu Met Gln Asp Arg Asp

225                 230                 235                 240
Ala Ala Ser Ala Arg Ala Trp Pro Lys Met His Thr Val Asn Gly Tyr
                245                 250                 255
Val Asn Arg Ser Leu Pro Gly Leu Ile Gly Cys His Arg Lys Ser Val
            260                 265                 270
Tyr Trp His Val Ile Gly Met Gly Thr Thr Pro Glu Val His Ser Ile
        275                 280                 285
Phe Leu Glu Gly His Thr Phe Leu Val Arg Asn His Arg Gln Ala Ser
    290                 295                 300
Leu Glu Ile Ser Pro Ile Thr Phe Leu Thr Ala Gln Thr Leu Leu Met
305                 310                 315                 320
Asp Leu Gly Gln Phe Leu Leu Phe Cys His Ile Ser Ser His Gln His
                325                 330                 335
Asp Gly Met Glu Ala Tyr Val Lys Val Asp Ser Cys Pro Glu Glu Pro
            340                 345                 350
Gln Leu Arg Met Lys Asn Asn Glu Glu Ala Glu Asp Tyr Asp Asp Asp
        355                 360                 365
Leu Thr Asp Ser Glu Met Asp Val Val Arg Phe Asp Asp Asp Asn Ser
    370                 375                 380
Pro Ser Phe Ile Gln Ile Arg Ser Val Ala Lys Lys His Pro Lys Thr
385                 390                 395                 400
Trp Val His Tyr Ile Ala Ala Glu Glu Glu Asp Trp Asp Tyr Ala Pro
                405                 410                 415
Leu Val Leu Ala Pro Asp Asp Arg Ser Tyr Lys Ser Gln Tyr Leu Asn
            420                 425                 430
Asn Gly Pro Gln Arg Ile Gly Arg Lys Tyr Lys Lys Val Arg Phe Met
        435                 440                 445
Ala Tyr Thr Asp Glu Thr Phe Lys Thr Arg Glu Ala Ile Gln His Glu
    450                 455                 460
Ser Gly Ile Leu Gly Pro Leu Leu Tyr Gly Glu Val Gly Asp Thr Leu
465                 470                 475                 480
Leu Ile Ile Phe Lys Asn Gln Ala Ser Arg Pro Tyr Asn Ile Tyr Pro
                485                 490                 495
His Gly Ile Thr Asp Val Arg Pro Leu Tyr Ser Arg Arg Leu Pro Lys
            500                 505                 510
Gly Val Lys His Leu Lys Asp Phe Pro Ile Leu Pro Gly Glu Ile Phe
        515                 520                 525
Lys Tyr Lys Trp Thr Val Thr Val Glu Asp Gly Pro Thr Lys Ser Asp
    530                 535                 540
Pro Arg Cys Leu Thr Arg Tyr Tyr Ser Ser Phe Val Asn Met Glu Arg
545                 550                 555                 560
Asp Leu Ala Ser Gly Leu Ile Gly Pro Leu Leu Ile Cys Tyr Lys Glu
                565                 570                 575
Ser Val Asp Gln Arg Gly Asn Gln Ile Met Ser Asp Lys Arg Asn Val
            580                 585                 590
Ile Leu Phe Ser Val Phe Asp Glu Asn Arg Ser Trp Tyr Leu Thr Glu
        595                 600                 605
Asn Ile Gln Arg Phe Leu Pro Asn Pro Ala Gly Val Gln Leu Glu Asp
    610                 615                 620
Pro Glu Phe Gln Ala Ser Asn Ile Met His Ser Ile Asn Gly Tyr Val
625                 630                 635                 640
Phe Asp Ser Leu Gln Leu Ser Val Cys Leu His Glu Val Ala Tyr Trp
                645                 650                 655
Tyr Ile Leu Ser Ile Gly Ala Gln Thr Asp Phe Leu Ser Val Phe Phe
            660                 665                 670
Ser Gly Tyr Thr Phe Lys His Lys Met Val Tyr Glu Asp Thr Leu Thr
        675                 680                 685
Leu Phe Pro Phe Ser Gly Glu Thr Val Phe Met Ser Met Glu Asn Pro
    690                 695                 700
Gly Leu Trp Ile Leu Gly Cys His Asn Ser Asp Phe Arg Asn Arg Gly
705                 710                 715                 720
Met Thr Ala Leu Leu Lys Val Ser Ser Cys Asp Lys Asn Thr Gly Asp
                725                 730                 735

Tyr Tyr Glu Asp Ser Tyr Glu Asp Ile Ser Ala Tyr Leu Leu Ser Lys
            740                 745                 750
Asn Asn Ala Ile Glu Pro Arg Ser Phe Ser Gln Asn Pro Pro Val Leu
        755                 760                 765
Gln Ser Asp Gln Glu Glu Ile Asp Tyr Asp Asp Thr Ile Ser Val Glu
    770                 775                 780
Met Lys Lys Glu Asp Phe Asp Ile Tyr Asp Glu Asp Glu Asn Gln Ser
785                 790                 795                 800
Pro Arg Ser Phe Gln Lys Lys Thr Arg His Tyr Phe Ile Ala Ala Val
                805                 810                 815
Glu Arg Leu Trp Asp Tyr Gly Met Ser Ser Ser Pro His Val Leu Arg
            820                 825                 830
Asn Arg Ala Gln Ser Gly Ser Val Pro Gln Phe Lys Lys Val Val Phe
        835                 840                 845
Gln Glu Phe Thr Asp Gly Ser Phe Thr Gln Pro Leu Tyr Arg Gly Glu
    850                 855                 860
Leu Asn Glu His Leu Gly Leu Leu Gly Pro Tyr Ile Arg Ala Glu Val
865                 870                 875                 880
Glu Asp Asn Ile Met Val Thr Phe Arg Asn Gln Ala Ser Arg Pro Tyr
                885                 890                 895
Ser Phe Tyr Ser Ser Leu Ile Ser Tyr Glu Glu Asp Gln Arg Gln Gly
            900                 905                 910
Ala Glu Pro Arg Lys Asn Phe Val Lys Pro Asn Glu Thr Lys Thr Tyr
        915                 920                 925
Phe Trp Lys Val Gln His His Met Ala Pro Thr Lys Asp Glu Phe Asp
    930                 935                 940
Cys Lys Ala Trp Ala Tyr Phe Ser Asp Val Asp Leu Glu Lys Asp Val
945                 950                 955                 960
His Ser Gly Leu Ile Gly Pro Leu Leu Val Cys His Thr Asn Thr Leu
                965                 970                 975
Asn Pro Ala His Gly Arg Gln Val Thr Val Gln Glu Phe Ala Leu Phe
            980                 985                 990
Phe Thr Ile Phe Asp Glu Thr Lys Ser Trp Tyr Phe Thr Glu Asn Met
        995                 1000                1005
Glu Arg Asn Cys Arg Ala Pro Cys Asn Ile Gln Met Glu Asp Pro Thr
    1010                1015                1020
Phe Lys Glu Asn Tyr Arg Phe His Ala Ile Asn Gly Tyr Ile Met Asp
1025                1030                1035                1040
Thr Leu Pro Gly Leu Val Met Ala Gln Asp Gln Arg Ile Arg Trp Tyr
                1045                1050                1055
Leu Leu Ser Met Gly Ser Asn Glu Asn Ile His Ser Ile His Phe Ser
            1060                1065                1070
Gly His Val Phe Thr Val Arg Lys Lys Glu Glu Tyr Lys Met Ala Leu
        1075                1080                1085
Tyr Asn Leu Tyr Pro Gly Val Phe Glu Thr Val Glu Met Leu Pro Ser
    1090                1095                1100
Lys Ala Gly Ile Trp Arg Val Glu Cys Leu Ile Gly Glu His Leu His
1105                1110                1115                1120
Ala Gly Met Ser Thr Leu Phe Leu Val Tyr Ser Asn Lys Cys Gln Thr
                1125                1130                1135
Pro Leu Gly Met Ala Ser Gly His Ile Arg Asp Phe Gln Ile Thr Ala
            1140                1145                1150
Ser Gly Gln Tyr Gly Gln Trp Ala Pro Lys Leu Ala Arg Leu His Tyr
        1155                1160                1165
Ser Gly Ser Ile Asn Ala Trp Ser Thr Lys Glu Pro Phe Ser Trp Ile
    1170                1175                1180
Lys Val Asp Leu Leu Ala Pro Met Ile Ile His Gly Ile Lys Thr Gln
1185                1190                1195                1200
Gly Ala Arg Gln Lys Phe Ser Ser Leu Tyr Ile Ser Gln Phe Ile Ile
                1205                1210                1215
Met Tyr Ser Leu Asp Gly Lys Lys Trp Gln Thr Tyr Arg Gly Asn Ser
            1220                1225                1230
Thr Gly Thr Leu Met Val Phe Phe Gly Asn Val Asp Ser Ser Gly Ile

1235                1240                1245
Lys His Asn Ile Phe Asn Pro Pro Ile Ile Ala Arg Tyr Ile Arg Leu
    1250                1255                1260
His Pro Thr His Tyr Ser Ile Arg Ser Thr Leu Arg Met Glu Leu Met
1265                1270                1275                1280
Gly Cys Asp Leu Asn Ser Cys Ser Met Pro Leu Gly Met Glu Ser Lys
                1285                1290                1295
Ala Ile Ser Asp Ala Gln Ile Thr Ala Ser Ser Tyr Phe Thr Asn Met
            1300                1305                1310
Phe Ala Thr Trp Ser Pro Ser Lys Ala Arg Leu His Leu Gln Gly Arg
        1315                1320                1325
Ser Asn Ala Trp Arg Pro Gln Val Asn Asn Pro Lys Glu Trp Leu Gln
    1330                1335                1340
Val Asp Phe Gln Lys Thr Met Lys Val Thr Gly Val Thr Thr Gln Gly
1345                1350                1355                1360
Val Lys Ser Leu Leu Thr Ser Met Tyr Val Lys Glu Phe Leu Ile Ser
                1365                1370                1375
Ser Ser Gln Asp Gly His Gln Trp Thr Leu Phe Phe Gln Asn Gly Lys
            1380                1385                1390
Val Lys Val Phe Gln Gly Asn Gln Asp Ser Phe Thr Pro Val Val Asn
        1395                1400                1405
Ser Leu Asp Pro Pro Leu Leu Thr Arg Tyr Leu Arg Ile His Pro Gln
    1410                1415                1420
Ser Trp Val His Gln Ile Ala Leu Arg Met Glu Val Leu Gly Cys Glu
1425                1430                1435                1440
Ala Gln Asp Leu Tyr
                1445

<210> 17
<400> 17
000

<210> 18
<400> 18
000

<210> 19
<400> 19
000

<210> 20
<400> 20
000

<210> 21
<400> 21
000

<210> 22
<400> 22
000

<210> 23
<400> 23
000

<210> 24
<400> 24
000

<210> 25
<400> 25
000

<210> 26
<400> 26
000

<210> 27
<400> 27
000

<210> 28
<211> 1457
<212> PRT
<213> Artificial Sequence

<220>
<221> SIGNAL
<222> 1..19

<220>
<221> SIGNAL
<222> 1..19

<220>
<223> AC-6rs-REF aa incl. signal

<400> 28
Met Gln Ile Glu Leu Ser Thr Cys Phe Phe Leu Cys Leu Leu Arg Phe
1               5                   10                  15
Cys Phe Ser Ala Thr Arg Arg Tyr Tyr Leu Gly Ala Val Glu Leu Ser
            20                  25                  30
Trp Asp Tyr Met Gln Ser Asp Leu Gly Glu Leu Pro Val Asp Ala Arg
        35                  40                  45
Phe Pro Pro Arg Val Pro Lys Ser Phe Pro Phe Asn Thr Ser Val Val
    50                  55                  60
Tyr Lys Lys Thr Leu Phe Val Glu Phe Thr Asp His Leu Phe Asn Ile
65                  70                  75                  80
Ala Lys Pro Arg Pro Pro Trp Met Gly Leu Leu Gly Pro Thr Ile Gln
                85                  90                  95
Ala Glu Val Tyr Asp Thr Val Val Ile Thr Leu Lys Asn Met Ala Ser
            100                 105                 110
His Pro Val Ser Leu His Ala Val Gly Val Ser Tyr Trp Lys Ala Ser
        115                 120                 125
Glu Gly Ala Glu Tyr Asp Asp Gln Thr Ser Gln Arg Glu Lys Glu Asp
    130                 135                 140
Asp Lys Val Phe Pro Gly Gly Ser His Thr Tyr Val Trp Gln Val Leu
145                 150                 155                 160
Lys Glu Asn Gly Pro Met Ala Ser Asp Pro Leu Cys Leu Thr Tyr Ser
                165                 170                 175
Tyr Leu Ser His Val Asp Leu Val Lys Asp Leu Asn Ser Gly Leu Ile
            180                 185                 190
Gly Ala Leu Leu Val Cys Arg Glu Gly Ser Leu Ala Lys Glu Lys Thr
        195                 200                 205
Gln Thr Leu His Lys Phe Ile Leu Leu Phe Ala Val Phe Asp Glu Gly
    210                 215                 220
Lys Ser Trp His Ser Glu Thr Lys Asn Ser Leu Met Gln Asp Arg Asp
225                 230                 235                 240
Ala Ala Ser Ala Arg Ala Trp Pro Lys Met His Thr Val Asn Gly Tyr
                245                 250                 255
Val Asn Arg Ser Leu Pro Gly Leu Ile Gly Cys His Arg Lys Ser Val
            260                 265                 270
Tyr Trp His Val Ile Gly Met Gly Thr Thr Pro Glu Val His Ser Ile
        275                 280                 285

Phe Leu Glu Gly His Thr Phe Leu Val Arg Asn His Arg Gln Ala Ser
    290                 295                 300
Leu Glu Ile Ser Pro Ile Thr Phe Leu Thr Ala Gln Thr Leu Leu Met
305                 310                 315                 320
Asp Leu Gly Gln Phe Leu Leu Phe Cys His Ile Ser Ser His Gln His
                325                 330                 335
Asp Gly Met Glu Ala Tyr Val Lys Val Asp Ser Cys Pro Glu Glu Pro
            340                 345                 350
Gln Leu Arg Met Lys Asn Asn Glu Glu Ala Glu Asp Tyr Asp Asp Asp
        355                 360                 365
Leu Thr Asp Ser Glu Met Asp Val Val Arg Phe Asp Asp Asp Asn Ser
    370                 375                 380
Pro Ser Phe Ile Gln Ile Arg Ser Val Ala Lys Lys His Pro Lys Thr
385                 390                 395                 400
Trp Val His Tyr Ile Ala Ala Glu Glu Glu Asp Trp Asp Tyr Ala Pro
                405                 410                 415
Leu Val Leu Ala Pro Asp Asp Arg Ser Tyr Lys Ser Gln Tyr Leu Asn
            420                 425                 430
Asn Gly Pro Gln Arg Ile Gly Arg Lys Tyr Lys Lys Val Arg Phe Met
        435                 440                 445
Ala Tyr Thr Asp Glu Thr Phe Lys Thr Arg Glu Ala Ile Gln His Glu
    450                 455                 460
Ser Gly Ile Leu Gly Pro Leu Leu Tyr Gly Glu Val Gly Asp Thr Leu
465                 470                 475                 480
Leu Ile Ile Phe Lys Asn Gln Ala Ser Arg Pro Tyr Asn Ile Tyr Pro
                485                 490                 495
His Gly Ile Thr Asp Val Arg Pro Leu Tyr Ser Arg Arg Leu Pro Lys
            500                 505                 510
Gly Val Lys His Leu Lys Asp Phe Pro Ile Leu Pro Gly Glu Ile Phe
        515                 520                 525
Lys Tyr Lys Trp Thr Val Thr Val Glu Asp Gly Pro Thr Lys Ser Asp
    530                 535                 540
Pro Arg Cys Leu Thr Arg Tyr Tyr Ser Ser Phe Val Asn Met Glu Arg
545                 550                 555                 560
Asp Leu Ala Ser Gly Leu Ile Gly Pro Leu Leu Ile Cys Tyr Lys Glu
                565                 570                 575
Ser Val Asp Gln Arg Gly Asn Gln Ile Met Ser Asp Lys Arg Asn Val
            580                 585                 590
Ile Leu Phe Ser Val Phe Asp Glu Asn Arg Ser Trp Tyr Leu Thr Glu
        595                 600                 605
Asn Ile Gln Arg Phe Leu Pro Asn Pro Ala Gly Val Gln Leu Glu Asp
    610                 615                 620
Pro Glu Phe Gln Ala Ser Asn Ile Met His Ser Ile Asn Gly Tyr Val
625                 630                 635                 640
Phe Asp Ser Leu Gln Leu Ser Val Cys Leu His Glu Val Ala Tyr Trp
                645                 650                 655
Tyr Ile Leu Ser Ile Gly Ala Gln Thr Asp Phe Leu Ser Val Phe Phe
            660                 665                 670
Ser Gly Tyr Thr Phe Lys His Lys Met Val Tyr Glu Asp Thr Leu Thr
        675                 680                 685
Leu Phe Pro Phe Ser Gly Glu Thr Val Phe Met Ser Met Glu Asn Pro
    690                 695                 700
Gly Leu Trp Ile Leu Gly Cys His Asn Ser Asp Phe Arg Asn Arg Gly
705                 710                 715                 720
Met Thr Ala Leu Leu Lys Val Ser Ser Cys Asp Lys Asn Thr Gly Asp
                725                 730                 735
Tyr Tyr Glu Asp Ser Tyr Glu Asp Ile Ser Ala Tyr Leu Leu Ser Lys
            740                 745                 750
Asn Asn Ala Ile Glu Pro Arg Ser Phe Ser Gln Asn Pro Pro Val Leu
        755                 760                 765
Lys Arg His Gln Arg Glu Ile Thr Arg Thr Thr Leu Gln Ser Asp Gln
    770                 775                 780
Glu Glu Ile Asp Tyr Asp Asp Thr Ile Ser Val Glu Met Lys Lys Glu

785                 790                 795                 800
Asp Phe Asp Ile Tyr Asp Glu Asp Glu Asn Gln Ser Pro Arg Ser Phe
                805                 810                 815
Gln Lys Lys Thr Arg His Tyr Phe Ile Ala Ala Val Glu Arg Leu Trp
            820                 825                 830
Asp Tyr Gly Met Ser Ser Ser Pro His Val Leu Arg Asn Arg Ala Gln
        835                 840                 845
Ser Gly Ser Val Pro Gln Phe Lys Lys Val Val Phe Gln Glu Phe Thr
    850                 855                 860
Asp Gly Ser Phe Thr Gln Pro Leu Tyr Arg Gly Glu Leu Asn Glu His
865                 870                 875                 880
Leu Gly Leu Leu Gly Pro Tyr Ile Arg Ala Glu Val Glu Asp Asn Ile
                885                 890                 895
Met Val Thr Phe Arg Asn Gln Ala Ser Arg Pro Tyr Ser Phe Tyr Ser
            900                 905                 910
Ser Leu Ile Ser Tyr Glu Glu Asp Gln Arg Gln Gly Ala Glu Pro Arg
        915                 920                 925
Lys Asn Phe Val Lys Pro Asn Glu Thr Lys Thr Tyr Phe Trp Lys Val
    930                 935                 940
Gln His His Met Ala Pro Thr Lys Asp Glu Phe Asp Cys Lys Ala Trp
945                 950                 955                 960
Ala Tyr Phe Ser Asp Val Asp Leu Glu Lys Asp Val His Ser Gly Leu
                965                 970                 975
Ile Gly Pro Leu Leu Val Cys His Thr Asn Thr Leu Asn Pro Ala His
            980                 985                 990
Gly Arg Gln Val Thr Val Gln Glu Phe Ala Leu Phe Phe Thr Ile Phe
        995                 1000                1005
Asp Glu Thr Lys Ser Trp Tyr Phe Thr Glu Asn Met Glu Arg Asn Cys
    1010                1015                1020
Arg Ala Pro Cys Asn Ile Gln Met Glu Asp Pro Thr Phe Lys Glu Asn
1025                1030                1035                1040
Tyr Arg Phe His Ala Ile Asn Gly Tyr Ile Met Asp Thr Leu Pro Gly
                1045                1050                1055
Leu Val Met Ala Gln Asp Gln Arg Ile Arg Trp Tyr Leu Leu Ser Met
            1060                1065                1070
Gly Ser Asn Glu Asn Ile His Ser Ile His Phe Ser Gly His Val Phe
        1075                1080                1085
Thr Val Arg Lys Lys Glu Glu Tyr Lys Met Ala Leu Tyr Asn Leu Tyr
    1090                1095                1100
Pro Gly Val Phe Glu Thr Val Glu Met Leu Pro Ser Lys Ala Gly Ile
1105                1110                1115                1120
Trp Arg Val Glu Cys Leu Ile Gly Glu His Leu His Ala Gly Met Ser
                1125                1130                1135
Thr Leu Phe Leu Val Tyr Ser Asn Lys Cys Gln Thr Pro Leu Gly Met
            1140                1145                1150
Ala Ser Gly His Ile Arg Asp Phe Gln Ile Thr Ala Ser Gly Gln Tyr
        1155                1160                1165
Gly Gln Trp Ala Pro Lys Leu Ala Arg Leu His Tyr Ser Gly Ser Ile
    1170                1175                1180
Asn Ala Trp Ser Thr Lys Glu Pro Phe Ser Trp Ile Lys Val Asp Leu
1185                1190                1195                1200
Leu Ala Pro Met Ile Ile His Gly Ile Lys Thr Gln Gly Ala Arg Gln
                1205                1210                1215
Lys Phe Ser Ser Leu Tyr Ile Ser Gln Phe Ile Ile Met Tyr Ser Leu
            1220                1225                1230
Asp Gly Lys Lys Trp Gln Thr Tyr Arg Gly Asn Ser Thr Gly Thr Leu
        1235                1240                1245
Met Val Phe Phe Gly Asn Val Asp Ser Ser Gly Ile Lys His Asn Ile
    1250                1255                1260
Phe Asn Pro Pro Ile Ile Ala Arg Tyr Ile Arg Leu His Pro Thr His
1265                1270                1275                1280
Tyr Ser Ile Arg Ser Thr Leu Arg Met Glu Leu Met Gly Cys Asp Leu
                1285                1290                1295

Asn Ser Cys Ser Met Pro Leu Gly Met Glu Ser Lys Ala Ile Ser Asp
                1300                1305                1310
Ala Gln Ile Thr Ala Ser Ser Tyr Phe Thr Asn Met Phe Ala Thr Trp
            1315                1320                1325
Ser Pro Ser Lys Ala Arg Leu His Leu Gln Gly Arg Ser Asn Ala Trp
        1330                1335                1340
Arg Pro Gln Val Asn Asn Pro Lys Glu Trp Leu Gln Val Asp Phe Gln
1345                1350                1355                1360
Lys Thr Met Lys Val Thr Gly Val Thr Thr Gln Gly Val Lys Ser Leu
                    1365                1370                1375
Leu Thr Ser Met Tyr Val Lys Glu Phe Leu Ile Ser Ser Ser Gln Asp
                1380                1385                1390
Gly His Gln Trp Thr Leu Phe Phe Gln Asn Gly Lys Val Lys Val Phe
            1395                1400                1405
Gln Gly Asn Gln Asp Ser Phe Thr Pro Val Val Asn Ser Leu Asp Pro
        1410                1415                1420
Pro Leu Leu Thr Arg Tyr Leu Arg Ile His Pro Gln Ser Trp Val His
1425                1430                1435                1440
Gln Ile Ala Leu Arg Met Glu Val Leu Gly Cys Glu Ala Gln Asp Leu
                    1445                1450                1455
Tyr

<210> 29
<400> 29
000

<210> 30
<400> 30
000

<210> 31
<400> 31
000

<210> 32
<400> 32
000

<210> 33
<400> 33
000

<210> 34
<400> 34
000

<210> 35
<400> 35
000

<210> 36
<400> 36
000

<210> 37
<400> 37
000

<210> 38
<400> 38
000

<210> 39
<211> 19
<212> PRT
<213> Artificial Sequence

<220>
<223> Thrombin-cleavable linker

<400> 39
Ser Lys Asn Asn Ala Ile Glu Pro Arg Ser Phe Ser Gln Asn Ser Arg
1               5                   10                  15
His Pro Ser

<210> 40
<211> 14
<212> PRT
<213> Artificial Sequence

<220>
<223> glycine-serine linker G1

<220>
<221> VARIANT
<222> 14
<223> G in position 14 is present or absent

<400> 40
Gly Ser Gly Gly Ser Gly Gly Ser Gly Gly Ser Gly Ser Gly
1               5                   10

<210> 41
<211> 12
<212> PRT
<213> Artificial Sequence

<220>
<223> glycine-serine linker G2

<400> 41
Gly Gly Ser Gly Gly Gly Ser Gly Gly Gly Ser Gly
1               5                   10

<210> 42
<211> 46
<212> PRT
<213> Artificial Sequence

<220>
<223> GLG-1 linker

<400> 42
Gly Ser Gly Gly Ser Gly Gly Ser Gly Gly Ser Gly Ser Gly Ser Lys
1               5                   10                  15
Asn Asn Ala Ile Glu Pro Arg Ser Phe Ser Gln Asn Ser Arg His Pro
            20                  25                  30
Ser Gly Ser Gly Gly Ser Gly Gly Ser Gly Gly Ser Gly Ser
        35                  40                  45

<210> 43
<211> 43
<212> PRT
<213> Artificial Sequence

<220>
<223> GLG-2 linker

<400> 43
Gly Gly Ser Gly Gly Gly Ser Gly Gly Gly Ser Gly Ser Lys Asn Asn
1               5                   10                  15
Ala Ile Glu Pro Arg Ser Phe Ser Gln Asn Ser Arg His Pro Ser Gly
            20                  25                  30
Gly Ser Gly Gly Gly Ser Gly Gly Gly Ser Gly
        35                  40

<210> 44
<211> 46
<212> PRT
<213> Artificial Sequence

<220>
<223> ABD consensus sequence

<220>
<221> VARIANT
<222> 3
<223> X3 is selected from E, S, Q and C

<220>
<221> VARIANT
<222> 6
<223> X6 is selected from E, S, C and V

<220>
<221> VARIANT
<222> 7
<223> X7 is selected from A, S and L

<220>
<221> VARIANT
<222> 10
<223> X10 is selected from A, S and R

<220>
<221> VARIANT
<222> 14
<223> X14 is selected from A, S, C and K

<220>
<221> VARIANT
<222> 26
<223> X26 is selected from D, E and N

<220>
<221> VARIANT
<222> 39
<223> X39 is selected from D, E and L

<220>
<221> VARIANT

<222> 40
<223> X40 is selected from A, E and H

<220>
<221> VARIANT
<222> 43
<223> X43 is selected from A and K

<220>
<221> VARIANT
<222> 44
<223> X44 is selected from A, S and E

<220>
<221> VARIANT
<222> 45
<223> L in position 45 is present or absent

<220>
<221> VARIANT
<222> 46
<223> P in position 46 is present or absent

<400> 44
Leu Ala Xaa Ala Lys Xaa Xaa Ala Asn Xaa Glu Leu Asp Xaa Tyr Gly
1               5                   10                  15
Val Ser Asp Phe Tyr Lys Arg Leu Ile Xaa Lys Ala Lys Thr Val Glu
            20                  25                  30
Gly Val Glu Ala Leu Lys Xaa Xaa Ile Leu Xaa Xaa Leu Pro
        35                  40                  45

<210> 45
<211> 46
<212> PRT
<213> Artificial Sequence

<220>
<223> ABD1

<400> 45
Leu Ala Glu Ala Lys Val Leu Ala Asn Arg Glu Leu Asp Lys Tyr Gly
1               5                   10                  15
Val Ser Asp Phe Tyr Lys Arg Leu Ile Asn Lys Ala Lys Thr Val Glu
            20                  25                  30
Gly Val Glu Ala Leu Lys Leu His Ile Leu Ala Ala Leu Pro
        35                  40                  45

<210> 46
<211> 46
<212> PRT
<213> Artificial Sequence

<220>
<223> ABD2

<400> 46
Leu Ala Glu Ala Lys Glu Ala Ala Asn Ala Glu Leu Asp Ser Tyr Gly
1               5                   10                  15
Val Ser Asp Phe Tyr Lys Arg Leu Ile Asp Lys Ala Lys Thr Val Glu
            20                  25                  30
Gly Val Glu Ala Leu Lys Asp Ala Ile Leu Ala Ala Leu Pro

35                  40                  45

<210> 47
<211> 1575
<212> PRT
<213> Artificial Sequence

<220>
<223> ADLCLD_SC aa

<400> 47
Met Gln Ile Glu Leu Ser Thr Cys Phe Phe Leu Cys Leu Leu Arg Phe
1               5                   10                  15
Cys Phe Ser Ala Thr Arg Arg Tyr Tyr Leu Gly Ala Val Glu Leu Ser
            20                  25                  30
Trp Asp Tyr Met Gln Ser Asp Leu Gly Glu Leu Pro Val Asp Ala Arg
        35                  40                  45
Phe Pro Pro Arg Val Pro Lys Ser Phe Pro Phe Asn Thr Ser Val Val
    50                  55                  60
Tyr Lys Lys Thr Leu Phe Val Glu Phe Thr Asp His Leu Phe Asn Ile
65                  70                  75                  80
Ala Lys Pro Arg Pro Pro Trp Met Gly Leu Leu Gly Pro Thr Ile Gln
                85                  90                  95
Ala Glu Val Tyr Asp Thr Val Val Ile Thr Leu Lys Asn Met Ala Ser
            100                 105                 110
His Pro Val Ser Leu His Ala Val Gly Val Ser Tyr Trp Lys Ala Ser
        115                 120                 125
Glu Gly Ala Glu Tyr Asp Asp Gln Thr Ser Gln Arg Glu Lys Glu Asp
    130                 135                 140
Asp Lys Val Phe Pro Gly Gly Ser His Thr Tyr Val Trp Gln Val Leu
145                 150                 155                 160
Lys Glu Asn Gly Pro Met Ala Ser Asp Pro Leu Cys Leu Thr Tyr Ser
                165                 170                 175
Tyr Leu Ser His Val Asp Leu Val Lys Asp Leu Asn Ser Gly Leu Ile
            180                 185                 190
Gly Ala Leu Leu Val Cys Arg Glu Gly Ser Leu Ala Lys Glu Lys Thr
        195                 200                 205
Gln Thr Leu His Lys Phe Ile Leu Leu Phe Ala Val Phe Asp Glu Gly
    210                 215                 220
Lys Ser Trp His Ser Glu Thr Lys Asn Ser Leu Met Gln Asp Arg Asp
225                 230                 235                 240
Ala Ala Ser Ala Arg Ala Trp Pro Lys Met His Thr Val Asn Gly Tyr
                245                 250                 255
Val Asn Arg Ser Leu Pro Gly Leu Ile Gly Cys His Arg Lys Ser Val
            260                 265                 270
Tyr Trp His Val Ile Gly Met Gly Thr Thr Pro Glu Val His Ser Ile
        275                 280                 285
Phe Leu Glu Gly His Thr Phe Leu Val Arg Asn His Arg Gln Ala Ser
    290                 295                 300
Leu Glu Ile Ser Pro Ile Thr Phe Leu Thr Ala Gln Thr Leu Leu Met
305                 310                 315                 320
Asp Leu Gly Gln Phe Leu Leu Phe Cys His Ile Ser Ser His Gln His
                325                 330                 335
Asp Gly Met Glu Ala Tyr Val Lys Val Asp Ser Cys Pro Glu Glu Pro
            340                 345                 350
Gln Leu Arg Met Lys Asn Asn Glu Glu Ala Glu Asp Tyr Asp Asp Asp
        355                 360                 365
Leu Thr Asp Ser Glu Met Asp Val Val Arg Phe Asp Asp Asp Asn Ser
    370                 375                 380
Pro Ser Phe Ile Gln Ile Arg Ser Val Ala Lys Lys His Pro Lys Thr
385                 390                 395                 400
Trp Val His Tyr Ile Ala Ala Glu Glu Glu Asp Trp Asp Tyr Ala Pro

                405                 410                 415
Leu Val Leu Ala Pro Asp Asp Arg Ser Tyr Lys Ser Gln Tyr Leu Asn
            420                 425                 430
Asn Gly Pro Gln Arg Ile Gly Arg Lys Tyr Lys Lys Val Arg Phe Met
        435                 440                 445
Ala Tyr Thr Asp Glu Thr Phe Lys Thr Arg Glu Ala Ile Gln His Glu
    450                 455                 460
Ser Gly Ile Leu Gly Pro Leu Leu Tyr Gly Glu Val Gly Asp Thr Leu
465                 470                 475                 480
Leu Ile Ile Phe Lys Asn Gln Ala Ser Arg Pro Tyr Asn Ile Tyr Pro
                485                 490                 495
His Gly Ile Thr Asp Val Arg Pro Leu Tyr Ser Arg Arg Leu Pro Lys
            500                 505                 510
Gly Val Lys His Leu Lys Asp Phe Pro Ile Leu Pro Gly Glu Ile Phe
        515                 520                 525
Lys Tyr Lys Trp Thr Val Thr Val Glu Asp Gly Pro Thr Lys Ser Asp
    530                 535                 540
Pro Arg Cys Leu Thr Arg Tyr Tyr Ser Ser Phe Val Asn Met Glu Arg
545                 550                 555                 560
Asp Leu Ala Ser Gly Leu Ile Gly Pro Leu Leu Ile Cys Tyr Lys Glu
                565                 570                 575
Ser Val Asp Gln Arg Gly Asn Gln Ile Met Ser Asp Lys Arg Asn Val
            580                 585                 590
Ile Leu Phe Ser Val Phe Asp Glu Asn Arg Ser Trp Tyr Leu Thr Glu
        595                 600                 605
Asn Ile Gln Arg Phe Leu Pro Asn Pro Ala Gly Val Gln Leu Glu Asp
    610                 615                 620
Pro Glu Phe Gln Ala Ser Asn Ile Met His Ser Ile Asn Gly Tyr Val
625                 630                 635                 640
Phe Asp Ser Leu Gln Leu Ser Val Cys Leu His Glu Val Ala Tyr Trp
                645                 650                 655
Tyr Ile Leu Ser Ile Gly Ala Gln Thr Asp Phe Leu Ser Val Phe Phe
            660                 665                 670
Ser Gly Tyr Thr Phe Lys His Lys Met Val Tyr Glu Asp Thr Leu Thr
        675                 680                 685
Leu Phe Pro Phe Ser Gly Glu Thr Val Phe Met Ser Met Glu Asn Pro
    690                 695                 700
Gly Leu Trp Ile Leu Gly Cys His Asn Ser Asp Phe Arg Asn Arg Gly
705                 710                 715                 720
Met Thr Ala Leu Leu Lys Val Ser Ser Cys Asp Lys Asn Thr Gly Asp
                725                 730                 735
Tyr Tyr Glu Asp Ser Tyr Glu Asp Ile Ser Ala Tyr Leu Leu Ser Lys
            740                 745                 750
Asn Asn Ala Ile Glu Pro Arg Ser Phe Ser Gln Asn Pro Pro Val Leu
        755                 760                 765
Ala Glu Ala Lys Glu Ala Ala Asn Ala Glu Leu Asp Ser Tyr Gly Val
    770                 775                 780
Ser Asp Phe Tyr Lys Arg Leu Ile Asp Lys Ala Lys Thr Val Glu Gly
785                 790                 795                 800
Val Glu Ala Leu Lys Asp Ala Ile Leu Ala Ala Leu Pro Ser Lys Asn
                805                 810                 815
Asn Ala Ile Glu Pro Arg Ser Phe Ser Gln Asn Ser Arg His Pro Ser
            820                 825                 830
Leu Gln Ser Asp Gln Glu Glu Ile Asp Tyr Asp Asp Thr Ile Ser Val
        835                 840                 845
Glu Met Lys Lys Glu Asp Phe Asp Ile Tyr Asp Glu Asp Glu Asn Gln
    850                 855                 860
Ser Pro Arg Ser Phe Gln Lys Lys Thr Arg His Tyr Phe Ile Ala Ala
865                 870                 875                 880
Val Glu Arg Leu Trp Asp Tyr Gly Met Ser Ser Ser Pro His Val Leu
                885                 890                 895
Arg Asn Arg Ala Gln Ser Gly Ser Val Pro Gln Phe Lys Lys Val Val
            900                 905                 910

Phe Gln Glu Phe Thr Asp Gly Ser Phe Thr Gln Pro Leu Tyr Arg Gly
        915                 920                 925
Glu Leu Asn Glu His Leu Gly Leu Leu Gly Pro Tyr Ile Arg Ala Glu
    930                 935                 940
Val Glu Asp Asn Ile Met Val Thr Phe Arg Asn Gln Ala Ser Arg Pro
945                 950                 955                 960
Tyr Ser Phe Tyr Ser Ser Leu Ile Ser Tyr Glu Glu Asp Gln Arg Gln
                965                 970                 975
Gly Ala Glu Pro Arg Lys Asn Phe Val Lys Pro Asn Glu Thr Lys Thr
            980                 985                 990
Tyr Phe Trp Lys Val Gln His His Met Ala Pro Thr Lys Asp Glu Phe
        995                 1000                1005
Asp Cys Lys Ala Trp Ala Tyr Phe Ser Asp Val Asp Leu Glu Lys Asp
    1010                1015                1020
Val His Ser Gly Leu Ile Gly Pro Leu Leu Val Cys His Thr Asn Thr
1025                1030                1035                1040
Leu Asn Pro Ala His Gly Arg Gln Val Thr Val Gln Glu Phe Ala Leu
                1045                1050                1055
Phe Phe Thr Ile Phe Asp Glu Thr Lys Ser Trp Tyr Phe Thr Glu Asn
            1060                1065                1070
Met Glu Arg Asn Cys Arg Ala Pro Cys Asn Ile Gln Met Glu Asp Pro
        1075                1080                1085
Thr Phe Lys Glu Asn Tyr Arg Phe His Ala Ile Asn Gly Tyr Ile Met
    1090                1095                1100
Asp Thr Leu Pro Gly Leu Val Met Ala Gln Asp Gln Arg Ile Arg Trp
1105                1110                1115                1120
Tyr Leu Leu Ser Met Gly Ser Asn Glu Asn Ile His Ser Ile His Phe
                1125                1130                1135
Ser Gly His Val Phe Thr Val Arg Lys Lys Glu Glu Tyr Lys Met Ala
            1140                1145                1150
Leu Tyr Asn Leu Tyr Pro Gly Val Phe Glu Thr Val Glu Met Leu Pro
        1155                1160                1165
Ser Lys Ala Gly Ile Trp Arg Val Glu Cys Leu Ile Gly Glu His Leu
    1170                1175                1180
His Ala Gly Met Ser Thr Leu Phe Leu Val Tyr Ser Asn Lys Cys Gln
1185                1190                1195                1200
Thr Pro Leu Gly Met Ala Ser Gly His Ile Arg Asp Phe Gln Ile Thr
                1205                1210                1215
Ala Ser Gly Gln Tyr Gly Gln Trp Ala Pro Lys Leu Ala Arg Leu His
            1220                1225                1230
Tyr Ser Gly Ser Ile Asn Ala Trp Ser Thr Lys Glu Pro Phe Ser Trp
        1235                1240                1245
Ile Lys Val Asp Leu Leu Ala Pro Met Ile Ile His Gly Ile Lys Thr
    1250                1255                1260
Gln Gly Ala Arg Gln Lys Phe Ser Ser Leu Tyr Ile Ser Gln Phe Ile
1265                1270                1275                1280
Ile Met Tyr Ser Leu Asp Gly Lys Lys Trp Gln Thr Tyr Arg Gly Asn
                1285                1290                1295
Ser Thr Gly Thr Leu Met Val Phe Phe Gly Asn Val Asp Ser Ser Gly
            1300                1305                1310
Ile Lys His Asn Ile Phe Asn Pro Pro Ile Ile Ala Arg Tyr Ile Arg
        1315                1320                1325
Leu His Pro Thr His Tyr Ser Ile Arg Ser Thr Leu Arg Met Glu Leu
    1330                1335                1340
Met Gly Cys Asp Leu Asn Ser Cys Ser Met Pro Leu Gly Met Glu Ser
1345                1350                1355                1360
Lys Ala Ile Ser Asp Ala Gln Ile Thr Ala Ser Ser Tyr Phe Thr Asn
                1365                1370                1375
Met Phe Ala Thr Trp Ser Pro Ser Lys Ala Arg Leu His Leu Gln Gly
            1380                1385                1390
Arg Ser Asn Ala Trp Arg Pro Gln Val Asn Asn Pro Lys Glu Trp Leu
        1395                1400                1405
Gln Val Asp Phe Gln Lys Thr Met Lys Val Thr Gly Val Thr Thr Gln

1410                    1415                    1420
Gly Val Lys Ser Leu Leu Thr Ser Met Tyr Val Lys Glu Phe Leu Ile
1425                    1430                    1435                    1440
Ser Ser Ser Gln Asp Gly His Gln Trp Thr Leu Phe Phe Gln Asn Gly
                1445                    1450                    1455
Lys Val Lys Val Phe Gln Gly Asn Gln Asp Ser Phe Thr Pro Val Val
            1460                    1465                    1470
Asn Ser Leu Asp Pro Pro Leu Leu Thr Arg Tyr Leu Arg Ile His Pro
        1475                    1480                    1485
Gln Ser Trp Val His Gln Ile Ala Leu Arg Met Glu Val Leu Gly Cys
    1490                    1495                    1500
Glu Ala Gln Asp Leu Tyr Ser Lys Asn Asn Ala Ile Glu Pro Arg Ser
1505                    1510                    1515                    1520
Phe Ser Gln Asn Ser Arg His Pro Ser Leu Ala Glu Ala Lys Glu Ala
                1525                    1530                    1535
Ala Asn Ala Glu Leu Asp Ser Tyr Gly Val Ser Asp Phe Tyr Lys Arg
            1540                    1545                    1550
Leu Ile Asp Lys Ala Lys Thr Val Glu Gly Val Glu Ala Leu Lys Asp
        1555                    1560                    1565
Ala Ile Leu Ala Ala Leu Pro
    1570                    1575

<210> 48
<211> 1778
<212> PRT
<213> Artificial Sequence

<220>
<223> AD2CD2_SC aa

<400> 48
Met Gln Ile Glu Leu Ser Thr Cys Phe Phe Leu Cys Leu Leu Arg Phe
1               5                   10                  15
Cys Phe Ser Ala Thr Arg Arg Tyr Tyr Leu Gly Ala Val Glu Leu Ser
            20                  25                  30
Trp Asp Tyr Met Gln Ser Asp Leu Gly Glu Leu Pro Val Asp Ala Arg
        35                  40                  45
Phe Pro Pro Arg Val Pro Lys Ser Phe Pro Phe Asn Thr Ser Val Val
    50                  55                  60
Tyr Lys Lys Thr Leu Phe Val Glu Phe Thr Asp His Leu Phe Asn Ile
65                  70                  75                  80
Ala Lys Pro Arg Pro Pro Trp Met Gly Leu Leu Gly Pro Thr Ile Gln
                85                  90                  95
Ala Glu Val Tyr Asp Thr Val Val Ile Thr Leu Lys Asn Met Ala Ser
            100                 105                 110
His Pro Val Ser Leu His Ala Val Gly Val Ser Tyr Trp Lys Ala Ser
        115                 120                 125
Glu Gly Ala Glu Tyr Asp Asp Gln Thr Ser Gln Arg Glu Lys Glu Asp
    130                 135                 140
Asp Lys Val Phe Pro Gly Gly Ser His Thr Tyr Val Trp Gln Val Leu
145                 150                 155                 160
Lys Glu Asn Gly Pro Met Ala Ser Asp Pro Leu Cys Leu Thr Tyr Ser
                165                 170                 175
Tyr Leu Ser His Val Asp Leu Val Lys Asp Leu Asn Ser Gly Leu Ile
            180                 185                 190
Gly Ala Leu Leu Val Cys Arg Glu Gly Ser Leu Ala Lys Glu Lys Thr
        195                 200                 205
Gln Thr Leu His Lys Phe Ile Leu Leu Phe Ala Val Phe Asp Glu Gly
    210                 215                 220
Lys Ser Trp His Ser Glu Thr Lys Asn Ser Leu Met Gln Asp Arg Asp
225                 230                 235                 240
Ala Ala Ser Ala Arg Ala Trp Pro Lys Met His Thr Val Asn Gly Tyr

245                 250                 255
Val Asn Arg Ser Leu Pro Gly Leu Ile Gly Cys His Arg Lys Ser Val
            260                 265                 270
Tyr Trp His Val Ile Gly Met Gly Thr Thr Pro Glu Val His Ser Ile
        275                 280                 285
Phe Leu Glu Gly His Thr Phe Leu Val Arg Asn His Arg Gln Ala Ser
    290                 295                 300
Leu Glu Ile Ser Pro Ile Thr Phe Leu Thr Ala Gln Thr Leu Leu Met
305                 310                 315                 320
Asp Leu Gly Gln Phe Leu Leu Phe Cys His Ile Ser Ser His Gln His
                325                 330                 335
Asp Gly Met Glu Ala Tyr Val Lys Val Asp Ser Cys Pro Glu Glu Pro
            340                 345                 350
Gln Leu Arg Met Lys Asn Asn Glu Glu Ala Glu Asp Tyr Asp Asp Asp
        355                 360                 365
Leu Thr Asp Ser Glu Met Asp Val Val Arg Phe Asp Asp Asp Asn Ser
    370                 375                 380
Pro Ser Phe Ile Gln Ile Arg Ser Val Ala Lys Lys His Pro Lys Thr
385                 390                 395                 400
Trp Val His Tyr Ile Ala Ala Glu Glu Glu Asp Trp Asp Tyr Ala Pro
                405                 410                 415
Leu Val Leu Ala Pro Asp Asp Arg Ser Tyr Lys Ser Gln Tyr Leu Asn
            420                 425                 430
Asn Gly Pro Gln Arg Ile Gly Arg Lys Tyr Lys Lys Val Arg Phe Met
        435                 440                 445
Ala Tyr Thr Asp Glu Thr Phe Lys Thr Arg Glu Ala Ile Gln His Glu
    450                 455                 460
Ser Gly Ile Leu Gly Pro Leu Leu Tyr Gly Glu Val Gly Asp Thr Leu
465                 470                 475                 480
Leu Ile Ile Phe Lys Asn Gln Ala Ser Arg Pro Tyr Asn Ile Tyr Pro
                485                 490                 495
His Gly Ile Thr Asp Val Arg Pro Leu Tyr Ser Arg Arg Leu Pro Lys
            500                 505                 510
Gly Val Lys His Leu Lys Asp Phe Pro Ile Leu Pro Gly Glu Ile Phe
        515                 520                 525
Lys Tyr Lys Trp Thr Val Thr Val Glu Asp Gly Pro Thr Lys Ser Asp
    530                 535                 540
Pro Arg Cys Leu Thr Arg Tyr Tyr Ser Ser Phe Val Asn Met Glu Arg
545                 550                 555                 560
Asp Leu Ala Ser Gly Leu Ile Gly Pro Leu Leu Ile Cys Tyr Lys Glu
                565                 570                 575
Ser Val Asp Gln Arg Gly Asn Gln Ile Met Ser Asp Lys Arg Asn Val
            580                 585                 590
Ile Leu Phe Ser Val Phe Asp Glu Asn Arg Ser Trp Tyr Leu Thr Glu
        595                 600                 605
Asn Ile Gln Arg Phe Leu Pro Asn Pro Ala Gly Val Gln Leu Glu Asp
    610                 615                 620
Pro Glu Phe Gln Ala Ser Asn Ile Met His Ser Ile Asn Gly Tyr Val
625                 630                 635                 640
Phe Asp Ser Leu Gln Leu Ser Val Cys Leu His Glu Val Ala Tyr Trp
                645                 650                 655
Tyr Ile Leu Ser Ile Gly Ala Gln Thr Asp Phe Leu Ser Val Phe Phe
            660                 665                 670
Ser Gly Tyr Thr Phe Lys His Lys Met Val Tyr Glu Asp Thr Leu Thr
        675                 680                 685
Leu Phe Pro Phe Ser Gly Glu Thr Val Phe Met Ser Met Glu Asn Pro
    690                 695                 700
Gly Leu Trp Ile Leu Gly Cys His Asn Ser Asp Phe Arg Asn Arg Gly
705                 710                 715                 720
Met Thr Ala Leu Leu Lys Val Ser Ser Cys Asp Lys Asn Thr Gly Asp
                725                 730                 735
Tyr Tyr Glu Asp Ser Tyr Glu Asp Ile Ser Ala Tyr Leu Leu Ser Lys
            740                 745                 750

Asn Asn Ala Ile Glu Pro Arg Ser Phe Ser Gln Asn Pro Pro Val Leu
        755                 760                 765
Ser Lys Asn Asn Ala Ile Glu Pro Arg Ser Phe Ser Gln Asn Ser Arg
    770                 775                 780
His Pro Ser Leu Ala Glu Ala Lys Glu Ala Ala Asn Ala Glu Leu Asp
785                 790                 795                 800
Ser Tyr Gly Val Ser Asp Phe Tyr Lys Arg Leu Ile Asp Lys Ala Lys
                805                 810                 815
Thr Val Glu Gly Val Glu Ala Leu Lys Asp Ala Ile Leu Ala Ala Leu
            820                 825                 830
Pro Gly Ser Gly Gly Ser Gly Gly Ser Gly Gly Ser Gly Ser Gly Ser
        835                 840                 845
Lys Asn Asn Ala Ile Glu Pro Arg Ser Phe Ser Gln Asn Ser Arg His
    850                 855                 860
Pro Ser Gly Ser Gly Gly Ser Gly Gly Ser Gly Gly Ser Gly Ser Leu
865                 870                 875                 880
Ala Glu Ala Lys Glu Ala Ala Asn Ala Glu Leu Asp Ser Tyr Gly Val
                885                 890                 895
Ser Asp Phe Tyr Lys Arg Leu Ile Asp Lys Ala Lys Thr Val Glu Gly
            900                 905                 910
Val Glu Ala Leu Lys Asp Ala Ile Leu Ala Ala Leu Pro Ser Lys Asn
        915                 920                 925
Asn Ala Ile Glu Pro Arg Ser Phe Ser Gln Asn Ser Arg His Pro Ser
    930                 935                 940
Gln Ser Asp Gln Glu Glu Ile Asp Tyr Asp Asp Thr Ile Ser Val Glu
945                 950                 955                 960
Met Lys Lys Glu Asp Phe Asp Ile Tyr Asp Glu Asp Glu Asn Gln Ser
                965                 970                 975
Pro Arg Ser Phe Gln Lys Lys Thr Arg His Tyr Phe Ile Ala Ala Val
            980                 985                 990
Glu Arg Leu Trp Asp Tyr Gly Met Ser Ser Ser Pro His Val Leu Arg
        995                 1000                1005
Asn Arg Ala Gln Ser Gly Ser Val Pro Gln Phe Lys Lys Val Val Phe
    1010                1015                1020
Gln Glu Phe Thr Asp Gly Ser Phe Thr Gln Pro Leu Tyr Arg Gly Glu
1025                1030                1035                1040
Leu Asn Glu His Leu Gly Leu Leu Gly Pro Tyr Ile Arg Ala Glu Val
                1045                1050                1055
Glu Asp Asn Ile Met Val Thr Phe Arg Asn Gln Ala Ser Arg Pro Tyr
            1060                1065                1070
Ser Phe Tyr Ser Ser Leu Ile Ser Tyr Glu Glu Asp Gln Arg Gln Gly
        1075                1080                1085
Ala Glu Pro Arg Lys Asn Phe Val Lys Pro Asn Glu Thr Lys Thr Tyr
    1090                1095                1100
Phe Trp Lys Val Gln His His Met Ala Pro Thr Lys Asp Glu Phe Asp
1105                1110                1115                1120
Cys Lys Ala Trp Ala Tyr Phe Ser Asp Val Asp Leu Glu Lys Asp Val
                1125                1130                1135
His Ser Gly Leu Ile Gly Pro Leu Leu Val Cys His Thr Asn Thr Leu
            1140                1145                1150
Asn Pro Ala His Gly Arg Gln Val Thr Val Gln Glu Phe Ala Leu Phe
        1155                1160                1165
Phe Thr Ile Phe Asp Glu Thr Lys Ser Trp Tyr Phe Thr Glu Asn Met
    1170                1175                1180
Glu Arg Asn Cys Arg Ala Pro Cys Asn Ile Gln Met Glu Asp Pro Thr
1185                1190                1195                1200
Phe Lys Glu Asn Tyr Arg Phe His Ala Ile Asn Gly Tyr Ile Met Asp
                1205                1210                1215
Thr Leu Pro Gly Leu Val Met Ala Gln Asp Gln Arg Ile Arg Trp Tyr
            1220                1225                1230
Leu Leu Ser Met Gly Ser Asn Glu Asn Ile His Ser Ile His Phe Ser
        1235                1240                1245
Gly His Val Phe Thr Val Arg Lys Lys Glu Glu Tyr Lys Met Ala Leu

1250                    1255                    1260
Tyr Asn Leu Tyr Pro Gly Val Phe Glu Thr Val Glu Met Leu Pro Ser
1265                    1270                    1275                    1280
Lys Ala Gly Ile Trp Arg Val Glu Cys Leu Ile Gly Glu His Leu His
                1285                    1290                    1295
Ala Gly Met Ser Thr Leu Phe Leu Val Tyr Ser Asn Lys Cys Gln Thr
            1300                    1305                    1310
Pro Leu Gly Met Ala Ser Gly His Ile Arg Asp Phe Gln Ile Thr Ala
        1315                    1320                    1325
Ser Gly Gln Tyr Gly Gln Trp Ala Pro Lys Leu Ala Arg Leu His Tyr
    1330                    1335                    1340
Ser Gly Ser Ile Asn Ala Trp Ser Thr Lys Glu Pro Phe Ser Trp Ile
1345                    1350                    1355                    1360
Lys Val Asp Leu Leu Ala Pro Met Ile Ile His Gly Ile Lys Thr Gln
                1365                    1370                    1375
Gly Ala Arg Gln Lys Phe Ser Ser Leu Tyr Ile Ser Gln Phe Ile Ile
            1380                    1385                    1390
Met Tyr Ser Leu Asp Gly Lys Lys Trp Gln Thr Tyr Arg Gly Asn Ser
        1395                    1400                    1405
Thr Gly Thr Leu Met Val Phe Phe Gly Asn Val Asp Ser Ser Gly Ile
    1410                    1415                    1420
Lys His Asn Ile Phe Asn Pro Pro Ile Ile Ala Arg Tyr Ile Arg Leu
1425                    1430                    1435                    1440
His Pro Thr His Tyr Ser Ile Arg Ser Thr Leu Arg Met Glu Leu Met
                1445                    1450                    1455
Gly Cys Asp Leu Asn Ser Cys Ser Met Pro Leu Gly Met Glu Ser Lys
            1460                    1465                    1470
Ala Ile Ser Asp Ala Gln Ile Thr Ala Ser Ser Tyr Phe Thr Asn Met
        1475                    1480                    1485
Phe Ala Thr Trp Ser Pro Ser Lys Ala Arg Leu His Leu Gln Gly Arg
    1490                    1495                    1500
Ser Asn Ala Trp Arg Pro Gln Val Asn Asn Pro Lys Glu Trp Leu Gln
1505                    1510                    1515                    1520
Val Asp Phe Gln Lys Thr Met Lys Val Thr Gly Val Thr Thr Gln Gly
                1525                    1530                    1535
Val Lys Ser Leu Leu Thr Ser Met Tyr Val Lys Glu Phe Leu Ile Ser
            1540                    1545                    1550
Ser Ser Gln Asp Gly His Gln Trp Thr Leu Phe Phe Gln Asn Gly Lys
        1555                    1560                    1565
Val Lys Val Phe Gln Gly Asn Gln Asp Ser Phe Thr Pro Val Val Asn
    1570                    1575                    1580
Ser Leu Asp Pro Pro Leu Leu Thr Arg Tyr Leu Arg Ile His Pro Gln
1585                    1590                    1595                    1600
Ser Trp Val His Gln Ile Ala Leu Arg Met Glu Val Leu Gly Cys Glu
                1605                    1610                    1615
Ala Gln Asp Leu Tyr Ser Lys Asn Asn Ala Ile Glu Pro Arg Ser Phe
            1620                    1625                    1630
Ser Gln Asn Ser Arg His Pro Ser Leu Ala Glu Ala Lys Glu Ala Ala
        1635                    1640                    1645
Asn Ala Glu Leu Asp Ser Tyr Gly Val Ser Asp Phe Tyr Lys Arg Leu
    1650                    1655                    1660
Ile Asp Lys Ala Lys Thr Val Glu Gly Val Glu Ala Leu Lys Asp Ala
1665                    1670                    1675                    1680
Ile Leu Ala Ala Leu Pro Gly Ser Gly Gly Ser Gly Gly Ser Gly Gly
                1685                    1690                    1695
Ser Gly Ser Gly Ser Lys Asn Asn Ala Ile Glu Pro Arg Ser Phe Ser
            1700                    1705                    1710
Gln Asn Ser Arg His Pro Ser Gly Ser Gly Gly Ser Gly Gly Ser Gly
        1715                    1720                    1725
Gly Ser Gly Ser Leu Ala Glu Ala Lys Glu Ala Ala Asn Ala Glu Leu
    1730                    1735                    1740
Asp Ser Tyr Gly Val Ser Asp Phe Tyr Lys Arg Leu Ile Asp Lys Ala
1745                    1750                    1755                    1760

Lys Thr Val Glu Gly Val Glu Ala Leu Lys Asp Ala Ile Leu Ala Ala
                1765                1770                1775
Leu Pro

<210> 49
<211> 1694
<212> PRT
<213> Artificial Sequence

<220>
<223> AD2CD2woL_SC aa

<400> 49
Met Gln Ile Glu Leu Ser Thr Cys Phe Phe Leu Cys Leu Leu Arg Phe
1               5                   10                  15
Cys Phe Ser Ala Thr Arg Arg Tyr Tyr Leu Gly Ala Val Glu Leu Ser
            20                  25                  30
Trp Asp Tyr Met Gln Ser Asp Leu Gly Glu Leu Pro Val Asp Ala Arg
        35                  40                  45
Phe Pro Pro Arg Val Pro Lys Ser Phe Pro Phe Asn Thr Ser Val Val
    50                  55                  60
Tyr Lys Lys Thr Leu Phe Val Glu Phe Thr Asp His Leu Phe Asn Ile
65                  70                  75                  80
Ala Lys Pro Arg Pro Pro Trp Met Gly Leu Leu Gly Pro Thr Ile Gln
                85                  90                  95
Ala Glu Val Tyr Asp Thr Val Val Ile Thr Leu Lys Asn Met Ala Ser
            100                 105                 110
His Pro Val Ser Leu His Ala Val Gly Val Ser Tyr Trp Lys Ala Ser
        115                 120                 125
Glu Gly Ala Glu Tyr Asp Asp Gln Thr Ser Gln Arg Glu Lys Glu Asp
    130                 135                 140
Asp Lys Val Phe Pro Gly Gly Ser His Thr Tyr Val Trp Gln Val Leu
145                 150                 155                 160
Lys Glu Asn Gly Pro Met Ala Ser Asp Pro Leu Cys Leu Thr Tyr Ser
                165                 170                 175
Tyr Leu Ser His Val Asp Leu Val Lys Asp Leu Asn Ser Gly Leu Ile
            180                 185                 190
Gly Ala Leu Leu Val Cys Arg Glu Gly Ser Leu Ala Lys Glu Lys Thr
        195                 200                 205
Gln Thr Leu His Lys Phe Ile Leu Leu Phe Ala Val Phe Asp Glu Gly
    210                 215                 220
Lys Ser Trp His Ser Glu Thr Lys Asn Ser Leu Met Gln Asp Arg Asp
225                 230                 235                 240
Ala Ala Ser Ala Arg Ala Trp Pro Lys Met His Thr Val Asn Gly Tyr
                245                 250                 255
Val Asn Arg Ser Leu Pro Gly Leu Ile Gly Cys His Arg Lys Ser Val
            260                 265                 270
Tyr Trp His Val Ile Gly Met Gly Thr Thr Pro Glu Val His Ser Ile
        275                 280                 285
Phe Leu Glu Gly His Thr Phe Leu Val Arg Asn His Arg Gln Ala Ser
    290                 295                 300
Leu Glu Ile Ser Pro Ile Thr Phe Leu Thr Ala Gln Thr Leu Leu Met
305                 310                 315                 320
Asp Leu Gly Gln Phe Leu Leu Phe Cys His Ile Ser Ser His Gln His
                325                 330                 335
Asp Gly Met Glu Ala Tyr Val Lys Val Asp Ser Cys Pro Glu Glu Pro
            340                 345                 350
Gln Leu Arg Met Lys Asn Asn Glu Glu Ala Glu Asp Tyr Asp Asp Asp
        355                 360                 365
Leu Thr Asp Ser Glu Met Asp Val Val Arg Phe Asp Asp Asp Asn Ser
    370                 375                 380

Pro Ser Phe Ile Gln Ile Arg Ser Val Ala Lys Lys His Pro Lys Thr
385                 390                 395                 400
Trp Val His Tyr Ile Ala Ala Glu Glu Glu Asp Trp Asp Tyr Ala Pro
                405                 410                 415
Leu Val Leu Ala Pro Asp Asp Arg Ser Tyr Lys Ser Gln Tyr Leu Asn
            420                 425                 430
Asn Gly Pro Gln Arg Ile Gly Arg Lys Tyr Lys Lys Val Arg Phe Met
        435                 440                 445
Ala Tyr Thr Asp Glu Thr Phe Lys Thr Arg Glu Ala Ile Gln His Glu
    450                 455                 460
Ser Gly Ile Leu Gly Pro Leu Leu Tyr Gly Glu Val Gly Asp Thr Leu
465                 470                 475                 480
Leu Ile Ile Phe Lys Asn Gln Ala Ser Arg Pro Tyr Asn Ile Tyr Pro
                485                 490                 495
His Gly Ile Thr Asp Val Arg Pro Leu Tyr Ser Arg Arg Leu Pro Lys
            500                 505                 510
Gly Val Lys His Leu Lys Asp Phe Pro Ile Leu Pro Gly Glu Ile Phe
        515                 520                 525
Lys Tyr Lys Trp Thr Val Thr Val Glu Asp Gly Pro Thr Lys Ser Asp
    530                 535                 540
Pro Arg Cys Leu Thr Arg Tyr Tyr Ser Ser Phe Val Asn Met Glu Arg
545                 550                 555                 560
Asp Leu Ala Ser Gly Leu Ile Gly Pro Leu Leu Ile Cys Tyr Lys Glu
                565                 570                 575
Ser Val Asp Gln Arg Gly Asn Gln Ile Met Ser Asp Lys Arg Asn Val
            580                 585                 590
Ile Leu Phe Ser Val Phe Asp Glu Asn Arg Ser Trp Tyr Leu Thr Glu
        595                 600                 605
Asn Ile Gln Arg Phe Leu Pro Asn Pro Ala Gly Val Gln Leu Glu Asp
    610                 615                 620
Pro Glu Phe Gln Ala Ser Asn Ile Met His Ser Ile Asn Gly Tyr Val
625                 630                 635                 640
Phe Asp Ser Leu Gln Leu Ser Val Cys Leu His Glu Val Ala Tyr Trp
                645                 650                 655
Tyr Ile Leu Ser Ile Gly Ala Gln Thr Asp Phe Leu Ser Val Phe Phe
            660                 665                 670
Ser Gly Tyr Thr Phe Lys His Lys Met Val Tyr Glu Asp Thr Leu Thr
        675                 680                 685
Leu Phe Pro Phe Ser Gly Glu Thr Val Phe Met Ser Met Glu Asn Pro
    690                 695                 700
Gly Leu Trp Ile Leu Gly Cys His Asn Ser Asp Phe Arg Asn Arg Gly
705                 710                 715                 720
Met Thr Ala Leu Leu Lys Val Ser Ser Cys Asp Lys Asn Thr Gly Asp
                725                 730                 735
Tyr Tyr Glu Asp Ser Tyr Glu Asp Ile Ser Ala Tyr Leu Leu Ser Lys
            740                 745                 750
Asn Asn Ala Ile Glu Pro Arg Ser Phe Ser Gln Asn Pro Pro Val Leu
        755                 760                 765
Gly Ser Gly Gly Ser Gly Gly Ser Gly Gly Ser Gly Ser Leu Ala Glu
    770                 775                 780
Ala Lys Glu Ala Ala Asn Ala Glu Leu Asp Ser Tyr Gly Val Ser Asp
785                 790                 795                 800
Phe Tyr Lys Arg Leu Ile Asp Lys Ala Lys Thr Val Glu Gly Val Glu
                805                 810                 815
Ala Leu Lys Asp Ala Ile Leu Ala Ala Leu Pro Gly Ser Gly Gly Ser
            820                 825                 830
Gly Gly Ser Gly Gly Ser Gly Ser Leu Ala Glu Ala Lys Glu Ala Ala
        835                 840                 845
Asn Ala Glu Leu Asp Ser Tyr Gly Val Ser Asp Phe Tyr Lys Arg Leu
    850                 855                 860
Ile Asp Lys Ala Lys Thr Val Glu Gly Val Glu Ala Leu Lys Asp Ala
865                 870                 875                 880
Ile Leu Ala Ala Leu Pro Gly Ser Gly Gly Ser Gly Gly Ser Gly Gly

885                 890                 895
Ser Gly Ser Gln Ser Asp Gln Glu Glu Ile Asp Tyr Asp Asp Thr Ile
            900                 905                 910
Ser Val Glu Met Lys Lys Glu Asp Phe Asp Ile Tyr Asp Glu Asp Glu
        915                 920                 925
Asn Gln Ser Pro Arg Ser Phe Gln Lys Lys Thr Arg His Tyr Phe Ile
    930                 935                 940
Ala Ala Val Glu Arg Leu Trp Asp Tyr Gly Met Ser Ser Ser Pro His
945                 950                 955                 960
Val Leu Arg Asn Arg Ala Gln Ser Gly Ser Val Pro Gln Phe Lys Lys
                965                 970                 975
Val Val Phe Gln Glu Phe Thr Asp Gly Ser Phe Thr Gln Pro Leu Tyr
            980                 985                 990
Arg Gly Glu Leu Asn Glu His Leu Gly Leu Leu Gly Pro Tyr Ile Arg
        995                 1000                1005
Ala Glu Val Glu Asp Asn Ile Met Val Thr Phe Arg Asn Gln Ala Ser
    1010                1015                1020
Arg Pro Tyr Ser Phe Tyr Ser Ser Leu Ile Ser Tyr Glu Glu Asp Gln
1025                1030                1035                1040
Arg Gln Gly Ala Glu Pro Arg Lys Asn Phe Val Lys Pro Asn Glu Thr
                1045                1050                1055
Lys Thr Tyr Phe Trp Lys Val Gln His His Met Ala Pro Thr Lys Asp
            1060                1065                1070
Glu Phe Asp Cys Lys Ala Trp Ala Tyr Phe Ser Asp Val Asp Leu Glu
        1075                1080                1085
Lys Asp Val His Ser Gly Leu Ile Gly Pro Leu Leu Val Cys His Thr
    1090                1095                1100
Asn Thr Leu Asn Pro Ala His Gly Arg Gln Val Thr Val Gln Glu Phe
1105                1110                1115                1120
Ala Leu Phe Phe Thr Ile Phe Asp Glu Thr Lys Ser Trp Tyr Phe Thr
                1125                1130                1135
Glu Asn Met Glu Arg Asn Cys Arg Ala Pro Cys Asn Ile Gln Met Glu
            1140                1145                1150
Asp Pro Thr Phe Lys Glu Asn Tyr Arg Phe His Ala Ile Asn Gly Tyr
        1155                1160                1165
Ile Met Asp Thr Leu Pro Gly Leu Val Met Ala Gln Asp Gln Arg Ile
    1170                1175                1180
Arg Trp Tyr Leu Leu Ser Met Gly Ser Asn Glu Asn Ile His Ser Ile
1185                1190                1195                1200
His Phe Ser Gly His Val Phe Thr Val Arg Lys Lys Glu Glu Tyr Lys
                1205                1210                1215
Met Ala Leu Tyr Asn Leu Tyr Pro Gly Val Phe Glu Thr Val Glu Met
            1220                1225                1230
Leu Pro Ser Lys Ala Gly Ile Trp Arg Val Glu Cys Leu Ile Gly Glu
        1235                1240                1245
His Leu His Ala Gly Met Ser Thr Leu Phe Leu Val Tyr Ser Asn Lys
    1250                1255                1260
Cys Gln Thr Pro Leu Gly Met Ala Ser Gly His Ile Arg Asp Phe Gln
1265                1270                1275                1280
Ile Thr Ala Ser Gly Gln Tyr Gly Gln Trp Ala Pro Lys Leu Ala Arg
                1285                1290                1295
Leu His Tyr Ser Gly Ser Ile Asn Ala Trp Ser Thr Lys Glu Pro Phe
            1300                1305                1310
Ser Trp Ile Lys Val Asp Leu Leu Ala Pro Met Ile Ile His Gly Ile
        1315                1320                1325
Lys Thr Gln Gly Ala Arg Gln Lys Phe Ser Ser Leu Tyr Ile Ser Gln
    1330                1335                1340
Phe Ile Ile Met Tyr Ser Leu Asp Gly Lys Lys Trp Gln Thr Tyr Arg
1345                1350                1355                1360
Gly Asn Ser Thr Gly Thr Leu Met Val Phe Phe Gly Asn Val Asp Ser
                1365                1370                1375
Ser Gly Ile Lys His Asn Ile Phe Asn Pro Pro Ile Ile Ala Arg Tyr
            1380                1385                1390

Ile Arg Leu His Pro Thr His Tyr Ser Ile Arg Ser Thr Leu Arg Met
        1395                1400                1405
Glu Leu Met Gly Cys Asp Leu Asn Ser Cys Ser Met Pro Leu Gly Met
    1410                1415                1420
Glu Ser Lys Ala Ile Ser Asp Ala Gln Ile Thr Ala Ser Ser Tyr Phe
1425                1430                1435                1440
Thr Asn Met Phe Ala Thr Trp Ser Pro Ser Lys Ala Arg Leu His Leu
                1445                1450                1455
Gln Gly Arg Ser Asn Ala Trp Arg Pro Gln Val Asn Asn Pro Lys Glu
            1460                1465                1470
Trp Leu Gln Val Asp Phe Gln Lys Thr Met Lys Val Thr Gly Val Thr
        1475                1480                1485
Thr Gln Gly Val Lys Ser Leu Leu Thr Ser Met Tyr Val Lys Glu Phe
    1490                1495                1500
Leu Ile Ser Ser Ser Gln Asp Gly His Gln Trp Thr Leu Phe Phe Gln
1505                1510                1515                1520
Asn Gly Lys Val Lys Val Phe Gln Gly Asn Gln Asp Ser Phe Thr Pro
                1525                1530                1535
Val Val Asn Ser Leu Asp Pro Pro Leu Leu Thr Arg Tyr Leu Arg Ile
            1540                1545                1550
His Pro Gln Ser Trp Val His Gln Ile Ala Leu Arg Met Glu Val Leu
        1555                1560                1565
Gly Cys Glu Ala Gln Asp Leu Tyr Gly Ser Gly Gly Ser Gly Gly Ser
    1570                1575                1580
Gly Gly Ser Gly Ser Leu Ala Glu Ala Lys Glu Ala Ala Asn Ala Glu
1585                1590                1595                1600
Leu Asp Ser Tyr Gly Val Ser Asp Phe Tyr Lys Arg Leu Ile Asp Lys
                1605                1610                1615
Ala Lys Thr Val Glu Gly Val Glu Ala Leu Lys Asp Ala Ile Leu Ala
            1620                1625                1630
Ala Leu Pro Gly Ser Gly Gly Ser Gly Gly Ser Gly Gly Ser Gly Ser
        1635                1640                1645
Leu Ala Glu Ala Lys Glu Ala Ala Asn Ala Glu Leu Asp Ser Tyr Gly
    1650                1655                1660
Val Ser Asp Phe Tyr Lys Arg Leu Ile Asp Lys Ala Lys Thr Val Glu
1665                1670                1675                1680
Gly Val Glu Ala Leu Lys Asp Ala Ile Leu Ala Ala Leu Pro
                1685                1690

<210> 50
<211> 1629
<212> PRT
<213> Artificial Sequence

<220>
<223> AD2CD2woLG_SC aa

<400> 50
Met Gln Ile Glu Leu Ser Thr Cys Phe Phe Leu Cys Leu Leu Arg Phe
1               5                   10                  15
Cys Phe Ser Ala Thr Arg Arg Tyr Tyr Leu Gly Ala Val Glu Leu Ser
            20                  25                  30
Trp Asp Tyr Met Gln Ser Asp Leu Gly Glu Leu Pro Val Asp Ala Arg
        35                  40                  45
Phe Pro Pro Arg Val Pro Lys Ser Phe Pro Phe Asn Thr Ser Val Val
    50                  55                  60
Tyr Lys Lys Thr Leu Phe Val Glu Phe Thr Asp His Leu Phe Asn Ile
65                  70                  75                  80
Ala Lys Pro Arg Pro Pro Trp Met Gly Leu Leu Gly Pro Thr Ile Gln
                85                  90                  95
Ala Glu Val Tyr Asp Thr Val Val Ile Thr Leu Lys Asn Met Ala Ser
            100                 105                 110

His Pro Val Ser Leu His Ala Val Gly Val Ser Tyr Trp Lys Ala Ser
        115                 120                 125
Glu Gly Ala Glu Tyr Asp Asp Gln Thr Ser Gln Arg Glu Lys Glu Asp
    130                 135                 140
Asp Lys Val Phe Pro Gly Gly Ser His Thr Tyr Val Trp Gln Val Leu
145                 150                 155                 160
Lys Glu Asn Gly Pro Met Ala Ser Asp Pro Leu Cys Leu Thr Tyr Ser
                165                 170                 175
Tyr Leu Ser His Val Asp Leu Val Lys Asp Leu Asn Ser Gly Leu Ile
            180                 185                 190
Gly Ala Leu Leu Val Cys Arg Glu Gly Ser Leu Ala Lys Glu Lys Thr
        195                 200                 205
Gln Thr Leu His Lys Phe Ile Leu Leu Phe Ala Val Phe Asp Glu Gly
    210                 215                 220
Lys Ser Trp His Ser Glu Thr Lys Asn Ser Leu Met Gln Asp Arg Asp
225                 230                 235                 240
Ala Ala Ser Ala Arg Ala Trp Pro Lys Met His Thr Val Asn Gly Tyr
                245                 250                 255
Val Asn Arg Ser Leu Pro Gly Leu Ile Gly Cys His Arg Lys Ser Val
            260                 265                 270
Tyr Trp His Val Ile Gly Met Gly Thr Thr Pro Glu Val His Ser Ile
        275                 280                 285
Phe Leu Glu Gly His Thr Phe Leu Val Arg Asn His Arg Gln Ala Ser
    290                 295                 300
Leu Glu Ile Ser Pro Ile Thr Phe Leu Thr Ala Gln Thr Leu Leu Met
305                 310                 315                 320
Asp Leu Gly Gln Phe Leu Leu Phe Cys His Ile Ser Ser His Gln His
                325                 330                 335
Asp Gly Met Glu Ala Tyr Val Lys Val Asp Ser Cys Pro Glu Glu Pro
            340                 345                 350
Gln Leu Arg Met Lys Asn Asn Glu Glu Ala Glu Asp Tyr Asp Asp Asp
        355                 360                 365
Leu Thr Asp Ser Glu Met Asp Val Val Arg Phe Asp Asp Asp Asn Ser
    370                 375                 380
Pro Ser Phe Ile Gln Ile Arg Ser Val Ala Lys Lys His Pro Lys Thr
385                 390                 395                 400
Trp Val His Tyr Ile Ala Ala Glu Glu Glu Asp Trp Asp Tyr Ala Pro
                405                 410                 415
Leu Val Leu Ala Pro Asp Asp Arg Ser Tyr Lys Ser Gln Tyr Leu Asn
            420                 425                 430
Asn Gly Pro Gln Arg Ile Gly Arg Lys Tyr Lys Lys Val Arg Phe Met
        435                 440                 445
Ala Tyr Thr Asp Glu Thr Phe Lys Thr Arg Glu Ala Ile Gln His Glu
    450                 455                 460
Ser Gly Ile Leu Gly Pro Leu Leu Tyr Gly Glu Val Gly Asp Thr Leu
465                 470                 475                 480
Leu Ile Ile Phe Lys Asn Gln Ala Ser Arg Pro Tyr Asn Ile Tyr Pro
                485                 490                 495
His Gly Ile Thr Asp Val Arg Pro Leu Tyr Ser Arg Arg Leu Pro Lys
            500                 505                 510
Gly Val Lys His Leu Lys Asp Phe Pro Ile Leu Pro Gly Glu Ile Phe
        515                 520                 525
Lys Tyr Lys Trp Thr Val Thr Val Glu Asp Gly Pro Thr Lys Ser Asp
    530                 535                 540
Pro Arg Cys Leu Thr Arg Tyr Tyr Ser Ser Phe Val Asn Met Glu Arg
545                 550                 555                 560
Asp Leu Ala Ser Gly Leu Ile Gly Pro Leu Leu Ile Cys Tyr Lys Glu
                565                 570                 575
Ser Val Asp Gln Arg Gly Asn Gln Ile Met Ser Asp Lys Arg Asn Val
            580                 585                 590
Ile Leu Phe Ser Val Phe Asp Glu Asn Arg Ser Trp Tyr Leu Thr Glu
        595                 600                 605
Asn Ile Gln Arg Phe Leu Pro Asn Pro Ala Gly Val Gln Leu Glu Asp

610                 615                 620
Pro Glu Phe Gln Ala Ser Asn Ile Met His Ser Ile Asn Gly Tyr Val
625                 630                 635                 640
Phe Asp Ser Leu Gln Leu Ser Val Cys Leu His Glu Val Ala Tyr Trp
                645                 650                 655
Tyr Ile Leu Ser Ile Gly Ala Gln Thr Asp Phe Leu Ser Val Phe Phe
            660                 665                 670
Ser Gly Tyr Thr Phe Lys His Lys Met Val Tyr Glu Asp Thr Leu Thr
        675                 680                 685
Leu Phe Pro Phe Ser Gly Glu Thr Val Phe Met Ser Met Glu Asn Pro
    690                 695                 700
Gly Leu Trp Ile Leu Gly Cys His Asn Ser Asp Phe Arg Asn Arg Gly
705                 710                 715                 720
Met Thr Ala Leu Leu Lys Val Ser Ser Cys Asp Lys Asn Thr Gly Asp
                725                 730                 735
Tyr Tyr Glu Asp Ser Tyr Glu Asp Ile Ser Ala Tyr Leu Leu Ser Lys
            740                 745                 750
Asn Asn Ala Ile Glu Pro Arg Ser Phe Ser Gln Asn Pro Pro Val Leu
        755                 760                 765
Leu Ala Glu Ala Lys Glu Ala Ala Asn Ala Glu Leu Asp Ser Tyr Gly
    770                 775                 780
Val Ser Asp Phe Tyr Lys Arg Leu Ile Asp Lys Ala Lys Thr Val Glu
785                 790                 795                 800
Gly Val Glu Ala Leu Lys Asp Ala Ile Leu Ala Ala Leu Pro Leu Ala
                805                 810                 815
Glu Ala Lys Glu Ala Ala Asn Ala Glu Leu Asp Ser Tyr Gly Val Ser
            820                 825                 830
Asp Phe Tyr Lys Arg Leu Ile Asp Lys Ala Lys Thr Val Glu Gly Val
        835                 840                 845
Glu Ala Leu Lys Asp Ala Ile Leu Ala Ala Leu Pro Gln Ser Asp Gln
    850                 855                 860
Glu Glu Ile Asp Tyr Asp Asp Thr Ile Ser Val Glu Met Lys Lys Glu
865                 870                 875                 880
Asp Phe Asp Ile Tyr Asp Glu Asp Glu Asn Gln Ser Pro Arg Ser Phe
                885                 890                 895
Gln Lys Lys Thr Arg His Tyr Phe Ile Ala Ala Val Glu Arg Leu Trp
            900                 905                 910
Asp Tyr Gly Met Ser Ser Ser Pro His Val Leu Arg Asn Arg Ala Gln
        915                 920                 925
Ser Gly Ser Val Pro Gln Phe Lys Lys Val Val Phe Gln Glu Phe Thr
    930                 935                 940
Asp Gly Ser Phe Thr Gln Pro Leu Tyr Arg Gly Glu Leu Asn Glu His
945                 950                 955                 960
Leu Gly Leu Leu Gly Pro Tyr Ile Arg Ala Glu Val Glu Asp Asn Ile
                965                 970                 975
Met Val Thr Phe Arg Asn Gln Ala Ser Arg Pro Tyr Ser Phe Tyr Ser
            980                 985                 990
Ser Leu Ile Ser Tyr Glu Glu Asp Gln Arg Gln Gly Ala Glu Pro Arg
        995                 1000                1005
Lys Asn Phe Val Lys Pro Asn Glu Thr Lys Thr Tyr Phe Trp Lys Val
    1010                1015                1020
Gln His His Met Ala Pro Thr Lys Asp Glu Phe Asp Cys Lys Ala Trp
1025                1030                1035                1040
Ala Tyr Phe Ser Asp Val Asp Leu Glu Lys Asp Val His Ser Gly Leu
                1045                1050                1055
Ile Gly Pro Leu Leu Val Cys His Thr Asn Thr Leu Asn Pro Ala His
            1060                1065                1070
Gly Arg Gln Val Thr Val Gln Glu Phe Ala Leu Phe Phe Thr Ile Phe
        1075                1080                1085
Asp Glu Thr Lys Ser Trp Tyr Phe Thr Glu Asn Met Glu Arg Asn Cys
    1090                1095                1100
Arg Ala Pro Cys Asn Ile Gln Met Glu Asp Pro Thr Phe Lys Glu Asn
1105                1110                1115                1120

Tyr Arg Phe His Ala Ile Asn Gly Tyr Ile Met Asp Thr Leu Pro Gly
                1125                1130                1135
Leu Val Met Ala Gln Asp Gln Arg Ile Arg Trp Tyr Leu Leu Ser Met
            1140                1145                1150
Gly Ser Asn Glu Asn Ile His Ser Ile His Phe Ser Gly His Val Phe
        1155                1160                1165
Thr Val Arg Lys Lys Glu Glu Tyr Lys Met Ala Leu Tyr Asn Leu Tyr
    1170                1175                1180
Pro Gly Val Phe Glu Thr Val Glu Met Leu Pro Ser Lys Ala Gly Ile
1185                1190                1195                1200
Trp Arg Val Glu Cys Leu Ile Gly Glu His Leu His Ala Gly Met Ser
                1205                1210                1215
Thr Leu Phe Leu Val Tyr Ser Asn Lys Cys Gln Thr Pro Leu Gly Met
            1220                1225                1230
Ala Ser Gly His Ile Arg Asp Phe Gln Ile Thr Ala Ser Gly Gln Tyr
        1235                1240                1245
Gly Gln Trp Ala Pro Lys Leu Ala Arg Leu His Tyr Ser Gly Ser Ile
    1250                1255                1260
Asn Ala Trp Ser Thr Lys Glu Pro Phe Ser Trp Ile Lys Val Asp Leu
1265                1270                1275                1280
Leu Ala Pro Met Ile Ile His Gly Ile Lys Thr Gln Gly Ala Arg Gln
                1285                1290                1295
Lys Phe Ser Ser Leu Tyr Ile Ser Gln Phe Ile Ile Met Tyr Ser Leu
            1300                1305                1310
Asp Gly Lys Lys Trp Gln Thr Tyr Arg Gly Asn Ser Thr Gly Thr Leu
        1315                1320                1325
Met Val Phe Phe Gly Asn Val Asp Ser Ser Gly Ile Lys His Asn Ile
    1330                1335                1340
Phe Asn Pro Pro Ile Ile Ala Arg Tyr Ile Arg Leu His Pro Thr His
1345                1350                1355                1360
Tyr Ser Ile Arg Ser Thr Leu Arg Met Glu Leu Met Gly Cys Asp Leu
                1365                1370                1375
Asn Ser Cys Ser Met Pro Leu Gly Met Glu Ser Lys Ala Ile Ser Asp
            1380                1385                1390
Ala Gln Ile Thr Ala Ser Ser Tyr Phe Thr Asn Met Phe Ala Thr Trp
        1395                1400                1405
Ser Pro Ser Lys Ala Arg Leu His Leu Gln Gly Arg Ser Asn Ala Trp
    1410                1415                1420
Arg Pro Gln Val Asn Asn Pro Lys Glu Trp Leu Gln Val Asp Phe Gln
1425                1430                1435                1440
Lys Thr Met Lys Val Thr Gly Val Thr Thr Gln Gly Val Lys Ser Leu
                1445                1450                1455
Leu Thr Ser Met Tyr Val Lys Glu Phe Leu Ile Ser Ser Ser Gln Asp
            1460                1465                1470
Gly His Gln Trp Thr Leu Phe Phe Gln Asn Gly Lys Val Lys Val Phe
        1475                1480                1485
Gln Gly Asn Gln Asp Ser Phe Thr Pro Val Val Asn Ser Leu Asp Pro
    1490                1495                1500
Pro Leu Leu Thr Arg Tyr Leu Arg Ile His Pro Gln Ser Trp Val His
1505                1510                1515                1520
Gln Ile Ala Leu Arg Met Glu Val Leu Gly Cys Glu Ala Gln Asp Leu
                1525                1530                1535
Tyr Leu Ala Glu Ala Lys Glu Ala Ala Asn Ala Glu Leu Asp Ser Tyr
            1540                1545                1550
Gly Val Ser Asp Phe Tyr Lys Arg Leu Ile Asp Lys Ala Lys Thr Val
        1555                1560                1565
Glu Gly Val Glu Ala Leu Lys Asp Ala Ile Leu Ala Ala Leu Pro Leu
    1570                1575                1580
Ala Glu Ala Lys Glu Ala Ala Asn Ala Glu Leu Asp Ser Tyr Gly Val
1585                1590                1595                1600
Ser Asp Phe Tyr Lys Arg Leu Ile Asp Lys Ala Lys Thr Val Glu Gly
                1605                1610                1615
Val Glu Ala Leu Lys Asp Ala Ile Leu Ala Ala Leu Pro

1620                1625

<210> 51
<211> 1778
<212> PRT
<213> Artificial Sequence

<220>
<223> AbD2CD2_SC aa

<400> 51
Met Gln Ile Glu Leu Ser Thr Cys Phe Phe Leu Cys Leu Leu Arg Phe
1               5                   10                  15
Cys Phe Ser Ala Thr Arg Arg Tyr Tyr Leu Gly Ala Val Glu Leu Ser
            20                  25                  30
Trp Asp Tyr Met Gln Ser Asp Leu Gly Glu Leu Pro Val Asp Ala Arg
        35                  40                  45
Phe Pro Pro Arg Val Pro Lys Ser Phe Pro Phe Asn Thr Ser Val Val
    50                  55                  60
Tyr Lys Lys Thr Leu Phe Val Glu Phe Thr Asp His Leu Phe Asn Ile
65                  70                  75                  80
Ala Lys Pro Arg Pro Pro Trp Met Gly Leu Leu Gly Pro Thr Ile Gln
                85                  90                  95
Ala Glu Val Tyr Asp Thr Val Val Ile Thr Leu Lys Asn Met Ala Ser
            100                 105                 110
His Pro Val Ser Leu His Ala Val Gly Val Ser Tyr Trp Lys Ala Ser
        115                 120                 125
Glu Gly Ala Glu Tyr Asp Asp Gln Thr Ser Gln Arg Glu Lys Glu Asp
    130                 135                 140
Asp Lys Val Phe Pro Gly Gly Ser His Thr Tyr Val Trp Gln Val Leu
145                 150                 155                 160
Lys Glu Asn Gly Pro Met Ala Ser Asp Pro Leu Cys Leu Thr Tyr Ser
                165                 170                 175
Tyr Leu Ser His Val Asp Leu Val Lys Asp Leu Asn Ser Gly Leu Ile
            180                 185                 190
Gly Ala Leu Leu Val Cys Arg Glu Gly Ser Leu Ala Lys Glu Lys Thr
        195                 200                 205
Gln Thr Leu His Lys Phe Ile Leu Leu Phe Ala Val Phe Asp Glu Gly
    210                 215                 220
Lys Ser Trp His Ser Glu Thr Lys Asn Ser Leu Met Gln Asp Arg Asp
225                 230                 235                 240
Ala Ala Ser Ala Arg Ala Trp Pro Lys Met His Thr Val Asn Gly Tyr
                245                 250                 255
Val Asn Arg Ser Leu Pro Gly Leu Ile Gly Cys His Arg Lys Ser Val
            260                 265                 270
Tyr Trp His Val Ile Gly Met Gly Thr Thr Pro Glu Val His Ser Ile
        275                 280                 285
Phe Leu Glu Gly His Thr Phe Leu Val Arg Asn His Arg Gln Ala Ser
    290                 295                 300
Leu Glu Ile Ser Pro Ile Thr Phe Leu Thr Ala Gln Thr Leu Leu Met
305                 310                 315                 320
Asp Leu Gly Gln Phe Leu Leu Phe Cys His Ile Ser Ser His Gln His
                325                 330                 335
Asp Gly Met Glu Ala Tyr Val Lys Val Asp Ser Cys Pro Glu Glu Pro
            340                 345                 350
Gln Leu Arg Met Lys Asn Asn Glu Glu Ala Glu Asp Tyr Asp Asp Asp
        355                 360                 365
Leu Thr Asp Ser Glu Met Asp Val Val Arg Phe Asp Asp Asp Asn Ser
    370                 375                 380
Pro Ser Phe Ile Gln Ile Arg Ser Val Ala Lys Lys His Pro Lys Thr
385                 390                 395                 400
Trp Val His Tyr Ile Ala Ala Glu Glu Glu Asp Trp Asp Tyr Ala Pro

                405                 410                 415
Leu Val Leu Ala Pro Asp Asp Arg Ser Tyr Lys Ser Gln Tyr Leu Asn
            420                 425                 430
Asn Gly Pro Gln Arg Ile Gly Arg Lys Tyr Lys Lys Val Arg Phe Met
        435                 440                 445
Ala Tyr Thr Asp Glu Thr Phe Lys Thr Arg Glu Ala Ile Gln His Glu
    450                 455                 460
Ser Gly Ile Leu Gly Pro Leu Leu Tyr Gly Glu Val Gly Asp Thr Leu
465                 470                 475                 480
Leu Ile Ile Phe Lys Asn Gln Ala Ser Arg Pro Tyr Asn Ile Tyr Pro
                485                 490                 495
His Gly Ile Thr Asp Val Arg Pro Leu Tyr Ser Arg Arg Leu Pro Lys
            500                 505                 510
Gly Val Lys His Leu Lys Asp Phe Pro Ile Leu Pro Gly Glu Ile Phe
        515                 520                 525
Lys Tyr Lys Trp Thr Val Thr Val Glu Asp Gly Pro Thr Lys Ser Asp
    530                 535                 540
Pro Arg Cys Leu Thr Arg Tyr Tyr Ser Ser Phe Val Asn Met Glu Arg
545                 550                 555                 560
Asp Leu Ala Ser Gly Leu Ile Gly Pro Leu Leu Ile Cys Tyr Lys Glu
                565                 570                 575
Ser Val Asp Gln Arg Gly Asn Gln Ile Met Ser Asp Lys Arg Asn Val
            580                 585                 590
Ile Leu Phe Ser Val Phe Asp Glu Asn Arg Ser Trp Tyr Leu Thr Glu
        595                 600                 605
Asn Ile Gln Arg Phe Leu Pro Asn Pro Ala Gly Val Gln Leu Glu Asp
    610                 615                 620
Pro Glu Phe Gln Ala Ser Asn Ile Met His Ser Ile Asn Gly Tyr Val
625                 630                 635                 640
Phe Asp Ser Leu Gln Leu Ser Val Cys Leu His Glu Val Ala Tyr Trp
                645                 650                 655
Tyr Ile Leu Ser Ile Gly Ala Gln Thr Asp Phe Leu Ser Val Phe Phe
            660                 665                 670
Ser Gly Tyr Thr Phe Lys His Lys Met Val Tyr Glu Asp Thr Leu Thr
        675                 680                 685
Leu Phe Pro Phe Ser Gly Glu Thr Val Phe Met Ser Met Glu Asn Pro
    690                 695                 700
Gly Leu Trp Ile Leu Gly Cys His Asn Ser Asp Phe Arg Asn Arg Gly
705                 710                 715                 720
Met Thr Ala Leu Leu Lys Val Ser Ser Cys Asp Lys Asn Thr Gly Asp
                725                 730                 735
Tyr Tyr Glu Asp Ser Tyr Glu Asp Ile Ser Ala Tyr Leu Leu Ser Lys
            740                 745                 750
Asn Asn Ala Ile Glu Pro Arg Ser Phe Ser Gln Asn Pro Pro Val Leu
        755                 760                 765
Ser Lys Asn Asn Ala Ile Glu Pro Arg Ser Phe Ser Gln Asn Ser Arg
    770                 775                 780
His Pro Ser Leu Ala Glu Ala Lys Glu Ala Ala Asn Ala Glu Leu Asp
785                 790                 795                 800
Ser Tyr Gly Val Ser Asp Phe Tyr Lys Arg Leu Ile Asp Lys Ala Lys
                805                 810                 815
Thr Val Glu Gly Val Glu Ala Leu Lys Asp Ala Ile Leu Ala Ala Leu
            820                 825                 830
Pro Gly Ser Gly Gly Ser Gly Gly Ser Gly Gly Ser Gly Ser Gly Ser
        835                 840                 845
Lys Asn Asn Ala Ile Glu Pro Arg Ser Phe Ser Gln Asn Ser Arg His
    850                 855                 860
Pro Ser Gly Ser Gly Gly Ser Gly Gly Ser Gly Gly Ser Gly Ser Leu
865                 870                 875                 880
Ala Glu Ala Lys Glu Ala Ala Asn Ala Glu Leu Asp Ser Tyr Gly Val
                885                 890                 895
Ser Asp Phe Tyr Lys Arg Leu Ile Asp Lys Ala Lys Thr Val Glu Gly
            900                 905                 910

Val Glu Ala Leu Lys Asp Ala Ile Leu Ala Ala Leu Pro Ser Lys Asn
        915                 920                 925
Asn Ala Ile Glu Pro Arg Ser Phe Ser Gln Asn Ser Arg His Pro Ser
    930                 935                 940
Gln Ser Asp Gln Glu Glu Ile Asp Phe Asp Asp Thr Ile Ser Val Glu
945                 950                 955                 960
Met Lys Lys Glu Asp Phe Asp Ile Phe Asp Glu Asp Glu Asn Gln Ser
                965                 970                 975
Pro Arg Ser Phe Gln Lys Lys Thr Arg His Tyr Phe Ile Ala Ala Val
            980                 985                 990
Glu Arg Leu Trp Asp Tyr Gly Met Ser Ser Ser Pro His Val Leu Arg
        995                 1000                1005
Asn Arg Ala Gln Ser Gly Ser Val Pro Gln Phe Lys Lys Val Val Phe
    1010                1015                1020
Gln Glu Phe Thr Asp Gly Ser Phe Thr Gln Pro Leu Tyr Arg Gly Glu
1025                1030                1035                1040
Leu Asn Glu His Leu Gly Leu Leu Gly Pro Tyr Ile Arg Ala Glu Val
                1045                1050                1055
Glu Asp Asn Ile Met Val Thr Phe Arg Asn Gln Ala Ser Arg Pro Tyr
            1060                1065                1070
Ser Phe Tyr Ser Ser Leu Ile Ser Tyr Glu Glu Asp Gln Arg Gln Gly
        1075                1080                1085
Ala Glu Pro Arg Lys Asn Phe Val Lys Pro Asn Glu Thr Lys Thr Tyr
    1090                1095                1100
Phe Trp Lys Val Gln His His Met Ala Pro Thr Lys Asp Glu Phe Asp
1105                1110                1115                1120
Cys Lys Ala Trp Ala Tyr Phe Ser Asp Val Asp Leu Glu Lys Asp Val
                1125                1130                1135
His Ser Gly Leu Ile Gly Pro Leu Leu Val Cys His Thr Asn Thr Leu
            1140                1145                1150
Asn Pro Ala His Gly Arg Gln Val Thr Val Gln Glu Phe Ala Leu Phe
        1155                1160                1165
Phe Thr Ile Phe Asp Glu Thr Lys Ser Trp Tyr Phe Thr Glu Asn Met
    1170                1175                1180
Glu Arg Asn Cys Arg Ala Pro Cys Asn Ile Gln Met Glu Asp Pro Thr
1185                1190                1195                1200
Phe Lys Glu Asn Tyr Arg Phe His Ala Ile Asn Gly Tyr Ile Met Asp
                1205                1210                1215
Thr Leu Pro Gly Leu Val Met Ala Gln Asp Gln Arg Ile Arg Trp Tyr
            1220                1225                1230
Leu Leu Ser Met Gly Ser Asn Glu Asn Ile His Ser Ile His Phe Ser
        1235                1240                1245
Gly His Val Phe Thr Val Arg Lys Lys Glu Glu Tyr Lys Met Ala Leu
    1250                1255                1260
Tyr Asn Leu Tyr Pro Gly Val Phe Glu Thr Val Glu Met Leu Pro Ser
1265                1270                1275                1280
Lys Ala Gly Ile Trp Arg Val Glu Cys Leu Ile Gly Glu His Leu His
                1285                1290                1295
Ala Gly Met Ser Thr Leu Phe Leu Val Tyr Ser Asn Lys Cys Gln Thr
            1300                1305                1310
Pro Leu Gly Met Ala Ser Gly His Ile Arg Asp Phe Gln Ile Thr Ala
        1315                1320                1325
Ser Gly Gln Tyr Gly Gln Trp Ala Pro Lys Leu Ala Arg Leu His Tyr
    1330                1335                1340
Ser Gly Ser Ile Asn Ala Trp Ser Thr Lys Glu Pro Phe Ser Trp Ile
1345                1350                1355                1360
Lys Val Asp Leu Leu Ala Pro Met Ile Ile His Gly Ile Lys Thr Gln
                1365                1370                1375
Gly Ala Arg Gln Lys Phe Ser Ser Leu Tyr Ile Ser Gln Phe Ile Ile
            1380                1385                1390
Met Tyr Ser Leu Asp Gly Lys Lys Trp Gln Thr Tyr Arg Gly Asn Ser
        1395                1400                1405
Thr Gly Thr Leu Met Val Phe Phe Gly Asn Val Asp Ser Ser Gly Ile

1410                1415                1420
Lys His Asn Ile Phe Asn Pro Pro Ile Ile Ala Arg Tyr Ile Arg Leu
1425                1430                1435                1440
His Pro Thr His Tyr Ser Ile Arg Ser Thr Leu Arg Met Glu Leu Met
                1445                1450                1455
Gly Cys Asp Leu Asn Ser Cys Ser Met Pro Leu Gly Met Glu Ser Lys
            1460                1465                1470
Ala Ile Ser Asp Ala Gln Ile Thr Ala Ser Ser Tyr Phe Thr Asn Met
        1475                1480                1485
Phe Ala Thr Trp Ser Pro Ser Lys Ala Arg Leu His Leu Gln Gly Arg
    1490                1495                1500
Ser Asn Ala Trp Arg Pro Gln Val Asn Asn Pro Lys Glu Trp Leu Gln
1505                1510                1515                1520
Val Asp Phe Gln Lys Thr Met Lys Val Thr Gly Val Thr Thr Gln Gly
                1525                1530                1535
Val Lys Ser Leu Leu Thr Ser Met Tyr Val Lys Glu Phe Leu Ile Ser
            1540                1545                1550
Ser Ser Gln Asp Gly His Gln Trp Thr Leu Phe Phe Gln Asn Gly Lys
        1555                1560                1565
Val Lys Val Phe Gln Gly Asn Gln Asp Ser Phe Thr Pro Val Val Asn
    1570                1575                1580
Ser Leu Asp Pro Pro Leu Leu Thr Arg Tyr Leu Arg Ile His Pro Gln
1585                1590                1595                1600
Ser Trp Val His Gln Ile Ala Leu Arg Met Glu Val Leu Gly Cys Glu
                1605                1610                1615
Ala Gln Asp Leu Tyr Ser Lys Asn Asn Ala Ile Glu Pro Arg Ser Phe
            1620                1625                1630
Ser Gln Asn Ser Arg His Pro Ser Leu Ala Glu Ala Lys Glu Ala Ala
        1635                1640                1645
Asn Ala Glu Leu Asp Ser Tyr Gly Val Ser Asp Phe Tyr Lys Arg Leu
    1650                1655                1660
Ile Asp Lys Ala Lys Thr Val Glu Gly Val Glu Ala Leu Lys Asp Ala
1665                1670                1675                1680
Ile Leu Ala Ala Leu Pro Gly Ser Gly Gly Ser Gly Gly Ser Gly Gly
                1685                1690                1695
Ser Gly Ser Gly Ser Lys Asn Asn Ala Ile Glu Pro Arg Ser Phe Ser
            1700                1705                1710
Gln Asn Ser Arg His Pro Ser Gly Ser Gly Gly Ser Gly Gly Ser Gly
        1715                1720                1725
Gly Ser Gly Ser Leu Ala Glu Ala Lys Glu Ala Ala Asn Ala Glu Leu
    1730                1735                1740
Asp Ser Tyr Gly Val Ser Asp Phe Tyr Lys Arg Leu Ile Asp Lys Ala
1745                1750                1755                1760
Lys Thr Val Glu Gly Val Glu Ala Leu Lys Asp Ala Ile Leu Ala Ala
                1765                1770                1775
Leu Pro

<210> 52
<211> 4728
<212> DNA
<213> Artificial Sequence

<220>
<223> ADLCLD_SC na

<400> 52
atgcagatcg agctgtctac ctgcttcttc ctgtgcctgc tgcggttctg cttcagcgcc      60

acccggcggt actacctggg cgccgtggaa ctgagctggg actacatgca gagcgacctg     120

ggcgagctgc ccgtggacgc cagattcccc ccaagagtgc ccaagagctt ccccttcaac     180

acctccgtgg tgtacaagaa aaccctgttc gtcgagttca ccgaccacct gttcaatatc 240

gccaagccca gacccccctg gatgggcctg ctgggcccta caatccaggc cgaggtgtac 300

gacaccgtgg tcatcaccct gaagaacatg gccagccacc ccgtgtccct gcacgccgtg 360

ggcgtgtcct actggaaggc cagcgagggc gccgagtacg acgaccagac cagccagcgc 420

gagaaagagg acgacaaagt ctttcctggc ggcagccata cctacgtgtg gcaggtcctg 480

aaagaaaacg gccccatggc ctccgacccc ctgtgcctga cctacagcta cctgagccac 540

gtggacctgg tcaaggacct gaacagcggc ctgatcggcg ccctgctcgt gtgcagagag 600

ggcagcctgg ccaaagagaa aacccagacc ctgcacaagt tcatcctgct gttcgccgtg 660

ttcgacgagg gcaagagctg gcacagcgag acaaagaaca gcctgatgca ggaccgggac 720

gccgcctctg ccagagcctg gcctaagatg cacaccgtga acggctacgt gaacagaagc 780

ctgcccggac tgatcggctg ccaccggaag tccgtgtact ggcacgtgat cggcatgggt 840

accacccccg aggtgcacag catctttctg gaaggacaca ccttcctcgt gcggaaccac 900

cggcaggcca gcctggaaat cagccctatc accttcctga ccgcccagac actgctgatg 960

gacctgggcc agttcctgct gttttgccac atcagcagcc accagcacga cggcatggaa 1020

gcctacgtga aggtggacag ctgccccgag gaaccccagc tgcggatgaa gaacaacgag 1080

gaagccgagg actacgacga cgacctgacc gacagcgaga tggacgtcgt cagattcgat 1140

gacgacaaca gccccagctt catccagatc agaagcgtgg ccaagaagca ccccaagacc 1200

tgggtgcact atatcgccgc cgaggaagag gactgggact acgcccctct ggtgctggcc 1260

cccgacgaca gaagctacaa gagccagtac ctgaacaatg gcccccagcg gatcggccgg 1320

aagtacaaga aagtgcggtt catggcctac accgacgaga cattcaagac cagagaggcc 1380

atccagcacg agagcggcat cctgggcccc ctgctgtacg gcgaagtggg cgacaccctg 1440

ctgattatct tcaagaacca ggccagccgg ccctacaaca tctaccccca cggcatcacc 1500

gacgtgcggc ccctgtacag cagacggctg cccaagggcg tgaagcacct gaaggacttc 1560

cccatcctgc ccggggagat cttcaagtac aagtggaccg tgaccgtgga agatggcccc 1620

accaagagcg accccagatg cctgacccgg tactacagca gcttcgtgaa catggaacgg 1680

gacctggcct ccgggctgat cggccctctg ctgatctgct acaaagaaag cgtggaccag 1740

cggggcaacc agatcatgag cgacaagcgg aacgtgatcc tgttcagcgt gttcgatgag 1800

aacagaagct ggtatctgac cgagaatatc cagcggttcc tgcccaaccc tgccggcgtg 1860

cagctggaag atcccgagtt ccaggccagc aacatcatgc actccatcaa tggctacgtg 1920

ttcgacagcc tgcagctgag cgtgtgcctg cacgaggtgg cctactggta catcctgagc 1980

atcggcgccc agaccgactt cctgagcgtg ttcttcagcg gctacacctt caagcacaag 2040

atggtgtacg aggataccct gaccctgttc cccttctccg gcgaaaccgt gttcatgagc 2100

atggaaaacc ccggcctgtg gattctgggc tgccacaaca gcgacttccg gaaccggggc 2160

atgaccgccc tgctgaaggt gtccagctgc gacaagaaca ccggcgacta ctacgaggac 2220

agctacgagg atatcagcgc ctacctgctg agcaagaaca acgccatcga gccccggtcc 2280

ttcagccaga acccccccgt gctggccgag gccaaggagg ccgccaacgc cgagctggac 2340

agctacggcg tgagcgactt ctacaagcgc ctgatcgaca aggccaagac cgtggagggc 2400

gtggaggccc tgaaggacgc catcctggcc gccctgccca gcaaaaataa cgctatagag 2460

cccaggagct ttagtcagaa tagcaggcac ccgagtctgc agagcgacca ggaagagatc 2520

gactacgacg acaccatcag cgtcgagatg aagaaagagg acttcgacat ctacgacgag 2580

gacgagaacc agagcccccg gtccttccag aagaaaaccc ggcactactt tatcgccgcc 2640

gtggaacggc tgtgggacta cggcatgagc agcagccccc acgtgctgcg gaatagagcc 2700

cagagcggca gcgtgcccca gttcaagaaa gtggtgttcc aggagttcac cgacggcagc 2760

ttcacccagc ctctgtaccg gggcgagctg aacgagcacc tgggcctgct gggcccctac 2820

atccgggccg aggtggaaga taacatcatg gtcaccttcc ggaaccaggc cagccggccc 2880

tacagcttct acagcagcct gatctcctac gaagaggacc agcggcaggg cgccgagccc 2940

cggaagaact tcgtgaagcc caacgagaca aagacctact tctggaaggt gcagcaccac 3000

atggccccca ccaaggacga attcgactgc aaggcctggg cctacttcag cgacgtggac 3060

ctggaaaagg acgtgcacag cggcctgatc ggccccctgc tcgtgtgcca caccaacacc 3120

ctgaaccccg cccacggccg gcaggtcaca gtgcaggaat ttgccctgtt cttcaccatc 3180

ttcgacgaga caaagagctg gtacttcacc gagaacatgg aacggaactg cagagccccc 3240

tgcaacatcc agatggaaga tcccaccttc aaagagaact accggttcca cgccatcaac 3300

ggctacatca tggacaccct gcccggcctg gtcatggccc aggaccagcg gatccggtgg 3360

tatctgctga gcatgggcag caacgagaac atccacagca tccacttcag cggccacgtg 3420

ttcaccgtgc ggaagaaaga agagtacaag atggccctgt acaacctgta ccccggcgtg 3480

ttcgagacag tggaaatgct gcccagcaag gccggcatct ggcgggtgga atgtctgatc 3540

ggcgagcatc tgcacgccgg catgagcacc ctgtttctgg tgtacagcaa caagtgccag 3600

acccccctgg gcatggccag cggccacatc cgggatttcc agatcaccgc ctccggccag 3660

tacggccagt gggcccctaa actggcccgg ctgcactaca gcggcagcat caacgcctgg 3720

tccaccaaag agcccttcag ctggatcaag gtggacctgc tggcccccat gatcatccac 3780

ggcatcaaga cccagggcgc cagacagaag ttcagcagcc tgtacatcag ccagttcatc 3840

atcatgtaca gcctggacgg caagaagtgg cagacctacc ggggcaacag caccggcacc 3900

ctgatggtgt tcttcggcaa cgtggacagc agcggcatca agcacaacat cttcaacccc 3960

cccatcattg cccggtacat ccggctgcac cccacccact acagcatccg gtccaccctg 4020

cggatggaac tgatgggctg cgacctgaac agctgcagca tgcctctggg catggaaagc 4080

aaggccatca gcgacgccca gatcacagcc agcagctact tcaccaacat gttcgccacc 4140

tggtccccat ccaaggccag actgcatctg cagggcagaa gcaatgcctg gcggcctcag 4200

gtcaacaacc ccaaagagtg gctgcaggtc gacttccaga aaaccatgaa ggtcacaggc 4260

gtgaccaccc agggcgtgaa gtccctgctg acctctatgt acgtgaaaga gttcctgatc 4320

agcagcagcc aggacggcca ccagtggacc ctgttctttc agaacggcaa agtgaaggtg 4380

ttccagggca accaggacag cttcaccccc gtggtcaact ccctggaccc cccactgctg 4440

accagatacc tgcggatcca cccccagagc tgggtgcacc agatcgccct gagaatggaa 4500

gtgctgggat gcgaggccca ggatctgtac agcaagaaca acgccatcga gcccagaagc 4560

ttcagccaga acagccggca ccctagcctg gccgaggcca aagaagccgc caacgccgag 4620

ctggacagct acggcgtgtc cgacttctac aagcggctga tcgacaaggc caagaccgtg 4680

gaaggcgtgg aagccctgaa ggacgccatc ctggccgccc tgccctga 4728

<210> 53
<211> 5337
<212> DNA
<213> Artificial Sequence

<220>
<223> AD2CD2_SC na

<400> 53
atgcagatcg agctgtctac ctgcttcttc ctgtgcctgc tgcggttctg cttcagcgcc 60

acccggcggt actacctggg cgccgtggaa ctgagctggg actacatgca gagcgacctg 120

ggcgagctgc ccgtggacgc cagattcccc ccaagagtgc ccaagagctt ccccttcaac 180

acctccgtgg tgtacaagaa aaccctgttc gtcgagttca ccgaccacct gttcaatatc 240

gccaagccca gacccccctg gatgggcctg ctgggcccta caatccaggc cgaggtgtac 300

gacaccgtgg tcatcaccct gaagaacatg gccagccacc ccgtgtccct gcacgccgtg 360

ggcgtgtcct actggaaggc cagcgagggc gccgagtacg acgaccagac cagccagcgc 420

gagaaagagg acgacaaagt ctttcctggc ggcagccata cctacgtgtg gcaggtcctg 480

aaagaaaacg gccccatggc ctccgacccc ctgtgcctga cctacagcta cctgagccac 540

gtggacctgg tcaaggacct gaacagcggc ctgatcggcg ccctgctcgt gtgcagagag 600

ggcagcctgg ccaaagagaa aacccagacc ctgcacaagt tcatcctgct gttcgccgtg 660

ttcgacgagg gcaagagctg gcacagcgag acaaagaaca gcctgatgca ggaccgggac 720

gccgcctctg ccagagcctg gcctaagatg cacaccgtga acggctacgt gaacagaagc 780

ctgcccggac tgatcggctg ccaccggaag tccgtgtact ggcacgtgat cggcatgggt 840

accacccccg aggtgcacag catctttctg gaaggacaca ccttcctcgt gcggaaccac 900

cggcaggcca gcctggaaat cagccctatc accttcctga ccgcccagac actgctgatg 960

gacctgggcc agttcctgct gttttgccac atcagcagcc accagcacga cggcatggaa 1020

gcctacgtga aggtggacag ctgccccgag gaaccccagc tgcggatgaa gaacaacgag 1080

gaagccgagg actacgacga cgacctgacc gacagcgaga tggacgtcgt cagattcgat 1140

gacgacaaca gccccagctt catccagatc agaagcgtgg ccaagaagca ccccaagacc 1200

tgggtgcact atatcgccgc cgaggaagag gactgggact acgcccctct ggtgctggcc 1260

cccgacgaca gaagctacaa gagccagtac ctgaacaatg gcccccagcg gatcggccgg 1320

aagtacaaga aagtgcggtt catggcctac accgacgaga cattcaagac cagagaggcc 1380

atccagcacg agagcggcat cctgggcccc ctgctgtacg gcgaagtggg cgacaccctg 1440

ctgattatct tcaagaacca ggccagccgg ccctacaaca tctaccccca cggcatcacc 1500

gacgtgcggc ccctgtacag cagacggctg cccaagggcg tgaagcacct gaaggacttc 1560

cccatcctgc ccggggagat cttcaagtac aagtggaccg tgaccgtgga agatggcccc 1620

accaagagcg accccagatg cctgacccgg tactacagca gcttcgtgaa catggaacgg 1680

gacctggcct ccgggctgat cggccctctg ctgatctgct acaaagaaag cgtggaccag 1740

cggggcaacc agatcatgag cgacaagcgg aacgtgatcc tgttcagcgt gttcgatgag 1800

aacagaagct ggtatctgac cgagaatatc cagcggttcc tgcccaaccc tgccggcgtg 1860

cagctggaag atcccgagtt ccaggccagc aacatcatgc actccatcaa tggctacgtg 1920

ttcgacagcc tgcagctgag cgtgtgcctg cacgaggtgg cctactggta catcctgagc 1980

atcggcgccc agaccgactt cctgagcgtg ttcttcagcg gctacacctt caagcacaag 2040

atggtgtacg aggataccct gaccctgttc cccttctccg gcgaaaccgt gttcatgagc 2100

atggaaaacc ccggcctgtg gattctgggc tgccacaaca gcgacttccg gaaccggggc 2160

atgaccgccc tgctgaaggt gtccagctgc gacaagaaca ccggcgacta ctacgaggac 2220

agctacgagg atatcagcgc ctacctgctg agcaagaaca acgccatcga gcctcggagc 2280

ttcagccaga atcctcctgt gctgtccaaa aacaatgcca ttgagccccg gtcattctcc 2340

cagaactcta gacacccaag cctggccgaa gccaaagaag ccgctaacgc cgagctggat 2400

agctacggcg tgtccgactt ctacaagcgg ctgatcgata aggccaagac cgtggaaggc 2460

gtggaagccc tgaaagatgc tattctggcc gctctgcctg gaagcggagg tagcggagga 2520

tctggcggtt ctggcagcgg atctaagaac aatgctatcg aaccccgctc ctttagccag 2580

aacagcagac acccttctgg aagtggtggt tctggtggat ccggcggatc tggatctctg 2640

gcagaggcta aagaggccgc caacgctgaa ctcgactcct atggggtgtc cgatttttac 2700

aaacgcctca ttgacaaggc caaaacagtc gagggcgtcg aggctctgaa ggatgccatt 2760

cttgctgccc tgccatctaa aaacaacgca atagagccca gatccttcag tcagaacagt 2820

cggcacccca gccagagcga ccaagaggaa atcgactacg acgacaccat cagcgtcgag 2880

atgaagaaag aggacttcga catctacgac gaggacgaga atcagagccc cagaagcttc 2940

cagaaaaaga cccggcacta ctttatcgcc gccgtggaaa gactgtggga ctacggaatg 3000

agcagcagcc ctcacgtgct gagaaataga gcacagagcg gcagcgtgcc ccagttcaag 3060

aaagtggtgt tccaagagtt caccgacggc agcttcaccc agcctctgta tagaggcgag 3120

ctgaatgagc acctgggcct gctgggacct tatatcagag ccgaggtgga agataacatc 3180

atggtcacct tccggaacca ggccagcaga ccctacagct tctacagcag cctgatcagc 3240

tacgaagagg accagagaca gggcgccgag cctagaaaga acttcgtgaa gcccaacgag 3300

acaaagacct acttttggaa ggtgcagcac cacatggccc ctaccaagga cgaattcgat 3360

tgcaaggcct gggcctactt cagcgacgtg gacctggaaa aggatgtgca ctctggcctg 3420

atcggccctc tgctcgtgtg tcacaccaac acactgaacc ccgctcacgg cagacaagtg 3480

accgtgcaag agttcgccct gttcttcacc atcttcgacg agacaaagag ctggtacttc 3540

accgagaaca tggaacggaa ctgcagagcc ccttgcaaca tccagatgga agatcccacc 3600

ttcaaagaga actaccggtt ccacgccatc aacggctaca tcatggatac cctgcctggc 3660

ctggttatgg cccaggacca gagaatcaga tggtatctgc tgagcatggg cagcaacgag 3720

aacatccaca gcatccactt cagcggccac gtgttcaccg tgcggaagaa agaagagtac 3780

aagatggccc tgtacaatct gtaccccggc gtgttcgaga cagtggaaat gctgccttct 3840

aaggccggca tttggagagt ggaatgtctg atcggagagc atctgcacgc cggcatgagc 3900

accctgtttc tggtgtacag caacaagtgt cagacccctc tcggcatggc ctccggccac 3960

atcagagatt ttcagatcac agccagcggc cagtatggcc agtgggctcc taaactggcc 4020

agactgcact acagcggcag catcaatgcc tggtccacca aagagccctt cagctggatt 4080

aaggtggacc tgctggcccc tatgatcatc cacggcatca aaacacaggg cgccagacag 4140

aagttcagca gcctgtacat cagccagttc atcatcatgt acagcctgga cggcaagaag 4200

tggcagacct acagaggcaa tagcaccggc acactgatgg tgttcttcgg caatgtggac 4260

agcagcggca tcaagcacaa catcttcaac cctccaatca ttgcccggta catccggctg 4320

caccccacac actacagcat ccggtctacc ctgagaatgg aactgatggg ctgcgacctg 4380

aacagctgta gcatgcccct cggaatggaa agcaaggcca tcagcgacgc ccagatcacc 4440

gcctccagct acttcaccaa catgttcgcc acttggagcc cctccaaagc cagactccat 4500

ctgcaaggca gatctaacgc ttggaggccc caagtgaaca accccaaaga gtggctgcag 4560

gtcgacttcc agaaaaccat gaaggtcaca ggcgtgacaa cccagggcgt gaagtctctg 4620

ctgacctcta tgtacgtgaa agagttcctg atcagcagca gccaggacgg ccaccagtgg 4680

accctgtttt tccagaacgg caaagtgaag gtgttccagg gcaaccagga cagcttcacc 4740

cctgtggtca atagcctgga tcctccactg ctgaccagat acctgcggat tcaccctcag 4800

agctgggtgc accagatcgc tctcagaatg gaagtgctgg gatgtgaagc ccaggatctg 4860

tactccaaga acaacgccat cgagcccaga agcttcagcc agaactctag acacccaagc 4920

ctggccgaag ccaaagaagc cgctaacgcc gagctggata gctacggcgt gtccgacttc 4980

tacaagcggc tgatcgataa ggccaagacc gtggaaggcg tggaagccct gaaagatgcc 5040

attctggctg ctctgcctgg aagcggagga agtggtggat ctggcggttc tggctccggc 5100

agcaaaaaca atgccattga gcctcggagc ttctcccaga acagcagaca tccttctgga 5160

tcaggcggta gcggaggatc cggcggatct ggatctctgg ctgaggctaa agaggccgcc 5220

aacgctgaac tcgactccta tggggtgtcc gatttttaca aacgcctcat tgacaaggcc 5280

aaaacagtcg agggcgtcga ggctctgaag gatgctattc ttgccgcact gccctga 5337

<210> 54
<211> 5085
<212> DNA
<213> Artificial Sequence

<220>
<223> AD2CD2woL_SC na

<400> 54
atgcagatcg agctgtctac ctgcttcttc ctgtgcctgc tgcggttctg cttcagcgcc 60

acccggcggt actacctggg cgccgtggaa ctgagctggg actacatgca gagcgacctg 120

ggcgagctgc ccgtggacgc cagattcccc ccaagagtgc ccaagagctt ccccttcaac 180

acctccgtgg tgtacaagaa aaccctgttc gtcgagttca ccgaccacct gttcaatatc 240

gccaagccca gacccccctg gatgggcctg ctgggcccta caatccaggc cgaggtgtac 300

gacaccgtgg tcatcaccct gaagaacatg gccagccacc ccgtgtccct gcacgccgtg 360

ggcgtgtcct actggaaggc cagcgagggc gccgagtacg acgaccagac cagccagcgc 420

gagaaagagg acgacaaagt ctttcctggc ggcagccata cctacgtgtg gcaggtcctg 480

aaagaaaacg gccccatggc ctccgacccc ctgtgcctga cctacagcta cctgagccac 540

gtggacctgg tcaaggacct gaacagcggc ctgatcggcg ccctgctcgt gtgcagagag 600

ggcagcctgg ccaaagagaa aacccagacc ctgcacaagt tcatcctgct gttcgccgtg 660

ttcgacgagg gcaagagctg gcacagcgag acaaagaaca gcctgatgca ggaccgggac 720

gccgcctctg ccagagcctg gcctaagatg cacaccgtga acggctacgt gaacagaagc 780

ctgcccggac tgatcggctg ccaccggaag tccgtgtact ggcacgtgat cggcatgggt 840

accacccccg aggtgcacag catctttctg gaaggacaca ccttcctcgt gcggaaccac 900

cggcaggcca gcctggaaat cagccctatc accttcctga ccgcccagac actgctgatg 960

gacctgggcc agttcctgct gttttgccac atcagcagcc accagcacga cggcatggaa 1020

gcctacgtga aggtggacag ctgccccgag gaaccccagc tgcggatgaa gaacaacgag 1080

gaagccgagg actacgacga cgacctgacc gacagcgaga tggacgtcgt cagattcgat 1140

gacgacaaca gccccagctt catccagatc agaagcgtgg ccaagaagca ccccaagacc 1200

tgggtgcact atatcgccgc cgaggaagag gactgggact acgcccctct ggtgctggcc 1260

cccgacgaca gaagctacaa gagccagtac ctgaacaatg gcccccagcg gatcggccgg 1320

aagtacaaga aagtgcggtt catggcctac accgacgaga cattcaagac cagagaggcc 1380

atccagcacg agagcggcat cctgggcccc ctgctgtacg gcgaagtggg cgacaccctg 1440

ctgattatct tcaagaacca ggccagccgg ccctacaaca tctaccccca cggcatcacc 1500

gacgtgcggc ccctgtacag cagacggctg cccaagggcg tgaagcacct gaaggacttc 1560

cccatcctgc ccggggagat cttcaagtac aagtggaccg tgaccgtgga agatggcccc 1620

accaagagcg accccagatg cctgacccgg tactacagca gcttcgtgaa catggaacgg 1680

gacctggcct ccgggctgat cggccctctg ctgatctgct acaaagaaag cgtggaccag 1740

cggggcaacc agatcatgag cgacaagcgg aacgtgatcc tgttcagcgt gttcgatgag 1800

aacagaagct ggtatctgac cgagaatatc cagcggttcc tgcccaaccc tgccggcgtg 1860

cagctggaag atcccgagtt ccaggccagc aacatcatgc actccatcaa tggctacgtg 1920

ttcgacagcc tgcagctgag cgtgtgcctg cacgaggtgg cctactggta catcctgagc 1980

atcggcgccc agaccgactt cctgagcgtg ttcttcagcg gctacacctt caagcacaag 2040

atggtgtacg aggataccct gaccctgttc cccttctccg gcgaaaccgt gttcatgagc 2100

atggaaaacc ccggcctgtg gattctgggc tgccacaaca gcgacttccg gaaccggggc 2160

atgaccgccc tgctgaaggt gtccagctgc gacaagaaca ccggcgacta ctacgaggac 2220

agctacgagg atatcagcgc ctacctgctg agcaagaaca acgccatcga gccccggtcc 2280

ttcagccaga acccccccgt gctgggaagc ggaggatctg gcggctcagg cggatctgga 2340

tctctggccg aagccaaaga agccgccaac gccgagctgg acagctatgg cgtgtccgac 2400

ttctacaagc ggctgatcga caaggccaag acagtggaag gcgtggaagc tctgaaggat 2460

gccatcctgg ccgctctgcc tggaagtggg ggatcaggcg gcagcggagg aagtggaagt 2520

ctggctgagg ctaaagaggc tgctaacgct gaactggact cttacggggt gtccgatttt 2580

tacaaaagac tgattgataa ggctaaaact gtggaagggg tggaagccct gaaagacgct 2640

attctggctg ccctgcctgg ctccgggggc tctggcggaa gtggcggcag tggatctcag 2700

agcgaccagg aagagatcga ctacgacgac accatcagcg tcgagatgaa gaaagaggac 2760

ttcgacatct acgacgagga cgagaaccag agcccccggt ccttccagaa gaaaacccgg 2820

cactacttta tcgccgccgt ggaacggctg tgggactacg gcatgagcag cagcccccac 2880

gtgctgcgga atagagccca gagcggcagc gtgccccagt tcaagaaagt ggtgttccag 2940

gagttcaccg acggcagctt cacccagcct ctgtaccggg gcgagctgaa cgagcacctg 3000

ggcctgctgg gcccctacat ccgggccgag gtggaagata acatcatggt caccttccgg 3060

aaccaggcca gccggcccta cagcttctac agcagcctga tctcctacga agaggaccag 3120

cggcagggcg ccgagccccg gaagaacttc gtgaagccca acgagacaaa gacctacttc 3180

tggaaggtgc agcaccacat ggcccccacc aaggacgaat tcgactgcaa ggcctgggcc 3240

tacttcagcg acgtggacct ggaaaaggac gtgcacagcg gcctgatcgg ccccctgctc 3300

gtgtgccaca ccaacaccct gaaccccgcc cacggccggc aggtcacagt gcaggaattt 3360

gccctgttct tcaccatctt cgacgagact aagagctggt acttcaccga gaacatggaa 3420

cggaactgca gagccccctg caacatccag atggaagatc ccaccttcaa agagaactac 3480

cggttccacg ccatcaatgg ctacatcatg gacaccctgc ccggcctggt catggcccag 3540

gaccagagaa tccggtggta tctgctgagc atgggcagca acgagaacat ccacagcatc 3600

cacttcagcg gccacgtgtt caccgtgcgg aagaaagaag agtacaagat ggccctgtac 3660

aacctgtacc ccggcgtgtt cgaaacagtg gaaatgctgc ccagcaaggc cggcatctgg 3720

cgggtggaat gtctgatcgg cgagcatctg cacgccggca tgtccaccct gtttctggtg 3780

tacagcaaca agtgccagac ccccctgggc atggccagcg gccacatccg ggatttccag 3840

atcaccgcct ccggccagta cggccagtgg gcccctaaac tggcccggct gcactacagc 3900

ggcagcatca acgcctggtc caccaaagag cccttcagct ggatcaaggt ggacctgctg 3960

gcccccatga ttatccacgg catcaagaca cagggcgcca gacagaagtt cagcagcctg 4020

tacatcagcc agttcatcat catgtacagc ctggatggca agaagtggca gacctaccgg 4080

ggcaacagca ccggcaccct gatggtgttc ttcggcaacg tggacagcag cggcatcaag 4140

cacaacatct tcaacccccc catcattgcc cggtacatcc ggctgcaccc cacccactac 4200

agcatccggt ccaccctgcg gatggaactg atgggctgcg acctgaacag ctgctccatg 4260

cctctgggca tggaaagcaa ggccatcagc gacgcccaga tcacagccag cagctacttc 4320

accaacatgt tcgccacctg gtccccatcc aaggccagac tgcatctgca gggcagaagc 4380

aatgcctggc ggcctcaggt caacaacccc aaagaatggc tccaggtgga cttccagaaa 4440

accatgaagg tcacaggcgt gaccacccag ggcgtgaagt ccctgctgac ctctatgtac 4500

gtgaaagagt tcctgatctc cagcagccag gacggccacc agtggaccct gttctttcag 4560

aacggcaaag tgaaagtgtt ccagggcaac caggactcct tcacccccgt ggtcaactcc 4620

ctggaccccc cactgctgac cagatacctg agaatccacc cccagagctg ggtgcaccag 4680

atcgccctga gaatggaagt gctgggatgc gaggcccagg atctgtacgg atctgggggc 4740

agtggcggat ccgggggaag cggctcactg gccgaggcaa aagaggccgc aaacgccgaa 4800

ctggattcat atggcgtgtc agatttctat aagagactga ttgacaaagc aaagaccgtg 4860

gaaggggtgg aagcactgaa ggacgcaatc ctggcagccc tgcccggatc tggcggaagc 4920

ggggggagtg gcggctctgg aagcctggca gaagcaaaag aggcagctaa cgcagagctg 4980

gatagttacg gcgtgtcaga cttttacaaa cgcctgattg acaaggccaa aaccgtggaa 5040

ggcgtggaag cactgaaaga tgcaattctg gccgcactgc cctga 5085

<210> 55
<211> 4890
<212> DNA
<213> Artificial Sequence

<220>
<223> AD2CD2woLG_SC na

<400> 55
atgcagatcg agctgtctac ctgcttcttc ctgtgcctgc tgcggttctg cttcagcgcc 60

acccggcggt actacctggg cgccgtggaa ctgagctggg actacatgca gagcgacctg 120

ggcgagctgc ccgtggacgc cagattcccc ccaagagtgc ccaagagctt ccccttcaac 180

acctccgtgg tgtacaagaa aaccctgttc gtcgagttca ccgaccacct gttcaatatc 240

gccaagccca gacccccctg gatgggcctg ctgggcccta caatccaggc cgaggtgtac 300

gacaccgtgg tcatcaccct gaagaacatg gccagccacc ccgtgtccct gcacgccgtg 360

ggcgtgtcct actggaaggc cagcgagggc gccgagtacg acgaccagac cagccagcgc 420

gagaaagagg acgacaaagt ctttcctggc ggcagccata cctacgtgtg gcaggtcctg 480

aaagaaaacg gccccatggc ctccgacccc ctgtgcctga cctacagcta cctgagccac 540

gtggacctgg tcaaggacct gaacagcggc ctgatcggcg ccctgctcgt gtgcagagag 600

ggcagcctgg ccaaagagaa aacccagacc ctgcacaagt tcatcctgct gttcgccgtg 660

ttcgacgagg gcaagagctg gcacagcgag acaaagaaca gcctgatgca ggaccgggac 720

gccgcctctg ccagagcctg gcctaagatg cacaccgtga acggctacgt gaacagaagc 780

ctgcccggac tgatcggctg ccaccggaag tccgtgtact ggcacgtgat cggcatgggt 840

accacccccg aggtgcacag catctttctg gaaggacaca ccttcctcgt gcggaaccac 900

cggcaggcca gcctggaaat cagccctatc accttcctga ccgcccagac actgctgatg 960

gacctgggcc agttcctgct gttttgccac atcagcagcc accagcacga cggcatggaa 1020

gcctacgtga aggtggacag ctgccccgag gaaccccagc tgcggatgaa gaacaacgag 1080

gaagccgagg actacgacga cgacctgacc gacagcgaga tggacgtcgt cagattcgat 1140

gacgacaaca gccccagctt catccagatc agaagcgtgg ccaagaagca ccccaagacc 1200

tgggtgcact atatcgccgc cgaggaagag gactgggact acgcccctct ggtgctggcc 1260

cccgacgaca gaagctacaa gagccagtac ctgaacaatg gcccccagcg gatcggccgg 1320

aagtacaaga aagtgcggtt catggcctac accgacgaga cattcaagac cagagaggcc 1380

atccagcacg agagcggcat cctgggcccc ctgctgtacg gcgaagtggg cgacaccctg 1440

ctgattatct tcaagaacca ggccagccgg ccctacaaca tctaccccca cggcatcacc 1500

gacgtgcggc ccctgtacag cagacggctg cccaagggcg tgaagcacct gaaggacttc 1560

cccatcctgc ccggggagat cttcaagtac aagtggaccg tgaccgtgga agatggcccc 1620

accaagagcg accccagatg cctgacccgg tactacagca gcttcgtgaa catggaacgg 1680

gacctggcct ccgggctgat cggccctctg ctgatctgct acaaagaaag cgtggaccag 1740

cggggcaacc agatcatgag cgacaagcgg aacgtgatcc tgttcagcgt gttcgatgag 1800

aacagaagct ggtatctgac cgagaatatc cagcggttcc tgcccaaccc tgccggcgtg 1860

cagctggaag atcccgagtt ccaggccagc aacatcatgc actccatcaa tggctacgtg 1920

ttcgacagcc tgcagctgag cgtgtgcctg cacgaggtgg cctactggta catcctgagc 1980

atcggcgccc agaccgactt cctgagcgtg ttcttcagcg gctacacctt caagcacaag 2040

atggtgtacg aggataccct gaccctgttc cccttctccg gcgaaaccgt gttcatgagc 2100

atggaaaacc ccggcctgtg gattctgggc tgccacaaca gcgacttccg gaaccggggc 2160

atgaccgccc tgctgaaggt gtccagctgc gacaagaaca ccggcgacta ctacgaggac 2220

agctacgagg atatcagcgc ctacctgctg agcaagaaca acgccatcga gccccggtcc 2280

ttcagccaga acccccccgt gctgctggcc gaagccaaag aagccgccaa cgccgagctg 2340

gacagctatg gcgtgtccga cttctacaag cggctgatcg acaaggccaa gacagtggaa 2400

ggcgtggaag ctctgaagga tgccatcctg gccgctctgc ctctggccga ggctaaagag 2460

gctgctaacg cagaactgga ctcttacggg gtgtccgatt tttacaaaag actgattgat 2520

aaggctaaaa ctgtggaagg ggtggaagcc ctgaaagacg ctattctggc tgccctgccc 2580

cagagcgacc aggaagagat cgactacgac gacaccatca gcgtcgagat gaagaaagag 2640

gacttcgaca tctacgacga ggacgagaac cagagccccc ggtccttcca gaagaaaacc 2700

cggcactact ttatcgccgc cgtggaacgg ctgtgggact acggcatgag cagcagcccc 2760

cacgtgctgc ggaatagagc ccagagcggc agcgtgcccc agttcaagaa agtggtgttc 2820

caggagttca ccgacggcag cttcacccag cctctgtacc ggggcgagct gaacgagcac 2880

ctgggcctgc tgggccccta catccgggcc gaggtggaag ataacatcat ggtcaccttc 2940

cggaaccagg ccagccggcc ctacagcttc tacagcagcc tgatctccta cgaagaggac 3000

cagcggcagg gcgccgagcc ccggaagaac ttcgtgaagc ccaacgagac caagacctac 3060

ttctggaagg tgcagcacca catggccccc accaaggacg aattcgactg caaggcctgg 3120

gcctacttca gcgacgtgga cctggaaaag gacgtgcaca gcggcctgat cggccccctg 3180

ctcgtgtgcc acaccaacac cctgaacccc gcccacggcc ggcaggtcac agtgcaggaa 3240

tttgccctgt tcttcaccat cttcgacgag actaagagct ggtacttcac cgagaacatg 3300

gaacggaact gcagagcccc ctgcaacatc cagatggaag atcccacctt caaagagaac 3360

taccggttcc acgccatcaa tggctacatc atggacaccc tgcccggcct ggtcatggcc 3420

caggaccaga gaatccggtg gtatctgctg agcatgggca gcaacgagaa catccacagc 3480

atccacttca gcggccacgt gttcaccgtg cggaagaaag aagagtacaa gatggccctg 3540

tacaacctgt accccggcgt gttcgaaaca gtggaaatgc tgcccagcaa ggccggcatc 3600

tggcgggtgg aatgtctgat cggcgagcat ctgcacgccg gcatgtccac cctgtttctg 3660

gtgtacagca acaagtgcca gacccccctg ggcatggcca gcggccacat ccgggatttc 3720

cagatcaccg cctccggcca gtacggccag tgggccccta aactggcccg gctgcactac 3780

agcggcagca tcaacgcctg gtccaccaaa gagcccttca gctggatcaa ggtggacctg 3840

ctggccccca tgattatcca cggcatcaag acacagggcg ccagacagaa gttcagcagc 3900

ctgtacatca gccagttcat catcatgtac agcctggatg gcaagaagtg gcagacctac 3960

cggggcaaca gcaccggcac cctgatggtg ttcttcggca acgtggacag cagcggcatc 4020

aagcacaaca tcttcaaccc ccccatcatt gcccggtaca tccggctgca ccccacccac 4080

tacagcatcc ggtccaccct gcggatggaa ctgatgggct gcgacctgaa cagctgctcc 4140

atgcctctgg gcatggaaag caaggccatc agcgacgccc agatcacagc cagcagctac 4200

ttcaccaaca tgttcgccac ctggtcccca tccaaggcca gactgcatct gcagggcaga 4260

agcaatgcct ggcggcctca ggtcaacaac cccaaagaat ggctccaggt ggacttccag 4320

aaaaccatga aggtcacagg cgtgaccacc cagggcgtga agtccctgct gacctctatg 4380

tacgtgaaag agttcctgat ctccagcagc caggacggcc accagtggac cctgttcttt 4440

cagaacggca aagtgaaagt gttccagggc aaccaggact ccttcacccc cgtggtcaac 4500

tccctggacc ccccactgct gaccagatac ctgagaatcc acccccagag ctgggtgcac 4560

cagatcgccc tgagaatgga agtgctggga tgcgaggccc aggatctgta cctggctgag 4620

gcaaaagaag cagcaaacgc cgaactggat tcctacggcg tgtcagactt ctataagaga 4680

ctgattgaca aagccaaaac cgtggaaggc gtggaagcac tgaaggacgc aattctggcc 4740

gcactgcccc tggcagaagc aaaagaggcc gctaacgctg agctggattc atatggggtg 4800

tcagatttct acaaacgcct gatcgacaaa gcaaaaacag tggaaggggt ggaagccctg 4860

aaggacgcca tcctggcagc cctgccatga 4890

<210> 56
<211> 5337
<212> DNA
<213> Artificial Sequence

<220>
<223> AbD2CD2_SC na

<400> 56
atgcagatcg agctgtctac ctgcttcttc ctgtgcctgc tgcggttctg cttcagcgcc 60

acccggcggt actacctggg cgccgtggaa ctgagctggg actacatgca gagcgacctg 120

ggcgagctgc ccgtggacgc cagattcccc ccaagagtgc ccaagagctt ccccttcaac 180

acctccgtgg tgtacaagaa aaccctgttc gtcgagttca ccgaccacct gttcaatatc 240

gccaagccca gacccccctg gatgggcctg ctgggcccta caatccaggc cgaggtgtac 300

gacaccgtgg tcatcaccct gaagaacatg gccagccacc ccgtgtccct gcacgccgtg 360

ggcgtgtcct actggaaggc cagcgagggc gccgagtacg acgaccagac cagccagcgc 420

gagaaagagg acgacaaagt ctttcctggc ggcagccata cctacgtgtg gcaggtcctg 480

aaagaaaacg gccccatggc ctccgacccc ctgtgcctga cctacagcta cctgagccac 540

gtggacctgg tcaaggacct gaacagcggc ctgatcggcg ccctgctcgt gtgcagagag 600

ggcagcctgg ccaaagagaa aacccagacc ctgcacaagt tcatcctgct gttcgccgtg 660

ttcgacgagg gcaagagctg gcacagcgag acaaagaaca gcctgatgca ggaccgggac 720

gccgcctctg ccagagcctg gcctaagatg cacaccgtga acggctacgt gaacagaagc 780

ctgcccggac tgatcggctg ccaccggaag tccgtgtact ggcacgtgat cggcatgggt 840

accacccccg aggtgcacag catctttctg gaaggacaca ccttcctcgt gcggaaccac 900

cggcaggcca gcctggaaat cagccctatc accttcctga ccgcccagac actgctgatg 960

gacctgggcc agttcctgct gttttgccac atcagcagcc accagcacga cggcatggaa 1020

gcctacgtga aggtggacag ctgccccgag gaaccccagc tgcggatgaa gaacaacgag 1080

gaagccgagg actacgacga cgacctgacc gacagcgaga tggacgtcgt cagattcgat 1140

gacgacaaca gccccagctt catccagatc agaagcgtgg ccaagaagca ccccaagacc 1200

tgggtgcact atatcgccgc cgaggaagag gactgggact acgcccctct ggtgctggcc 1260

cccgacgaca gaagctacaa gagccagtac ctgaacaatg gcccccagcg gatcggccgg 1320

aagtacaaga aagtgcggtt catggcctac accgacgaga cattcaagac cagagaggcc 1380

atccagcacg agagcggcat cctgggcccc ctgctgtacg gcgaagtggg cgacaccctg 1440

ctgattatct tcaagaacca ggccagccgg ccctacaaca tctaccccca cggcatcacc 1500

gacgtgcggc ccctgtacag cagacggctg cccaagggcg tgaagcacct gaaggacttc 1560

cccatcctgc ccggggagat cttcaagtac aagtggaccg tgaccgtgga agatggcccc 1620

accaagagcg accccagatg cctgacccgg tactacagca gcttcgtgaa catggaacgg 1680

gacctggcct ccgggctgat cggccctctg ctgatctgct acaaagaaag cgtggaccag 1740

cggggcaacc agatcatgag cgacaagcgg aacgtgatcc tgttcagcgt gttcgatgag 1800

aacagaagct ggtatctgac cgagaatatc cagcggttcc tgcccaaccc tgccggcgtg 1860

cagctggaag atcccgagtt ccaggccagc aacatcatgc actccatcaa tggctacgtg 1920

ttcgacagcc tgcagctgag cgtgtgcctg cacgaggtgg cctactggta catcctgagc 1980

atcggcgccc agaccgactt cctgagcgtg ttcttcagcg gctacacctt caagcacaag 2040

atggtgtacg aggataccct gaccctgttc cccttctccg gcgaaaccgt gttcatgagc 2100

atggaaaacc ccggcctgtg gattctgggc tgccacaaca gcgacttccg gaaccggggc 2160

atgaccgccc tgctgaaggt gtccagctgc gacaagaaca ccggcgacta ctacgaggac 2220

agctacgagg atatcagcgc ctacctgctg agcaagaaca acgccatcga gcctcggagc 2280

ttcagccaga atcctcctgt gctgtccaaa aacaatgcca ttgagccccg gtcattctcc 2340

cagaactcta gacacccaag cctggccgaa gccaaagaag ccgctaacgc cgagctggat 2400

agctacggcg tgtccgactt ctacaagcgg ctgatcgata aggccaagac cgtggaaggc 2460

gtggaagccc tgaaagatgc tattctggcc gctctgcctg gaagcggagg tagcggagga 2520

tctggcggtt ctggcagcgg atctaagaac aatgctatcg aaccccgctc ctttagccag 2580

aacagcagac acccttctgg aagtggtggt tctggtggat ccggcggatc tggatctctg 2640

gcagaggcta aagaggccgc caacgctgaa ctcgactcct atggggtgtc cgatttttac 2700

aaacgcctca ttgacaaggc caaaacagtc gagggcgtcg aggctctgaa ggatgccatt 2760

cttgctgccc tgccatctaa aaacaacgca atagagccca gatccttcag tcagaacagt 2820

cggcacccca gccagagcga ccaagaggaa atcgacttcg acgacaccat cagcgtcgag 2880

atgaagaaag aggacttcga catcttcgac gaggacgaga atcagagccc cagaagcttc 2940

cagaaaaaga cccggcacta ctttatcgcc gccgtggaaa gactgtggga ctacggaatg 3000

agcagcagcc ctcacgtgct gagaaataga gcacagagcg gcagcgtgcc ccagttcaag 3060

aaagtggtgt tccaagagtt caccgacggc agcttcaccc agcctctgta tagaggcgag 3120

ctgaatgagc acctgggcct gctgggacct tatatcagag ccgaggtgga agataacatc 3180

atggtcacct tccggaacca ggccagcaga ccctacagct tctacagcag cctgatcagc 3240

tacgaagagg accagagaca gggcgccgag cctagaaaga acttcgtgaa gcccaacgag 3300

acaaagacct acttttggaa ggtgcagcac cacatggccc ctaccaagga cgaattcgat 3360
tgcaaggcct gggcctactt cagcgacgtg gacctggaaa aggatgtgca ctctggcctg 3420
atcggccctc tgctcgtgtg tcacaccaac acactgaacc ccgctcacgg cagacaagtg 3480
accgtgcaag agttcgccct gttcttcacc atcttcgacg agacaaagag ctggtacttc 3540
accgagaaca tggaacggaa ctgcagagcc ccttgcaaca tccagatgga agatcccacc 3600
ttcaaagaga actaccggtt ccacgccatc aacggctaca tcatggatac cctgcctggc 3660
ctggttatgg cccaggacca gagaatcaga tggtatctgc tgagcatggg cagcaacgag 3720
aacatccaca gcatccactt cagcggccac gtgttcaccg tgcggaagaa agaagagtac 3780
aagatggccc tgtacaatct gtaccccggc gtgttcgaga cagtggaaat gctgccttct 3840
aaggccggca tttggagagt ggaatgtctg atcggagagc atctgcacgc cggcatgagc 3900
accctgtttc tggtgtacag caacaagtgt cagacccctc tcggcatggc ctccggccac 3960
atcagagatt ttcagatcac agccagcggc cagtatggcc agtgggctcc taaactggcc 4020
agactgcact acagcggcag catcaatgcc tggtccacca aagagccctt cagctggatt 4080
aaggtggacc tgctggcccc tatgatcatc cacggcatca aaacacaggg cgccagacag 4140
aagttcagca gcctgtacat cagccagttc atcatcatgt acagcctgga cggcaagaag 4200
tggcagacct acagaggcaa tagcaccggc acactgatgg tgttcttcgg caatgtggac 4260
agcagcggca tcaagcacaa catcttcaac cctccaatca ttgcccggta catccggctg 4320
caccccacac actacagcat ccggtctacc ctgagaatgg aactgatggg ctgcgacctg 4380
aacagctgta gcatgcccct cggaatggaa agcaaggcca tcagcgacgc ccagatcacc 4440
gcctccagct acttcaccaa catgttcgcc acttggagcc cctccaaagc cagactccat 4500
ctgcaaggca gatctaacgc ttggaggccc caagtgaaca accccaaaga gtggctgcag 4560
gtcgacttcc agaaaaccat gaaggtcaca ggcgtgacaa cccagggcgt gaagtctctg 4620
ctgacctcta tgtacgtgaa agagttcctg atcagcagca gccaggacgg ccaccagtgg 4680
accctgtttt tccagaacgg caaagtgaag gtgttccagg gcaaccagga cagcttcacc 4740
cctgtggtca atagcctgga tcctccactg ctgaccagat acctgcggat tcaccctcag 4800
agctgggtgc accagatcgc tctcagaatg gaagtgctgg gatgtgaagc ccaggatctg 4860
tactccaaga acaacgccat cgagcccaga agcttcagcc agaactctag acacccaagc 4920
ctggccgaag ccaaagaagc cgctaacgcc gagctggata gctacggcgt gtccgacttc 4980
tacaagcggc tgatcgataa ggccaagacc gtggaaggcg tggaagccct gaaagatgcc 5040
attctggctg ctctgcctgg aagcggagga agtggtggat ctggcggttc tggctccggc 5100
agcaaaaaca atgccattga gcctcggagc ttctcccaga acagcagaca tccttctgga 5160
tcaggcggta gcggaggatc cggcggatct ggatctctgg ctgaggctaa agaggccgcc 5220

aacgctgaac tcgactccta tggggtgtcc gatttttaca aacgcctcat tgacaaggcc 5280

aaaacagtcg agggcgtcga ggctctgaag gatgctattc ttgccgcact gccctga 5337

<210> 57
<211> 138
<212> DNA
<213> Artificial Sequence

<220>
<223> optimized DNA sequence encoding SEQ ID NO: 46

<400> 57
ctggctgaag caaaagaagc agccaatgcg gaactggatt cttatggcgt gagcgatttt 60

tataaacgcc tgatcgataa agcaaaaacc gttgaaggtg ttgaagcact gaaagatgcg 120

attctggcag cactgccg 138

<210> 58
<211> 42
<212> DNA
<213> Artificial Sequence

<220>
<223> exemplary DNA encoding Glycine-serine Linker G1 of SEQ ID NO:40

<400> 58
ggatcaggag gttcaggagg aagtggtgga tctggaagtg ga 42

<210> 59
<211> 36
<212> DNA
<213> Artificial Sequence

<220>
<223> exemplary DNA encoding Glycine-serine Linker G2 of SEQ ID NO:41

<400> 59
ggcggcagcg gcggcggcag cggcggcggc agcggc 36

## Description

The present invention relates to recombinant coagulation factors, in particular, recombinant Factor VIII (FVIII) proteins having an increased half-life. They comprise a heavy chain portion and a light chain portion of Factor VIII and at least two albumin binding domains, wherein at least one albumin binding domain is C-terminal to the heavy chain portion and at least one albumin binding domain is C-terminal to the light chain portion. If the protein is a single chain protein, the albumin binding domain(s) C-terminal to the heavy chain portion is/are N-terminal to the light chain portion. The invention also relates to nucleic acids encoding the proteins of the invention, methods of producing them and pharmaceutical compositions comprising any of these, wherein the pharmaceutical composition preferably is for use in treatment of hemophilia A.

FVIII is an important co-factor in the coagulation cascade. Wildtype human FVIII is synthesized as a single chain consisting of 2351 amino acids and comprises three A domains (A1-A3), one B domain and two C domains (C1 and C2), interrupted by short acidic sequences (a1-a3). The first 19 amino acids are the signal sequence, which is cleaved by intracellular proteases, leading to a FVIII molecule of 2332 amino acids. The resulting domain structure is A1-a1-A2-a2-B-a3-A3-C1-C2. During post-translational modification, FVIII becomes glycosylated, sulfated and proteolytically processed. Sulfation is important for the extracellular interaction with different proteins, especially Thrombin and von Willebrand factor (vWF). It takes place on six tyrosines in the acidic regions a1, a2 and a3. Intracellular cleavage, by the serine protease furin, divides FVIII into a heavy chain (A1-a1-A2-a2-B) and a light chain (a3-A3-C1-C2). During this cleavage, parts of the B domain can be lost. Therefore, the light chain has a molecular weight of 80 kDa, whereas the heavy chain can be slightly heterogeneous, with a molecular weight around 210 kDa. The binding between heavy and light chain is not covalent, but mediated by the divalent metal ion Cu²⁺ between the A1 and A3 domain.

In the circulation, FVIII is bound to vWF via the a3, C1 and C2 domain, which protects FVIII from early activation as well as degradation.

Upon activation, FVIII is cleaved by Thrombin at three positions, leading to a heterotrimer and loss of the B domain (heterotrimeric FVIIIa). The heterotrimer forms a complex with the activated coagulation Factor IXa and coagulation Factor X, and the light chain binds to a phospholipid bilayer, e.g., the cell membrane of (activated) platelets.

Hemophilia A mainly is a genetic bleeding disorder linked to the X-chromosome, occurring in 1 of 5000 newborn males. However, Hemophilia A can also occur spontaneously due to an auto-immune response against FVIII. Patients with Hemophilia A suffer from longer bleeding durations, spontaneous and internal bleedings, affecting their everyday life.

Hemophilia A patients are generally treated by administration of FVIII. Depending on the severity of the disease (mild, moderate or severe), treatment is on demand or prophylactic. Therapeutic FVIII products are either purified from human plasma (pFVIII) or the products are produced recombinantly in cell culture (rFVIII).

During the development of recombinant FVIII molecules for therapy, B-domain deleted FVIII molecules have been designed, because the B-domain is not important for the functionality of FVIII in clotting. This predominantly leads to a reduction in size. One of the most common B-domain deleted FVIII product is ReFacto® or ReFacto AF® produced by Pfizer. This FVIII variant lacks 894 amino acids of the B domain.

One issue with regard to FVIII substitution therapies is the relatively low in vivo half-life of the plasma-derived or recombinant Factor VIII proteins. There have been different approaches for enhancing FVIII half-life reaching from PEGylation to albumin incorporation, single chain molecules and Fc fusion.

WO 2014/070953 A1 relates to a method of reducing or decreasing the bleeding rate of a hemophilia patient by administering a long acting Factor VIII polypeptide, wherein the long acting Factor VIII polypeptide may be a fusion of the Factor VIII polypeptide and a heterologous moiety, which is an FcRn binding partner and may comprise an Fc region.

WO 91/01743 A1 describes a process for extending the half-life of a biologically active protein or peptide in vivo by covalently coupling said protein or peptide to a polypeptide fragment capable of binding to a serum protein, especially to serum albumin. Preferably, the albumin-binding fragment derives from streptococcal protein G or staphylococcal protein A. The generated fusion protein binds to serum albumin in vivo, and benefits from its longer half-life, thereby increasing the net half-life of the fused therapeutically interesting protein or peptide. WO 2005/097202 A2 generally mentions fusion proteins combining a therapeutic protein and a plasma protein in a single polypeptide chain, wherein such fusion proteins may provide clinical benefits in requiring less frequent injection and higher levels of therapeutic protein in vivo.

WO 2009/016043 A2 discloses an albumin binding polypeptide. WO 2010/054699 A1 discloses a capture molecule for modulation of pharmacokinetics and/or pharmacodynamics of a target having a biological function in a mammal, wherein said molecule comprises at least one albumin binding moiety.

WO 2011/101284 A1 describes Factor VIII fusion proteins. Reference to albumin binding moieties is made. Fusion partners are presumed to delay in vivo clearance of FVIII by interaction with serum albumin. Albumin binding polypeptides, such as the ABD1 polypeptide, are mentioned as examples for fusion partners. Suggested fusion proteins comprise four albumin-binding moieties (albumin binding domain - ABD) comprising ABD1, e.g. 4 x ABD1 in the B domain or 4 x ABD1 at the C-terminus of the FVIII light chain.

WO 2012/004384 A2 discloses an albumin binding sequence. It describes a fusion protein or conjugate with the albumin binding polypeptide, wherein the second moiety may be Factor VIII.

WO 2013/143890 A1 discloses a compound for oral administration comprising a moiety with desired therapeutic activity and a moiety that binds to albumin.

WO 2014/048977 A1 discloses a class of engineered polypeptides having a binding affinity for albumin. WO 2014/064237 A1 provides albumin binding domain binding polypeptides comprising an ABD binding motif. WO 2015/091957 A1 relates to a class of engineered polypeptides having a binding affinity for albumin, wherein the polypeptides have a high resistance to proteolytic cleavage.

In light of the state of the art, the inventors of the present invention addressed the problem of providing recombinant FVIII proteins with increased half-life, which preferably allow a more convenient substitution therapy for the patient, allowing administration intervals of more than three days or even more than one week or more.

This problem is solved by the present invention, e.g., by the claimed subject-matter.

### Description of the invention

The inventors have constructed new FVIII variants having a longer in vivo half-life and excellent specific activity as evidenced by different biological activity assays. These proteins further have a high level of expression and a low profile of fragments and side products. Further advantages and preferred embodiments are explained elsewhere in this description. The inventors have found that a particular arrangement of albumin binding domains contributes to an increase in in vivo half-life.

The invention thus provides a recombinant Factor VIII protein comprising a heavy chain portion and a light chain portion of Factor VIII and at least two albumin binding domains, wherein at least one albumin binding domain is C-terminal to the heavy chain portion and at least one albumin binding domain is C-terminal to the light chain portion. If the protein is a single chain protein, the albumin binding domain(s) C-terminal to the heavy chain portion is/are N-terminal to the light chain portion.

Factor VIII in complex with von Willebrand factor (vWF) has an in vivo half-life of about 12 hours. FVIII not associated with vWF is typically degraded much more rapidly.

Albumin has an in vivo half-life of about 19 days. By introducing at least two albumin binding domains in the FVIII sequence, it was possible to obtain a significant half-life prolongation. Different positions and different numbers of the albumin binding moiety have been tested in order to identify the optimal positions and numbers of integrated albumin binding moieties.

Without intending to be bound by the theory, it is believed that albumin binding to the FVIII protein of the invention through the albumin binding domains in the specific positions described herein is particularly effective in inhibiting breakdown of the FVIII protein of the invention. This appears to increase in vivo half-life more than association with vWF associated with native FVIII in blood.

### FVIII

The skilled person understands the term FVIII (or Factor VIII) and is aware of the structure and biological functions of wild type FVIII and typical variants thereof. Apart from the features specified herein, the FVIII protein of the invention may be designed as deemed appropriate and advantageous by the skilled person.

In particular, the Factor VIII protein of the invention should typically comprise all necessary portions and domains known to be important for biological function. For example, preferably, the FVIII protein further comprises domains corresponding to, substantially corresponding to, and/or functionally corresponding to the A and C domains of wild type FVIII, especially to A1, A2, A3, C1 and C2 domains. It may further comprise additional portions and domains. For example, preferably, the FVIII protein further comprises an a1 domain between the A1 and the A2 domains and an a2 domain C-terminal to the A2 domain. For a double chain protein, on a separate chain, or for a single chain protein, C-terminal to said domain, and, optionally, C-terminal to the B-domain or a truncated B-domain and to at least one albumin binding domain, the FVIII protein comprises at least a truncated a3 domain. Before processing, the Factor VIII protein of the invention may also comprise a signal sequence. Thus, the heavy chain portion preferably comprises the domains A1 and A2, and typically comprises the domains A1-a1-A2-a2 or A1-a1-A2-a2-B. Preferably, the B-domain of the Factor VIII protein is at least partly deleted. The light chain portion preferably comprises the domains A3 and C1 and C2, and typically comprises the domains a3-A3-C1-C2. Any or all of said domains may be wildtype (wt) FVIII domains, or they may differ from the wildtype domains, e.g., as known in the state of the art or deemed appropriate by the skilled person.

The domains are preferably contained in the protein in that order, i.e., from N-terminus to C-terminus of the protein.

While parts of the FVIII protein of the invention can be designed as desired by the skilled person, the FVIII preferably maintains a high FVIII biological activity. As shown in the examples, the invention allows generation of a FVIII protein with a high biological activity, as measured e.g. by the chromogenic activity. Therefore, preferably the FVIII protein according to the invention has a chromogenic activity which is at least comparable to the activity of the wt FVIII protein, i.e., it has at least 50% of the specific chromogenic activity of the wt protein (SEQ ID NO: 1). Preferably, the FVIII protein according to the invention has at least 80%, at least 100 % or more than 100% of the specific chromogenic activity of the wt protein. Preferably, the chromogenic activity also is at least 80%, at least 90%, at least 100% or more than 100% of the chromogenic activity of ReFacto AF® (international non-proprietary name: Moroctocog Alfa), a commercially available B-domain deleted FVIII (Pfizer).

A FVIII protein according to the present invention shall have at least one biological activity or function of a wt FVIII protein, in particular with regard to the function in coagulation. The FVIII protein should be cleavable by thrombin, leading to activation. Preferably, the FVIII protein according to the invention comprises at least one thrombin recognition and/or thrombin cleavage site, wherein said thrombin recognition and/or thrombin cleavage sites may correspond to or substantially correspond to those of wild type FVIII. It is then capable of forming a complex with the activated coagulation Factor IXa and coagulation Factor X, and the light chain is capable of binding to a phospholipid bilayer, e.g., the cell membrane of (activated) platelets.

The biological activity of FVIII can be determined by analyzing the chromogenic, the clotting or the coagulant activity of the protein, as described herein. Typically, the chromogenic activity is taken as a measure of biological activity.

An increased in vivo half-life may be achieved for Factor VIII proteins of the invention that are double chain proteins. Double chain proteins which may form a basis for the FVIII proteins of the invention are known in the art, e.g., wt FVIII or B-domain deleted or truncated versions thereof, e.g., ReFacto AF®.

Moreover, the inventors have found an excellent increase in in vivo half-life with Factor VIII proteins of the invention that are a single chain proteins. Single chain Factor VIII proteins which may form a basis for the FVIII proteins of the invention are known in the art. In general, singe chain FVIII proteins do not comprise a functional furin cleavage site and thus, before activation, remain in the circulation as a single chain. Such proteins are also disclosed in EP application No. 19173440 or taught herein.

As a single chain backbone for the inventive protein, a single chain FVIII molecule has been developed, in which several amino acids including the furin cleavage site (positions R1664 - R1667, wherein the signal peptide is also counted) have been deleted. The B domain is deleted to a large extent, wherein an internal fragment (at least NPP) of the B-domain is maintained and an intact Thrombin cleavage site is preceding the internal fragment. This single chain Factor VIII protein (FVIII-sc) has been shown to be more stable than wt Factor VIII. Thus, in a preferred embodiment, the recombinant Factor VIII protein of the invention comprises, in a single chain, a heavy chain portion comprising an A1 and an A2 domain and a light chain portion comprising an A3, C1 and C2 domain of Factor VIII, wherein
a) in said recombinant Factor VIII protein, 894 amino acids corresponding to consecutive amino acids between F761 and P1659 of wild type Factor VIII as defined in SEQ ID NO: 1 are deleted, leading to a first deletion;
b) said recombinant Factor VIII protein comprises, spanning the site of the first deletion, a processing sequence comprising SEQ ID NO: 2 or a sequence having at most one amino acid substitution in SEQ ID NO: 2, wherein said processing sequence comprises a first thrombin cleavage site;
c) in said recombinant Factor VIII protein, at least the amino acids corresponding to amino acids R1664 to R1667 of wild type Factor VIII are deleted, leading to a second deletion; and
d) said recombinant Factor VIII protein comprises, C-terminal to the second deletion and N-terminal of the A3 domain, a second thrombin cleavage site.

As defined in a), in the FVIII of the invention, 894 amino acids corresponding to consecutive amino acids between F761 and P1659 of wild type Factor VIII as defined in SEQ ID NO: 1 are deleted in the Factor VIII protein of the invention, leading to a first deletion. In certain embodiments, in particular, starting from a numbering of amino acids in FVIII without deletions or insertions, the term "corresponding to" should be understood to mean "identical to".

For specific amino acids which may be mutated compared to the wt, an amino acid corresponding to the wild type aa is determined by an alignment e.g. using EMBOSS Needle (based on the Needleman-Wunsch algorithm; settings: MATRIX: "BLOSUM62", GAP OPEN: "20", GAP EXTEND:"0.5", END GAP PENALTY: "false", END GAP OPEN: "10", END GAP EXTEND: "0.5").

In order to assess sequence identities of two polypeptides, such an alignment may be performed in a two-step approach: I. A global protein alignment is performed using EMBOSS Needle (settings: MATRIX: "BLOSUM62", GAP OPEN: "20", GAP EXTEND:"0.5", END GAP PENALTY: "false", END GAP OPEN: "10", END GAP EXTEND: "0.5") to identify a particular region having the highest similarity. II. The exact sequence identity is defined by a second alignment using EMBOSS Needle (settings: MATRIX: "BLOSUM62", GAP OPEN: "20", GAP EXTEND:"0.5", END GAP PENALTY: "false", END GAP OPEN: "10", END GAP EXTEND: "0.5") comparing the fully overlapping polypeptide sequences identified in (I) while excluding non-paired amino acids.

"between" excludes the recited amino acids, e.g., it means that the recited amino acids are maintained. "deletion" or "deleted" does not necessitate that the protein was actually prepared by deleting amino acids previously present in a predecessor molecule, but it merely defines that the amino acids are absent, independent from the preparation of the molecule. For example, the protein can be produced based on nucleic acids prepared by *de novo* synthesis or by genetic engineering techniques.

As defined in b), the
recombinant Factor VIII protein comprises, spanning the site of the first deletion, a processing sequence comprising SEQ ID NO: 2 (PRSFSQNPP) or a sequence having at most one amino acid substitution in SEQ ID NO: 2, wherein said processing sequence comprises a first thrombin cleavage site. Accordingly, at least one amino acid of the processing sequence corresponds to an amino acid C-terminal to the deletion and at least one amino acid of the processing sequence corresponds to an amino acid N-terminal to the deletion. The processing sequence comprises SEQ ID NO: 2 or a sequence having at most one amino acid substitution in SEQ ID NO: 2, i.e., the processing sequence can be longer. In particular, the processing sequence is selected from the group comprising SEQ ID NO: 2, 4, 5, 6, 7 or 8. The inventors have found that a processing sequence of the invention enables a particularly good cleavage by thrombin.

In preferred embodiments, the processing sequence is no longer than SEQ ID NO: 4. The processing sequence may be directly C-terminal to sequences from the a2 domain, e.g., wt a2 domain sequences. The first N-terminal two amino acids of the processing sequence may already belong to the a2 domain. Preferably, the amino acid directly N-terminal to the processing sequence is E.

One amino acid in SEQ ID NO: 2 can be substituted, e.g., to reduce immunogenicity. Optionally, the F, the S C-terminal to the F, the Q or the N are substituted.

The processing sequence may be SEQ ID NO: 4 (PRSFSQNPPVL) or a sequence having at most one amino acid substitution in said sequence, wherein, optionally, the F, the S C-terminal to the F, the Q or the N are substituted. Moreover, the present inventors have shown that an L at the C-terminus of the processing sequence, as in SEQ ID NO: 4, 5, 6, 7 or 8, endows the FVIII with particularly good activity. One especially preferred example of a single chain FVIII protein, which may form the backbone for the protein of the invention, is shown in the examples in further detail under the name V0 (SEQ ID NO: 16). The processing sequence of the FVIII protein V0, which has been found to be particularly advantageous, consists of SEQ ID NO: 4, which is a specific embodiment of SEQ ID NO: 5-8.

The alternative processing sequences SEQ ID NO: 5 (PRSXSQNPPVL), SEQ ID NO: 6 (PRSFXQNPPVL), SEQ ID NO: 7 (PRSFSXNPPVL) and SEQ ID NO: 8 (PRSFSQXPPVL) are variants in which X can be any naturally occurring amino acid. Optionally, X is a conservative substitution compared to the corresponding amino acid in SEQ ID NO: 4, i.e. a hydrophobic amino acid is substituted by a hydrophobic amino acid, a hydrophilic amino acid is substituted by a hydrophilic amino acid, an aromatic amino acid by an aromatic amino acid, an acid amino acid by an acid amino acid and a basic amino acid by a basic amino acid.

As defined in c), in the FVIII protein of the invention, the amino acids corresponding to amino acids R1664 to R1667 of wild type Factor VIII are deleted, leading to a second deletion. These amino acid correspond to the furin cleavage recognition site of wt FVIII. Accordingly, the protein is essentially not cleaved by furin. In a composition, at least 80%, optionally, at least 90% or at least 95% of the FVIII protein of the invention are present in a single chain form.

As defined in d), the recombinant Factor VIII protein of the invention comprises, C-terminal to the second deletion and N-terminal of the A3 domain, a second thrombin cleavage site. Accordingly, upon activation, the part of the FVIII protein between the thrombin cleavage site in the processing sequence and the second thrombin cleavage site are excised from the activated FVIII protein.

Further, the invention provides a recombinant Factor VIII protein comprising, in a single chain, a heavy chain portion comprising an A1 and an A2 domain and a light chain portion comprising an A3, C1 and C2 domain of Factor VIII, wherein,
a) said recombinant Factor VIII protein comprises a processing sequence comprising SEQ ID NO: 2 or a sequence having at most one amino acid substitution in SEQ ID NO: 2, wherein said processing sequence comprises a first thrombin cleavage site;
b) directly C-terminal to said processing sequence, said Factor VIII protein comprises a heterologous sequence comprising at least one, preferably, two albumin binding domain(s);
c) directly C-terminal to said heterologous sequence, said Factor VIII protein comprises a merging sequence having at least 90% sequence identity to SEQ ID NO: 9 (e.g., SEQ ID NO: 9); and
d) said recombinant Factor VIII protein comprises, C-terminal to SEQ ID NO: 9, a second thrombin cleavage site; and
e) said recombinant Factor VIII protein comprises, C-terminal to the light chain portion, at least one, preferably, two albumin binding domain(s).

Said recombinant FVIII protein may be a FVIII protein as described above. The FVIII protein typically comprises at least one further thrombin cleavage site.

In one embodiment, a FVIII protein of the invention that optionally is a single chain protein comprises a heavy chain portion having at least 90% sequence identity to aa20-aa1667 of SEQ ID NO: 1, and a light chain portion having at least 90% sequence identity to aa1668-aa2351 of SEQ ID NO: 1. Optionally, the respective sequence identity aa20-aa1667 of SEQ ID NO: 1 and sequence identity to aa1668-aa2351 of SEQ ID NO: 1 are at least 95%. The respective sequence identity to aa20-aa1667 of SEQ ID NO: 1 and sequence identity to aa1668-aa2351of SEQ ID NO: 1 may be at least 98%. Optionally, the respective sequence identity to said sequences is at least 99%. The invention also provides a FVIII protein of the invention comprising a heavy chain portion having aa20-aa1667 of SEQ ID NO: 1 and a light chain portion having aa1668-aa2351 of SEQ ID NO: 1.

Several experiments performed by the inventors have been carried out with a single chain FVIII of the invention on the basis of the V0 single chain construct (SEQ ID NO: 16) with at least one albumin binding domain introduced C-terminal to the heavy chain portion and C-terminal to the light chain portion, as described herein. Such proteins have shown advantageous characteristics with regard to expression, stability, in vivo half-life and purification. Accordingly, a preferred FVIII protein of the invention, which may be a single chain protein, comprises a heavy chain portion having at least 90% sequence identity to aa20-aa768 of SEQ ID NO: 16, and a light chain portion having at least 90% sequence identity to aa769-aa1445 of SEQ ID NO: 16. Optionally, the respective sequence identity to aa20-aa768 of SEQ ID NO: 16 and sequence identity to aa769-aa1445 of SEQ ID NO: 16 are at least 95%. The respective sequence identity to aa20-aa768 of SEQ ID NO: 16 and sequence identity to aa769-aa1445 of SEQ ID NO: 16 may be at least 98%. Optionally, the respective sequence identity to said sequences is at least 99%. The invention also provides a FVIII protein of the invention comprising a heavy chain portion having aa20-aa768 of SEQ ID NO: 16 and a light chain portion having aa769-aa1445 of SEQ ID NO: 16.

wt FVIII typically is bound by vWF. vWF shields FVIII from proteolytic degradation and receptor-mediated clearance, e.g. via low-density lipoprotein (LDL) receptor-related protein (LRP1), LDL-receptor (LDLR) and heparan-sulfate proteoglycans (HSPG), within the liver (Lenting etal., 2007. J Thromb Haematol 5:1353-60). However, it has been shown that the half-live of vWF is approx. 15 h, thereby limiting the FVIII:vWF complex half-life to the vWF-related clearance pathway. The inventors found that vWF binding potency of the FVIII protein of the invention may be diminished compared to wt FVIII or ReFacto AF®, which may be explained by sterical hindrance due to albumin binding. For example, FVIII proteins of the invention may have 0%-90%, 10%-80%, 20-70%, 30-60%, or 40-50% of the binding potency of ReFacto AF® to vWF, which can be determined by an assay as described below. Preferably, in the presence of human serum albumin in physiological concentrations, said binding potency is less than 50% of the binding potency of ReFacto AF® to vWF.

vWF binding is mediated in particular by amino acid positions Y1683 and Y1699. To avoid vWF binding, e.g. amino acids corresponding to Y1683 and/or Y1699 of wt FVIII of SEQ ID NO: 1 may be mutated. For example, the amino acid corresponding to Y1683 and/or Y1699 of wt FVIII of SEQ ID NO: 1 may be mutated to a C or F, e.g., Y1699C or Y1699F. In particular, a mutation of the amino acid corresponding to Y1699 to F and a mutation of the amino acid corresponding to Y1683 to F, both mutations together also designated "b mutation" have been confirmed to further decrease binding of vWF to FVIII proteins of the invention. Beside the "b mutation", the inventors have additionally tested an "a mutation" comprising the amino acid substitutions Y737F, Y738F, and Y742F of wt FVIII of SEQ ID NO: 1 and a "c mutation" comprising the amino acid substitutions I2117S and R2169H of wt FVIII of SEQ ID NO: 1. Additionally, the inventors have tested combinations of "a mutation" and "b mutation" and further combinations of "a mutation" and "b mutation" and "c mutation". It was observed, that the "c mutation" negatively influenced the protein expression and functionality. The "b mutation" either alone or in combination with the "a mutation" did not influence the protein expression and functionality, but strongly decreased the binding to vWF. In comparison, the "a mutation" did not decrease the binding to vWF.

Thus, to further decrease vWF binding, the recombinant Factor VIII protein of the invention may have a suitable mutation as described herein, e.g., a "b mutation", i.e., a mutation of the amino acid corresponding to Y1699 to F at position 1699 and a mutation of the amino acid corresponding to Y1683 to F at position 1683 in wt Factor VIII protein of SEQ ID NO: 1. For example, the FVIII protein of the invention may comprise a heavy chain portion and a light chain portion of Factor VIII and at least two albumin binding domains, wherein at least two albumin binding domains (e.g., two) are C-terminal to the heavy chain portion and at least two albumin binding domains (e.g., two) are C-terminal to the light chain portion, wherein the FVIII protein further comprises a b mutation. Such a FVIII protein may further comprise linkers, e.g., a thrombin cleavable linker optionally flanked by a glycine-serine linker, between the albumin binding domains and other parts of the protein and between the albumin binding domains. Alternatively, such a FVIII protein does not comprise linkers. In the context of the invention, "flanked" means that the relevant portions are in a close vicinity, preferably, with a distance of at most 10, 5 or 2 amino acids positions. Optionally, the relevant portions are immediately adjacent.

### Albumin binding domains

The recombinant Factor VIII protein of the invention comprises a heavy chain portion and a light chain portion of Factor VIII and at least two albumin binding domains, wherein at least one albumin binding domain is C-terminal to the heavy chain portion and at least one albumin binding domain is C-terminal to the light chain portion.

Different albumin-binding domains (ABDs) may be employed in the context of the invention. ABDs comprise or are protein domains capable of binding albumin, preferably with high affinity, e.g., with at least 50% or at least 80% of the affinity of the ABD2 to albumin. Historically, the ABD typically is a small, three-helical protein domain derived from one of various surface proteins expressed by gram-positive bacteria. For example, domains derived from streptococcal protein G and protein PAB from *Finegoldia magna,* which share a common origin and therefore represent an interesting evolutionary system, have been thoroughly studied structurally and functionally. Their albumin-binding sites have been mapped and these domains form the basis for a wide range of protein engineering approaches. By substitution-mutagenesis they have been engineered to achieve a broader specificity, an increased stability or an improved binding affinity, respectively.

For example, albumin binding domains disclosed by Nilvebrant et al. (2013, Comput Struct Biotechnol J. 6: e201303009), Johansson et al. (2001, JBC 277: 8114-8120), Jacobs et al. (2015, Protein Engineering, Design and Selection 28 (10); 385-393), WO 91/01743 A1, WO 2009/016043 A2, WO 2010/054699 A1, WO 2012/004384 A2, WO 2014/048977 A1 or WO 2015/091957 A1 may be used.

Preferably, the albumin binding domain comprises a sequence according to SEQ ID NO: 44: LAX₃AKX₆X₇ANX₁₀ELDX₁₄YGVSDFYKRLIX₂₆KAKTVEGVEALKX₃₉X₄₀ILX₄₃X₄₄LP wherein independently of each other
X₃ is selected from E, S, Q and C, preferably, E;
X₆ is selected from E, S, C and V, preferably, E;
X₇ is selected from A, S and L, preferably, A;
X₁₀ is selected from A, S and R, preferably, A;
X₁₄ is selected from A, S, C and K, preferably, S;
X₂₆ is selected from D, E and N, preferably, D;
X₃₉ is selected from D, E and L, preferably, D;
X₄₀ is selected from A, E and H, preferably, A;
X₄₃ is selected from A and K, preferably, A;
X₄₄ is selected from A, S and E, preferably, A;
L in position 45 is present or absent, preferably, present; and
P in position 46 is present or absent, preferably, present.

Alternatively, the albumin binding domain may comprise an amino acid sequence which has at least 95 % identity to the sequence of SEQ ID NO: 44.

The inventors have achieved good results using an albumin binding domain designated ABD1 (SEQ ID NO: 45). It is preferred to use the sequence of ABD2 (SEQ ID NO: 46) that has been deimmunized for the human immune system, i.e., adapted to avoid immune responses in humans. If not otherwise mentioned, said albumin binding domain is used in the experiments shown herein. ABD2 may be encoded by a nucleic acid of SEQ ID NO: 57, which is codon-optimized for expression in human cells.

While it is possible to use different albumin binding domains at different locations in the FVIII protein, typically, all albumin binding domains in the FVIII protein will have the same sequence, preferably ABD2. Alternatively, it is possible to use different albumin binding domains at different locations within the FVIII protein in order to obtain multivalent albumin binding.

For example, in the FVIII protein of the invention, one albumin binding domain may be C-terminal to the heavy chain portion and one albumin binding domain C-terminal to the light chain portion. Alternatively, in one of the two selected positions, there may be one albumin binding domain, and two, three, four or more albumin binding domains in the other. For example, one albumin binding domain may be C-terminal to the heavy chain portion and two albumin binding domains C-terminal to the light chain portion, or one albumin binding domain may be C-terminal to the heavy chain portion and three albumin binding domains C-terminal to the light chain portion, or one albumin binding domain may be C-terminal to the heavy chain portion and four albumin binding domains C-terminal to the light chain portion.

In a FVIII protein of the invention, two albumin binding domains may be C-terminal to the heavy chain portion and one albumin binding domain C-terminal to the light chain portion, or three albumin binding domains may be C-terminal to the heavy chain portion and one albumin binding domain C-terminal to the light chain portion, or four albumin binding domains may be C-terminal to the heavy chain portion and one albumin binding domain C-terminal to the light chain portion.

Preferably, the number of albumin binding domains in each of the two positions is the same. It has also been shown to be advantageous if the FVIII protein of the invention comprises at least four albumin binding domains. The inventors have found a still better increase in the half-life for Factor VIII protein of the invention comprising at least two albumin binding domains C-terminal to the heavy chain portion and at least two albumin binding domains C-terminal to the light chain portion, preferably, two albumin binding domains C-terminal to the heavy chain portion and two albumin binding domains C-terminal to the light chain portion.

The invention also provides Factor VIII proteins of the invention with two albumin binding domains C-terminal to the heavy chain portion and three albumin binding domains C-terminal to the light chain portion, or with two albumin binding domains C-terminal to the heavy chain portion and four albumin binding domains C-terminal to the light chain portion, or with three albumin binding domains C-terminal to the heavy chain portion and two albumin binding domains C-terminal to the light chain portion, or with four albumin binding domains C-terminal to the heavy chain portion and two albumin binding domains C-terminal to the light chain portion. Optionally, there is an even number of albumin-binding domains both C-terminal to the heavy chain and C-terminal to the light chain.

### Linker

While the inventors have shown that linkers are not principally required for activity and stability of the FVIII proteins of the invention, to increase accessibility of all domains of the FVIII of the invention, in particular, access to albumin in blood, linkers were introduced into some FVIII proteins of the invention. The inventors have shown that the linkers, in particular, inclusion of at least glycine-serine linker sections, further improve expression and function. In particular, in addition to access to albumin, access for thrombin also appears to be improved. Accordingly, preferably, albumin binding domains may be separated from the heavy chain portion and/or the light chain portion and/or other albumin-binding domains by a linker, wherein, optionally, albumin-binding domains are separated from the heavy chain portion and the light chain portion and (if directly adjacent otherwise) other albumin-binding domains by a linker. It is also possible that albumin-binding domains are separated from the heavy chain portion and the light chain portion and (if directly adjacent otherwise) other albumin-binding domains by a linker, except that there is no linker N-terminal to the light chain, because the a3 domain contains a thrombin cleavage site.

In one preferred embodiment, in the recombinant Factor VIII protein of the invention, the linker comprises a thrombin-cleavable linker section. Optionally, a thrombin cleavable linker has the sequence of SEQ ID NO: 39 (abbr. L). Further thrombin cleavable sites are known in the art, e.g., disclosed in Gallwitz et al. (2012, PLoS ONE 7 (2): e31756). Thrombin cleavable linkers may thus also comprise any of these cleavable sites. Thrombin-cleavable linkers have the advantage that in generation of the active protein, i.e., after activation by thrombin, the linkers may be cleaved and, consequently, the albumin binding domains may be removed from the active protein.

Alternatively, in the recombinant Factor VIII protein of the invention, uncleavable glycine-serine linker sections may be used to introduce flexible, sterical distance between motifs to avoid structural influences. Thus, optionally, the linker comprises a glycine-serine linker section that optionally has the sequence of SEQ ID NO: 40 (abbr. G1, preferred) or SEQ ID NO: 41 (abbr. G2). The linker G1 may, e.g., be encoded by SEQ ID NO: 58. The linker G2 may, e.g., be encoded by SEQ ID NO: 59.

In preferred embodiments, different linker sections are combined. E.g. uncleavable linker sections were used to flank a central thrombin cleavable linker section to maintain thrombin accessibility of the thrombin cleavable linker section. Thus, in some embodiments, the linker comprises a thrombin cleavable linker section flanked on each side by a glycine-serine linker section, wherein said combined linker optionally has the sequence of SEQ ID NO: 42 or SEQ ID NO: 43, preferably, SEQ ID NO: 42.

The polynucleotide sequence for all linkers preferably is codon-optimized for human use. Exemplary codon-optimized sequences are provided herein in the context of FVIII proteins of the invention.

### Specific FVIII proteins of the invention

All FVIII proteins of the invention demonstrated good in vitro functionality, wherein the FVIII proteins showed reduced vWF binding in correlation to increasing numbers of albumin binding domains. vWF has a major impact on the half-life of FVIII. It was found that shielding FVIII from vWF by albumin positively influences the half-life of the FVIII protein. A broad distribution of albumin binding domains with one position between heavy chain and light chain and one position at the C-terminus of the protein was shown to enhance the shielding of FVIII from vWF.

Preferably, the recombinant Factor VIII protein the invention comprises one albumin binding domain between the heavy chain portion and the light chain portion and one albumin binding domain C-terminal to the light chain portion, wherein the sequence has at least 70%, optionally, at least 80%, at least 90%, at least 95%, at least 99% or 100% sequence identity sequence identity to SEQ ID NO: 47. Preferably, said protein is a single chain protein. The single chain protein having SEQ ID NO: 47 is designated ADLCLD_SC. It was shown to have an in vivo half-life increased by a factor of about 1.5 compared to ReFacto AF®.

In an especially preferred embodiment, the recombinant Factor VIII protein of the invention comprises at least two albumin binding domains between the heavy chain portion and the light chain portion and at least two albumin binding domain C-terminal to the light chain portion, wherein the protein has at least 80% sequence identity, optionally, at least 90%, at least 95% or at least 99% sequence identity to any of SEQ ID NO: 48, 49, 51. Preferably, the recombinant Factor VIII protein has at least 80% sequence identity, optionally, at least 90%, at least 95%, or at least 99% sequence identity to SEQ ID NO: 48. Preferably, said protein is a single chain protein.

The recombinant Factor VIII protein may also have at least 80% sequence identity, optionally, at least 90%, at least 95%, or at least 99% sequence identity to SEQ ID NO: 49. Preferably, said protein is a single chain protein.

The recombinant Factor VIII protein may also have at least 80% sequence identity, optionally, at least 90%, at least 95%, or at least 99% sequence identity to SEQ ID NO: 51. Preferably, said protein is a single chain protein.

For example, the invention provides a recombinant FVIII protein having SEQ ID NO: 48 (AD2CD2_SC), SEQ ID NO: 49 (AD2CD2woL_SC), or SEQ ID NO: 51 (AbD2CD2_SC). Such FVIII proteins have been shown to have a particularly extended in vivo half-life, e.g., for AD2CD2_SC, an in vivo half-life extended by a factor of 2.5 has been found in hemophilia A mice and a half-life extension of factor 4 has been found in albumin-deficient, transgenic neonatal Fc-receptor mice (see Examples). For AbD2CD2_SC, an in vivo half-life extended by a factor of 2.2 has been found. An increase in half-life can be analyzed on the level of FVIII antigen or on the level of activity, e.g., chromogenic activity, or both. Preferably, it is analyzed on the level of chromogenic activity.

The invention thus provides FVIII proteins of the invention, wherein the in vivo half-life of the recombinant Factor VIII protein is prolonged (i.e. increased) by a factor of at least 1.2, preferably, by a factor of at least 1.5, optionally, at least 2 or at least 2.5 in comparison to a recombinant Factor VIII protein of SEQ ID NO: 28 (ReFacto AF®). While the increase in in vivo half-life may be analyzed in model systems, e.g., mice, rats or dogs, such as in hemophilia A mice or albumin-deficient Tg32 mice having a knock-out of murine albumin and expressing human FcRn a-chain instead of the murine one (B6.Cg*-Alb^{em12}Mvw Fcgrt^{tm1Dcr}* Tg(FCGRT)32Dcr/MvwJ), the observed increase in in vivo half-life may be underestimated, because human albumin has a longer half-life than e.g. murine albumin, and it is expected that an increase seen in a murine model will be still more pronounced in humans.

A fusion partner may be employed to extend the in vivo plasma half-life of the FVIII protein of the invention. In one embodiment, the recombinant Factor VIII protein of the invention is a fusion protein with at least one further heterologous fusion partner in addition to the albumin binding moiety, preferably with a further fusion partner extending the in vivo plasma half-life of the FVIII protein. The fusion partner may e.g. be selected from the group comprising an Fc region, albumin, PAS polypeptides, HAP polypeptides, the C-terminal peptide of the beta subunit of chorionic gonadotropin, , and combinations thereof. The FVIII protein may alternatively or additionally be covalently linked to non-protein fusion partners such as albumin-binding small molecules (e.g., dabigatran), PEG (polyethylenglycol) and/or HES (hydroxyethyl starch). PAS polypeptides or PAS sequences are polypeptides comprising an amino acid sequence comprising mainly alanine and serine residues or comprising mainly alanine, proline and serine residues, the PAS sequences forming a random coil conformation under physiological conditions, as defined in WO 2015/023894. HAP polypeptides or sequences are homo-amino acid polymer (HAP), comprising e.g., repetitive sequences of glycine or glycine and serine, as defined in WO 2015/023894. Potential fusions, fusion partners and combinations thereof are described in more detail e.g., in WO 2015/023894.

Optionally, for certain therapeutic applications, the recombinant FVIII protein may be fused to an Fc region. A fusion to an Fc region may be used to extend the half-life and reduce immunogenicity.

Optionally, said heterologous fusion partner may be inserted directly N-terminal or directly C-terminal to one of the albumin binding domains, e.g., C-terminal to the heavy chain, and/or C-terminal to the C2-domain, or C-terminal to the albumin binding domain(s) C-terminal to the heavy chain or C-terminal to the albumin binding domain(s) C-terminal to the heavy chain. These locations have been found by the inventors to be advantageous for fusion, while maintaining good biological activity of the FVIII protein. Optionally, the fusion protein further comprises at least one linker.

The protein may further be glycosylated and/or sulfated. Preferably, post-translational modifications such as glycosylation and/or sulfation of the protein occur in a human cell. A particularly suitable profile of post-translational modifications can be achieved using human cell lines for production, e.g. CAP cells, in particular CAP-T cells or CAP-Go cells (WO 2001/36615; WO 2007/056994; WO 2010/094280; WO 2016/110302). CAP cells, available from Cevec Pharmaceuticals GmbH (Cologne, Germany), originate from human amniocytes as they were isolated trans-abdominally during routine amniocentesis. Obtained amniocytes were transformed with adenoviral functions (E1A, E1B, and pIX functions) and subsequently adapted to growth in suspension in serum-free medium.

During post-translational modification, the FVIII protein of the invention may be sulfated, e.g., on one, two, three, four, five or six tyrosines in the acidic regions a1, a2 and a3.

Optionally, the recombinant Factor VIII protein of the invention is a de-immunized protein, i.e., the protein has a reduced immunogenicity as compared to wt FVIII in hemophilia patients. For example, certain mutations, preferably, substitutions, have been introduced to avoid the presence of epitopes which can be presented on human HLA molecules, preferably, common human HLA molecules.

### Nucleic acids and host cells

The invention also provides a nucleic acid encoding a recombinant Factor VIII protein of the invention. Said nucleic acid may be an expression vector, e.g., suitable for expression of said recombinant Factor VIII protein in a mammalian cell, such as a human cell, such as a CAP cell.

The nucleic acid preferably encodes the FVIII with an N-terminal signal sequence, e.g., the 19 aa signal sequence of SEQ ID NO: 1. Preferred nucleic acids of the invention encode a recombinant FVIII protein having SEQ ID NO: 47 (ADLCLD_SC), SEQ ID NO: 48 (AD2CD2_SC), SEQ ID NO: 49 (AD2CD2woL_SC), SEQ ID NO: 50 (AD2CD2woLG_SC) or SEQ ID NO: 51 (AbD2CD2_SC). They may be SEQ ID NO: 52-56. The nucleic acids of the invention may be DNA molecules or RNA molecules. The nucleic acids may be optimized for expression in the respective host cell, e.g., in a human cell, e.g., a CAP cell.

The expression vector comprises the sequence encoding the FVIII protein, preferably, in codon-optimized form, under the functional control of a suitable promoter, which may be a constitutive or an inducible promoter. The promoter may be a promoter not associated with expression of FVIII in nature, e.g., EF-1 alpha or a heterologous promoter, e.g., CMV or SV40. It may further comprise pro- and/or eukaryotic selection markers, such as ampicillin resistance and dihydrofolate reductase (dhfr), and origins of replication, e.g., an SV40 origin and/or a pBR322 origin. "codon-optimized" means optimized for expression in the host cell, preferably, for expression in a human host cell.

In certain embodiments, the nucleic acid may be a vector suitable for gene therapy, e.g., for gene therapy of a human patient. Vectors suitable for gene therapy are known in the art, e.g., virus-based vectors e.g., based on adenovirus or adeno-associated virus (AAV) or based on retrovirus, such as lentiviral vectors etc., or non virus-based vectors such as but not limited to small plasmids and minicircles or transposon-based vectors. An AAV-based vector of the invention may e.g., be packaged in AAV particles for gene therapy of Hemophilia A patients.

The invention also provides a host cell comprising a nucleic acid of the invention. The host cell may be a bacterial cell, a plant cell, a fungal cell, a yeast cell or an animal cell. Preferably, the host cell is an animal cell, in particular, a mammalian cell comprising an expression vector suitable for expression of said recombinant Factor VIII protein in said cell. The host cell preferably is a human cell comprising an expression vector suitable for expression of said recombinant Factor VIII protein in said human cell. The cell may be transiently or stably transfected with the nucleic acid of the invention. The cell may be a cell line, a primary cell or a stem cell. For production of the protein, the cell typically is a cell line such as a HEK cell, such as a HEK-293 cell, a CHO cell, a BHK cell, a human embryonic retinal cell such as Crucell's Per.C6 or a human amniocyte cell such as CAP. For treatment of human patients with the protein, the host cell preferably is a human cell, e.g., a HEK293 cell line or a CAP cell line (e.g. a CAP-T cell or a CAP-Go cell). The inventors have found that in a CAP cell line, a particularly high single chain content of FVIII protein of the invention is produced. Among the CAP cells, CAP-T cells are preferred for transient expression, while CAP-Go cells may be used for creation of stable cell lines conveying an advantageous glycosylation profile to the FVIII molecule.

The cell may be an autologous cell of a Hemophilia A patient, in particular, a human Hemophilia A patient, suitable for producing FVIII in the patient after transfection and reintroduction into the patient's body. The cell may be a stem cell, e.g., a hematopoietic stem cell, but preferably it is not an embryonic stem cell, in particular when the patient is a human. The cell may also be hepatocyte, a liver sinusoidal endothelial cell or a thrombocyte.

Cell lines expressing the protein of the invention may also be used in a method of preparing the protein of the invention, comprising cultivating said cells under conditions suitable for expression of the FVIII protein and purifying said protein, e.g., using a plurality of methods known to the skilled person, such as described herein. Such purification methods may comprise standard harvesting procedures for cell removal, e.g. centrifugation, followed by chromatography steps, e.g. affinity chromatography, and methods for exchanging the FVIII proteins into a suitable buffer. The invention thus also provides a method for preparing a Factor VIII protein, comprising culturing the host cell of the invention under conditions suitable for expression of the Factor VIII protein and isolating the Factor VIII protein, wherein the method optionally comprises formulating the Factor VIII protein as a pharmaceutical composition.

### Pharmaceutical compositions

The invention provides a pharmaceutical composition comprising the recombinant Factor VIII protein of the invention, the nucleic acid of the invention or the host cell of the invention. Such pharmaceutical compositions may comprise suitable excipients or carriers, e.g., a buffer, a stabilizing agent, a bulking agent, a preservative, another (e.g., recombinant) protein or combinations thereof. In the context of the invention, if not explicitly stated otherwise, "a" is understood to mean one or more.

A suitable buffer for formulation of the protein of the invention may e.g. contain 205 mM NaCl, 5.3 mM CaCl2, 6.7 mM L-Histidine, 1.3 % Sucrose and 0.013 % Tween 20 in distilled water and have a pH of 7.0 (FVIII formulation buffer). Said buffer is used in the experiments described herein if not otherwise stated. Formulations of FVIII may be sterile, e.g., sterile filtered, in particular for in vivo use.

Optionally, the pharmaceutical composition of the invention comprising FVIII protein further comprises albumin, preferably, for a human patient, human serum albumin. Albumin may, e.g., be in a concentration of 0.1-10% w/w, such as 1-5% human serum albumin (w/w). Albumin may bind to the ABD of the FVIII proteins of the invention either before administration, or after administration to a human subject.

The pharmaceutical composition may be formulated as desired appropriate by the skilled person, e.g., for intravenous (i.v.) or subcutaneous application, intraperitoneal or intramuscular application. Generally, it is for administration as slow i.v. push bolus injection. Continuous infusion is indicated e.g., for patients requiring admission for severe bleeds or surgical procedures. Oral application, which may contribute to tolerance induction, is also possible, e.g., after expression in plants. The pharmaceutical composition may be for slow release.

Pharmaceutical compositions comprising FVIII can be lyophilized.

Due to the increase in half-life in vivo, the pharmaceutical compositions of the invention may be administered at longer intervals than previous FVIII compositions. For example, they may be for use in administration every 5 to 14 days, preferably, every 7 to 10 days.

Dosages and treatment schemes may be chosen as appropriate, e.g., for prophylaxis of bleeding or with intermittent, on-demand therapy for bleeding events. Decisions on dosing may be made by the physician. Dosing depends on the patent, e.g., weight, FVIII status, severity of disease etc. For example, the FVIII of the invention may be administered in dosages of 0.5 to 250 IU/kg body weight every 0.5 to 14 or every 6-7 days intravenously depending on the severity of the disease, typically, 0.5 to 200 IU/kg body weight.

The invention also provides a pharmaceutical composition comprising the FVIII protein of the invention in combination with an immunosuppressive agent (e.g., methylprednisolone, prednisolone, dexamethasone, cyclophosphamide, rituximab, and/or cyclosporin), and/or it may be for administration at substantially the same time (e.g. within five minutes to within 12 hours) with such an agent. The invention thus also provides a kit comprising, in addition to a FVIII protein of the invention, optionally, combined with albumin, an immunosuppressive agent, e.g., an immunosuppressive agent selected from the group comprising methylprednisolone, prednisolone, dexamethason, cyclophosphamide, rituximab, and/or cyclosporin.

The pharmaceutical composition, e.g., comprising the protein of the invention, may be for use in treating a patient in need thereof, in particular, a Hemophilia A patient, e.g., a patient with acquired hemophilia involving an autoimmune response to FVIII or a congenital Hemophilia A patient. Mammals such as mice or dogs may be treated with the pharmaceutical composition of the invention, but the patient typically is a human patient.

The pharmaceutical composition of the invention may also be used for treatment of a patient previously already treated with a recombinant and/or plasmatic Factor VIII protein. In a patient who has an antibody including an inhibitory antibody response to a recombinant and/or plasmatic Factor VIII protein, the pharmaceutical compositions may, e.g., be used for immune tolerance induction (ITI) treatment. The compositions of the invention may thus also be used for rescue ITI. The pharmaceutical compositions may also be advantageously used in a patient who has had an antibody response including an inhibitory antibody response to a recombinant and/or plasmatic Factor VIII protein, e.g., who has been treated by ITI. The pharmaceutical compositions may also be advantageously used in a patient who has had an antibody response including an inhibitory antibody response to a recombinant and/or plasmatic Factor VIII protein, who has not been treated by ITI.

The invention also provides a vial comprising the pharmaceutical composition of the invention, e.g., a syringe. The syringe may be a pre-filled syringe, e.g., a ready-to-use syringe.

The inventors contemplate that, in the context of gene therapy, due to the presence of the albumin binding domains, long-term expression and folding of the FVIII protein of the invention may be improved compared to expression of conventional FVIII.

All publications cited herein are fully incorporated herewith. The invention is further illustrated by the following embodiments, figures and examples, which are not to be understood as limiting the scope of the invention.

### Embodiments

In summary, in a first embodiment, the invention provides a recombinant Factor VIII protein comprising a heavy chain portion and a light chain portion of Factor VIII and at least two albumin binding domains, wherein at least one albumin binding domain is C-terminal to the heavy chain portion and at least one albumin binding domain is C-terminal to the light chain portion, wherein, if the protein is a single chain protein, the albumin binding domain(s) C-terminal to the heavy chain portion is/are N-terminal to the light chain portion.

In a second embodiment, the recombinant Factor VIII protein of the first embodiment (embodiment 1) is a single chain protein.

In a third embodiment, the recombinant Factor VIII protein of embodiment 1 is a double chain protein.

In a fourth embodiment, in the recombinant Factor VIII protein of any of embodiments 1-3, one albumin binding domain is C-terminal to the heavy chain portion and one albumin binding domain is C-terminal to the light chain portion.

In a fifth embodiment, in the recombinant Factor VIII protein of any of embodiments 1-3, one albumin binding domain is C-terminal to the heavy chain portion and two albumin binding domains are C-terminal to the light chain portion. In a sixth embodiment, in the recombinant Factor VIII protein of any of embodiments 1-3, one albumin binding domain is C-terminal to the heavy chain portion and three albumin binding domains are C-terminal to the light chain portion. In a seventh embodiment, in the recombinant Factor VIII protein of any of embodiments 1-3, one albumin binding domain is C-terminal to the heavy chain portion and four albumin binding domains are C-terminal to the light chain portion.

In an eighth embodiment, in the recombinant Factor VIII protein of any of embodiments 1-3, two albumin binding domains are C-terminal to the heavy chain portion and one albumin binding domain is C-terminal to the light chain portion. In a ninth embodiment, in the recombinant Factor VIII protein of any of embodiments 1-3, three albumin binding domains are C-terminal to the heavy chain portion and one albumin binding domain is C-terminal to the light chain portion. In a tenth embodiment, in the recombinant Factor VIII protein of any of embodiments 1-3, four albumin binding domains are C-terminal to the heavy chain portion and one albumin binding domain is C-terminal to the light chain portion.

In an eleventh embodiment, in the recombinant Factor VIII protein of any of embodiments 1-3, at least two albumin binding domains are C-terminal to the heavy chain portion and at least two albumin binding domains are C-terminal to the light chain portion, preferably, two albumin binding domains are C-terminal to the heavy chain portion and two albumin binding domains are C-terminal to the light chain portion. In a twelfth embodiment, in the recombinant Factor VIII protein of embodiment 11, two albumin binding domains are C-terminal to the heavy chain portion and three albumin binding domains are C-terminal to the light chain portion. In a thirteenth embodiment, in the recombinant Factor VIII protein of embodiment 11, two albumin binding domains are C-terminal to the heavy chain portion and four albumin binding domains are C-terminal to the light chain portion. In a fourteenth embodiment, in the recombinant Factor VIII protein of embodiment 11, three albumin binding domains are C-terminal to the heavy chain portion and two albumin binding domains are C-terminal to the light chain portion. In a fifteenth embodiment, in the recombinant Factor VIII protein of embodiment 11, four albumin binding domains are C-terminal to the heavy chain portion and two albumin binding domains are C-terminal to the light chain portion.

In a sixteenth embodiment, in the recombinant Factor VIII protein of any of embodiments 1-15, albumin-binding domains are separated from the heavy chain portion and/or the light chain portion and/or other albumin-binding domains by a linker, wherein, preferably, albumin-binding domains are separated from the heavy chain portion and the light chain portion and other albumin-binding domains by a linker.

In a seventeenth embodiment, in the recombinant Factor VIII protein of embodiment 16, the linker comprises a Thrombin-cleavable linker section that optionally has the sequence of SEQ ID NO: 39.

In an eighteenth embodiment, in the recombinant Factor VIII protein of any of embodiments 16 or 17, the linker comprises a glycine-serine linker section that optionally has the sequence of SEQ ID NO: 40 or SEQ ID NO: 41.

In a nineteenth embodiment, in the recombinant Factor VIII protein of any of embodiments 16-18, said linker is a combination of different linker sections, e.g. the linker comprises a Thrombin-cleavable linker section flanked on each side by a glycine-serine linker section, wherein said linker optionally has the sequence of SEQ ID NO: 42 or SEQ ID NO: 43.

In a twentieth embodiment, in the recombinant Factor VIII protein of any of embodiments 1-19, the albumin binding domain comprises a sequence according to SEQ ID NO: 44.

In a twenty-first embodiment, in the recombinant Factor VIII protein of embodiment 20, the albumin binding domain comprises a sequence according to SEQ ID NO: 46.

In a twenty-second embodiment, in the recombinant Factor VIII protein of any of embodiments 1-21, the heavy chain portion comprises the domains A1 and A2, and optionally comprises the domains A1-a1-A2-a2 or A1-a1-A2-a2-B.

In a twenty-third embodiment, in the recombinant Factor VIII protein of any of embodiments 1-22, the light chain portion comprises the domains A3 and C1 and C2, and optionally comprises the domains a3-A3-C1-C2.

In a twenty-fourth embodiment, in the recombinant Factor VIII protein of any of embodiments 1-23, the B-domain of the Factor VIII protein is at least partly deleted.

In a twenty-fifth embodiment, the recombinant Factor VIII protein of any of embodiments 1-24 comprises, in a single chain, a heavy chain portion comprising an A1 and an A2 domain and a light chain portion comprising an A3, C1 and C2 domain of Factor VIII, wherein
a) in said recombinant Factor VIII protein, 894 amino acids corresponding to consecutive amino acids between F761 and P1659 of wild type Factor VIII as defined in SEQ ID NO: 1 are deleted, leading to a first deletion;
b) said recombinant Factor VIII protein comprises, spanning the site of the first deletion, a processing sequence comprising SEQ ID NO: 2 or a sequence having at most one amino acid substitution in SEQ ID NO: 2, wherein said processing sequence comprises a first thrombin cleavage site;
c) in said recombinant Factor VIII protein, at least the amino acids corresponding to amino acids R1664 to R1667 of wild type Factor VIII are deleted, leading to a second deletion; and
d) said recombinant Factor VIII protein comprises, C-terminal to the second deletion and N-terminal of the A3 domain, a second thrombin cleavage site.

In a twenty-sixth embodiment, the recombinant Factor VIII protein of any of embodiments 1-25 that optionally is a single chain protein comprises a heavy chain portion having at least 90% sequence identity to aa20-aa768 of SEQ ID NO: 16 and a light chain portion having at least 90% sequence identity to aa769-aa1445 of SEQ ID NO: 16, wherein said sequence identities preferably are at least 95%, at least 98% or 100%.

In a twenty-seventh embodiment, the recombinant Factor VIII protein of any of embodiments 1-26 that optionally is a single chain protein comprises a heavy chain portion having at least 90% sequence identity to aa20-aa1667 of SEQ ID NO: 1 and a light chain portion having at least 90% sequence identity to aa1668-aa2351 of SEQ ID NO: 1, wherein said sequence identities optionally are at least 95%, at least 98% or 100%.In a twenty-eigth embodiment, the recombinant Factor VIII protein of any of embodiments 1-4 and 16-27, comprises one albumin binding domain between the heavy chain portion and the light chain portion and one albumin binding domain C-terminal to the light chain portion,
wherein the sequence has at least 70% sequence identity to SEQ ID NO: 47.

In a twenty-ninth embodiment, the recombinant Factor VIII protein of any of embodiments 11-28 is a single chain protein comprising at least two albumin binding domains between the heavy chain portion and the light chain portion and at least two albumin binding domain C-terminal to the light chain portion, wherein the protein has at least 80% sequence identity, optionally, at least 90% sequence identity, at least 95% sequence identity or at least 98% sequence identity to any of SEQ ID NO: 48, 49 or 51. In a thirtieth embodiment, the recombinant Factor VIII protein of embodiment 29 has at least 80% sequence identity to SEQ ID NO: 48, e.g., it has SEQ ID NO: 48 or SEQ ID NO: 51.

In a thirty-first embodiment, the recombinant Factor VIII protein of any one of embodiments 1-30 has a "b mutation", i.e., a mutation of the amino acid corresponding to Y1699 to F at position 1699 and a mutation of the amino acid corresponding to Y1683 to F at position 1683 in wt Factor VIII protein of SEQ ID NO: 1.

In a thirty-second embodiment, the in vivo half-life of the recombinant Factor VIII protein of any one of embodiments 1-31 in a human subject is prolonged by a factor of at least 1.2, preferably, by a factor of at least 1.5, optionally, at least 2 or at least 2.5 in comparison to a recombinant Factor VIII protein of SEQ ID NO: 28.

In a thirty-third embodiment, the recombinant Factor VIII protein of any one of embodiments 1-32 is a fusion protein with at least one fusion partner selected from the group consisting of an Fc region, albumin, PAS polypeptides, HAP polypeptides, the C-terminal peptide of the beta subunit of chorionic gonadotropin, polyethylenglycol, and hydroxyethyl starch.

In a thirty-fourth embodiment, the recombinant Factor VIII protein of any one of embodiments 1-33 is a de-immunized protein.

In a thirty-fifth embodiment, the invention provides a nucleic acid encoding a recombinant Factor VIII protein of any one of embodiments 1-34. In a thirty-sixth embodiment, the nucleic acid of embodiment 35 is an expression vector, preferably, suitable for expression of said recombinant Factor VIII protein in a mammalian cell, preferably, in a human cell, such as a CAP cell.

In a thirty-seventh embodiment, the invention provides a host cell comprising a nucleic acid of any of embodiments 35 or 36. In a thirty-eighth embodiment, the host cell of embodiment 37 is a mammalian cell comprising an expression vector suitable for expression of said recombinant Factor VIII protein in said cell, preferably, a human cell selected from the group comprising a Hek293 cell or a CAP cell (e.g., CAP-T cell or CAP-Go cell).

In a thirty-ninth embodiment, the invention provides a method of preparing a recombinant Factor VIII protein, comprising culturing the host cell of embodiments 37 or 38 under conditions suitable for expression of the Factor VIII protein and isolating the recombinant Factor VIII protein, wherein the method optionally comprises formulating the Factor VIII protein as a pharmaceutical composition.

In a fortieth embodiment, the invention provides a pharmaceutical composition comprising the recombinant Factor VIII protein of any of embodiments 1-34, the nucleic acid of any of embodiments 35-36 or the host cell of any of embodiments 37-38. In a forty-first embodiment, the pharmaceutical composition of embodiment 40 further comprises a biologically acceptable carrier such as water or a buffer, optionally, at a physiologic pH, preferably, FVIII formulation buffer, and/or pharmaceutically acceptable excipients. In a forty-second embodiment, the pharmaceutical composition of any of embodiments 40 or 41 further comprises albumin, e.g., 0.1-5%, such as 1% human serum albumin.

In a forty-third embodiment, the invention provides a pharmaceutical composition of any of embodiments 40-42 or a kit further comprising an immunosuppressive agent, e.g., an immunosuppressive agent selected from the group comprising methylprednisolone, prednisolone, dexamethason, cyclophosphamide, rituximab, and/or cyclosporin.

The invention further provides, as a forty-fourth embodiment, a pharmaceutical composition of any of embodiments 40-43 for use in treatment of hemophilia A, wherein, optionally, the treatment is immune tolerance induction (ITI). In a forty-fifth embodiment, the pharmaceutical composition of any of embodiments 40-44 is for use in treating a patient with Hemophilia A selected from the group comprising a patient not previously treated with any Factor VIII protein, a patient previously treated with a Factor VIII protein, a patient who has an antibody response including an inhibitory antibody response to a Factor VIII protein, and a patient who has had an antibody response including an inhibitory antibody response to a Factor VIII protein who has been treated by ITI, or who has not been treated by ITI.

In a forty-sixth embodiment, the pharmaceutical composition of any of embodiments 40-45 is for administration every 5 to 14 days, preferably, every 7 to 10 days.

In a forty-seventh embodiment, the invention provides a vial, e.g., a prefilled or ready-to use syringe, comprising the pharmaceutical composition of any of embodiments 40-46.

In a forty-eighth embodiment, the invention provides a method of treatment, comprising administering an effective amount of the pharmaceutical composition of any of embodiments 40-46 to a patient in need thereof, e.g., a patient with hemophilia A, which may be selected from the patient groups defined herein.

| | |
|---|---|
| SEQ ID NO: 1 | wt human FVIII |
| SEQ ID NO: 2 | processing sequence in preferred single chain constructs |
| SEQ ID NO: 4 | processing sequence in V0 |
| SEQ ID NO: 5 | variant processing sequence, X can be varied for de-immunization |
| SEQ ID NO: 6 | variant processing sequence, X can be varied for de-immunization |
| SEQ ID NO: 7 | variant processing sequence, X can be varied for de-immunization |
| SEQ ID NO: 8 | variant processing sequence, X can be varied for de-immunization |
| SEQ ID NO: 9 | merging sequence, e.g., in V0 |
| SEQ ID NO: 16 | single chain FVIII V0 |
| SEQ ID NO: 28 | 6rs-REF |
| SEQ ID NO: 39 | Thrombin-cleavable linker |
| SEQ ID NO: 40 | glycine-serine linker G1, G in position 14 is present or absent |
| SEQ ID NO: 41 | glycine-serine linker G2 |
| SEQ ID NO: 42 | Thrombin-cleavable linker flanked on each side by a glycine-serine linker, strictly repetitive |
| SEQ ID NO: 43 | Thrombin-cleavable linker flanked on each side by a glycine-serine linker, non-repetitive |
| SEQ ID NO: 44 | ABD consensus sequence, see above |
| SEQ ID NO: 45 | ABD1 |
| SEQ ID NO: 46 | ABD2 |
| SEQ ID NO: 47 | ADLCLD_SC aa |
| SEQ ID NO: 48 | AD2CD2_SC aa |
| SEQ ID NO: 49 | AD2CD2woL_SC aa |
| SEQ ID NO: 50 | AD2CD2woLG_SC aa |
| SEQ ID NO: 51 | AbD2CD2_SC aa |
| SEQ ID NO: 52 | ADLCLD_SC na |
| SEQ ID NO: 53 | AD2CD2_SC na |
| SEQ ID NO: 54 | AD2CD2woL_SC na |
| SEQ ID NO: 55 | AD2CD2woLG_SC na |
| SEQ ID NO: 56 | AbD2CD2_SC na |
| SEQ ID NO: 57 | optimized DNA sequence encoding SEQ ID NO: 46 |
| SEQ ID NO: 58 | exemplary DNA encoding Glycine-serine linker G1 of SEQ ID NO: 40 |
| SEQ ID NO: 59 | exemplary DNA encoding Glycine-serine linker G2 of SEQ ID NO: 41 |

### Figure Legends

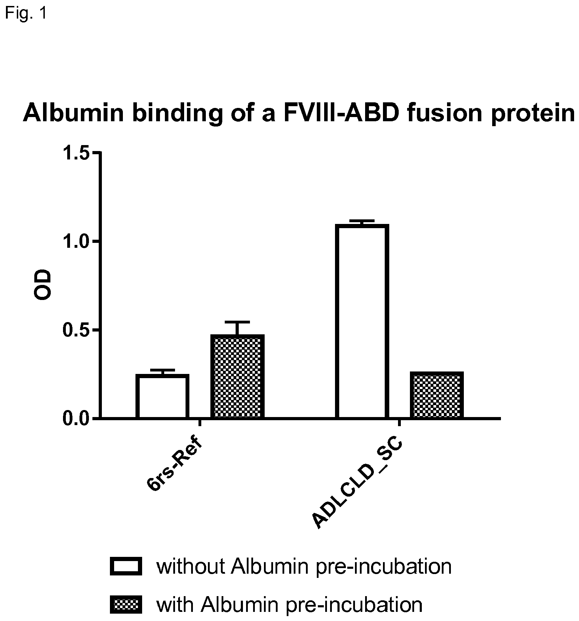

Fig. 1 shows the human albumin binding of ADLCLD_SC, a FVIII protein of the invention comprising two albumin-binding domains in comparison to FVIII 6rs-Ref, a protein having the ReFacto AF sequence. Both FVIII proteins were tested in the presents and absence of HSA via the albumin binding capacity assay as described.

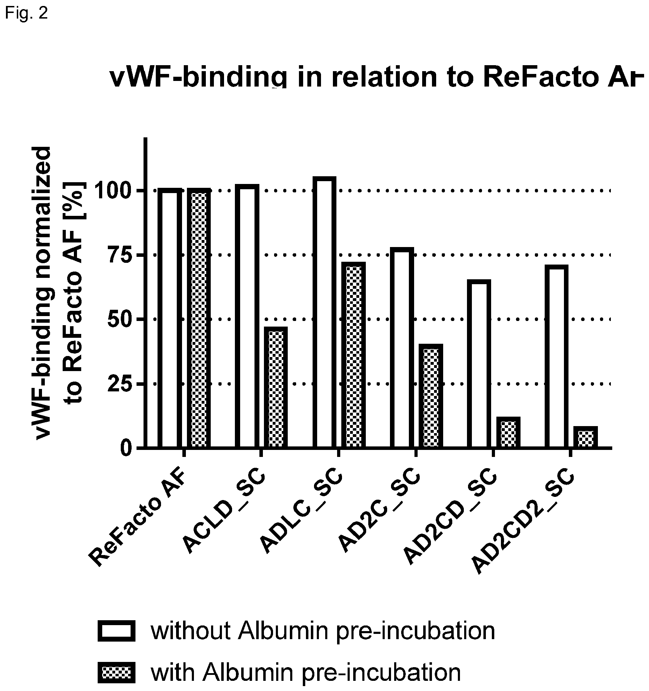

Fig. 2 shows the von-Willebrand factor (vWF) binding capacity of different FVIII-albumin-binding-domain fusion proteins in relation of ReFacto AF. All FVIII molecules were tested for their vWF binding in either the presence or absence of human albumin. The more albumin-binding domains were incorporated into FVIII, the lower was the binding to vWF in general. The presence of human albumin dramatically decreased the binding to vWF.

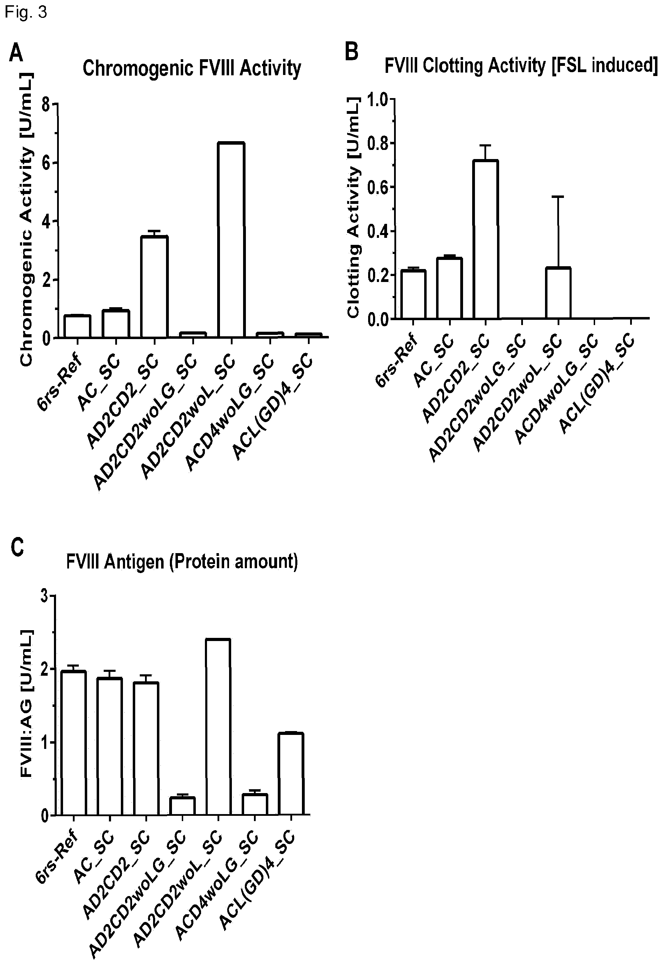

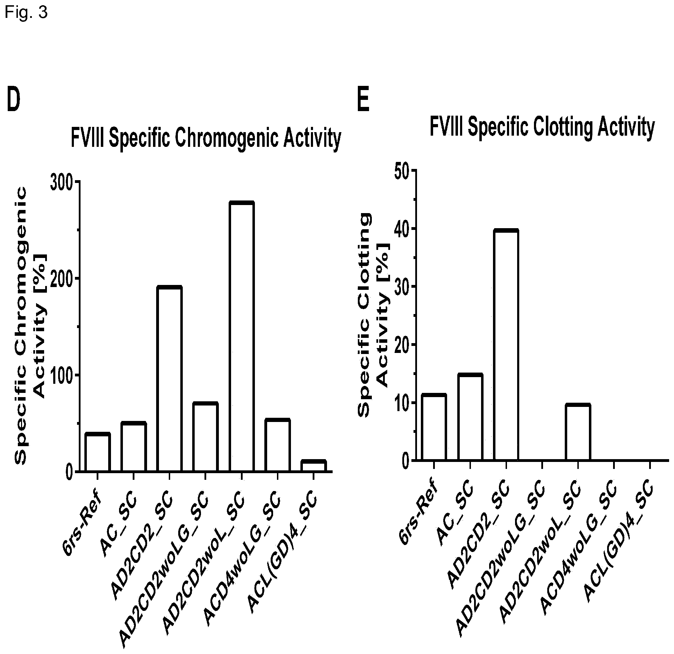

Fig. 3 Comparison of unpurified FVIII-ABD fusion variants and FVIII controls for their in vitro functionality. Cell culture supernatants of CAP-T cells expressing the double chain FVIII molecule 6rs-REF, the single chain FVIII molecule AC_SC, and the FVIII-ABD fusion molecules AD2CD2_SC, AD2CD2woLG_SC, AD2CD2wL_SC, ACD4woLG_SC, and ACL(GD)₄_SC were analyzed for chromogenic FVIII activity (A), FVIII clotting activity induced by Actin FSL (B) and FVIII antigen levels indicating total FVIII protein amount (C). Specific chromogenic activity was calculated as chromogenic FVIII activity to FVIII antigen ratio displayed in % (D). Specific clotting activity was calculated as FVIII clotting activity to FVIII antigen ratio displayed in % (E). n=2.

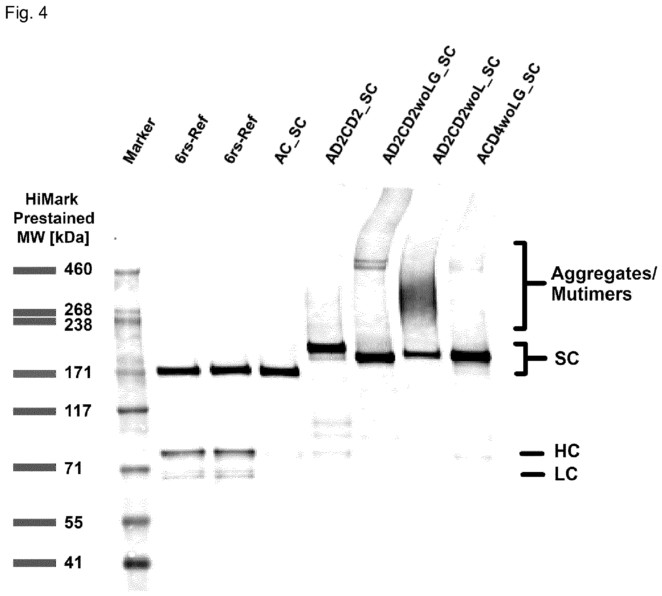

Fig. 4 Western blot analysis of unpurified FVIII-ABD fusion variants and FVIII control proteins for assessing structural properties. Cell culture supernatants of CAP-T cells expressing the double chain FVIII molecule 6rs-REF, the single chain FVIII molecule AC_SC, and the FVIII-ABD fusion molecules AD2CD2_SC, AD2CD2woLG_SC, AD2CD2wL_SC, and ACD4woLG_SC were separated by non-reduced sodium dodecyl sulfate polyacrylamide gel electrophoresis and subsequent blotting onto a PVDF membrane was performed. A purified Sheep anti-Human Factor VIII primary antibody and CF680-conjugated donkey anti-sheep IgG (H&L) antibody was used for detection. For size determination, Precision Plus All Blue was applied as marker.

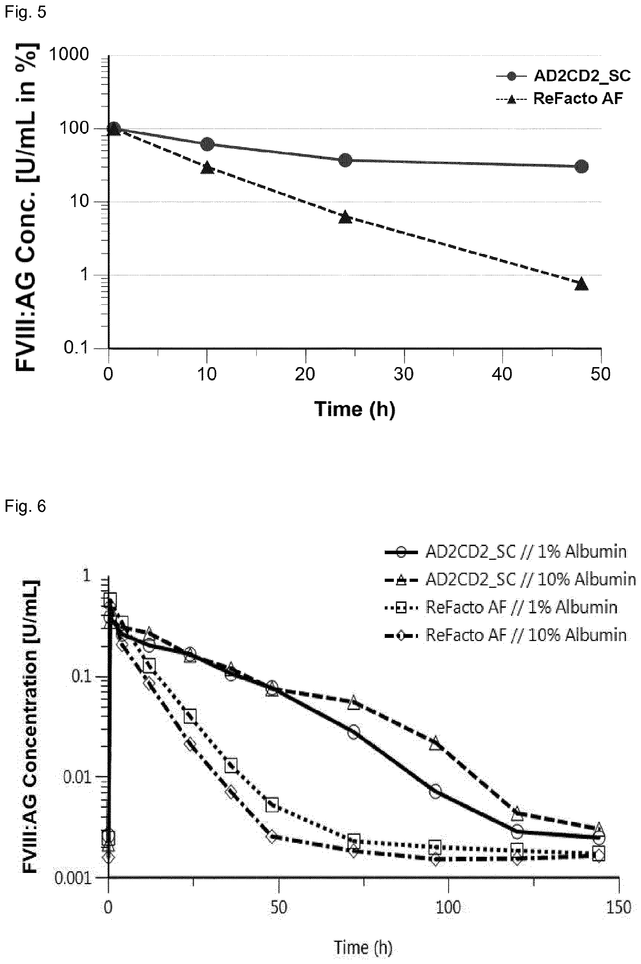

Fig. 5 demonstrates the in vivo pharmacokinetics of AD2CD2_SC compared to ReFacto AF after a single injection of 200 U/kg FVIII (with 1% human albumin) into mice having a knock-out for murine albumin and expressing the a-chain of human instead of murine neonatal Fc-receptor. Determined FVIII antigen values were normalized and are shown in percent over time.
Fig. 6 demonstrates the in vivo pharmacokinetics of AD2CD2_SC compared to ReFacto AF after a single injection of 30 U FVIII Antigen/kg formulated with either 1 or 10% human albumin into Göttingen minipigs. Plasma samples were withdrawn and FVIII antigen levels were measured by ELISA. Mean FVIII antigen levels are shown in U/ml over time in hours. n=3 minipigs per group.

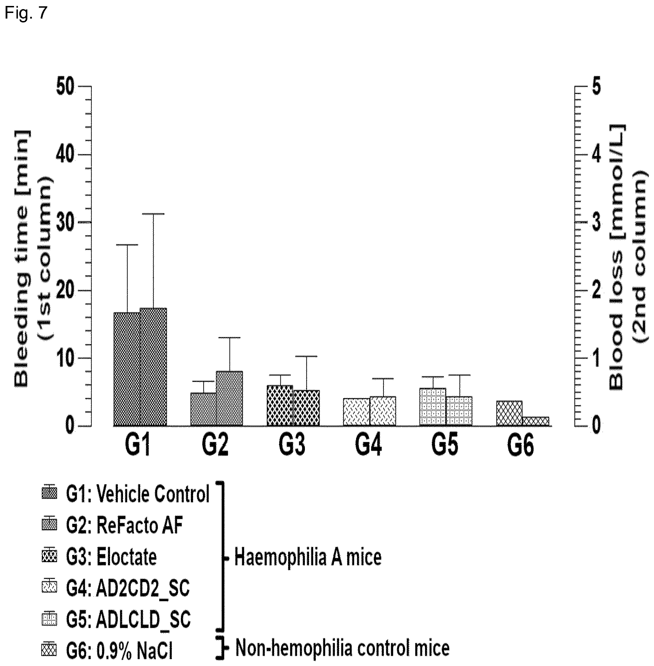

Fig. 7 shows the total bleeding time (first column of each group, left Y-axis) and the total blood loss (second column of each group, right Y-axis) after tail transection of hemophilia A mice which were administered 20 h earlier with either Vehicle Control, ReFacto AF, Eloctate, AD2CD2_SC or ADLCLD_SC. Non-hemophilia C57BL/6NCrl mice were treated with 0.9% NaCl and used as control.

### Examples

### 1. Evaluation of Factor VIII proteins with additions of albumin-binding domains for the development of a new hemophilia A therapeutic

### Material and methods

### Preparation of constructs

Experiments were performed to find and develop a suitable backbone for integration of the albumin-binding domains. The experiments were done on the basis of a B-domain deleted version of FVIII and single chain variants of FVIII. The basic double chain construct was a codon-optimized sequence of ReFacto AF® (Pfizer), wherein for simplifying cloning, 6 restriction sites were added through silent mutations, but some of these restriction sites were again excluded due to codon-optimization. The basic double chain sequence is 6rs-REF (SEQ ID NO: 28). The basic single chain construct used was V0 (SEQ ID NO: 16, EP19173440)

Firstly, the ABD protein sequence (Affibody AB, Solna, Sweden) was taken as a basis for design of the DNA sequence. If not mentioned otherwise, the ABD2 sequence was used. Moreover, codon optimized linkers were developed, which are partly cleavable by thrombin. If not otherwise stated, the glycine-serine linker was G1 and the thrombin-cleavable linker was L. Table 1 below demonstrates structures of fusion proteins with albumin-binding domains (ABD) for single chain molecules.

For the constructs encoding the FVIII of the invention and comparative constructs also analysed in this context, either the complete FVIII sequence or DNA regions carrying approx. 700-1200 bp from the FVIII a2 domain to the A3 domain were synthesized. The synthesized DNA was codon-optimized for the total target gene. The a2 to A3 DNA fragments were 5' terminally flanked by an EcoRV restrictions site, and 3' terminally flanked by an EcoRI restriction site, and these restriction sites were also present in the basic FVIII sequence used. For C-terminal fusion to the light chain, DNA fragments carrying approx. 1500-2100 bp were synthesized also in a codon-optimized form. Such DNA fragments were 5' terminally flanked by an EcoRI restrictions site within the A3 domain, and 3' terminally flanked by an Notl restriction site. Restriction of the DNA inserts and the FVIII backbone plasmid allowed for targeted ligation and generation of FVIII single chain plasmids. Completely synthesized FVIII DNA was 5' terminally flanked by a Hindlll restrictions site, and 3' terminally flanked by a Notl restriction site.

By transformation of *E.coli* K12 with said plasmids, expansion of transformed bacteria under ampicillin selection and plasmid preparation, large amounts of the plasmids could be prepared. Genetic engineering work was carried out by Thermo Fisher Scientific after design with VectorNTI Software (Thermo Fisher Scientific, Massachusetts, USA).

### Cultivation of CAP-T cells

For analyzing the candidates for new recombinant FVIII molecules, the constructs, integrated in expression vectors, were transiently and stably expressed in human cell lines. The preferred cell lines are Hek293 and CAP cells, both of which originate from human amniocytes. Because of higher yields of active FVIII molecules CAP cells, in particular, CAP-T cells were chosen as the preferred expression system for transient transfection and CAP-Go cells for stable expression.

Transient transfection was performed with nucleofection programs. The supernatants were screened for FVIII activity and antigen. Purification of the recombinant proteins from CAP cells was done, including FVIII affinity chromatography.

In detail, CAP-T cells (Cevec Pharmaceuticals, Köln, Germany) were cultured in PEM medium supplemented with 4 mM GlutaMAX (Thermo Fisher Scientific, 35050038) and 5 µg/ml blasticidin (Thermo Fisher Scientific, R21001; complete PEM medium). In order to thaw the cells, the required amount of frozen vials were transferred to a 37 °C water bath. After thawing, each vial was transferred to 10 ml of chilled, complete PEM medium. The cell suspension was centrifuged at 150 x g for 5 minutes. During this washing step the dimethyl sulfoxide (DMSO) used for cryopreservation was removed. The pellet was resuspended in 15 ml warm, complete PEM medium and transferred to a 125 ml shaker flask. The cells were incubated at 37 °C in a humidified incubator with an atmosphere containing 5 % CO₂. The flasks were set on a shaking platform, rotating at 185 rpm with an orbit of 50 mm. Subculturing of the cells was performed every 3 to 4 days. The fresh culture was set to 0.5x10⁶ cells/ml by transferring the required amount of cultured cell suspension to a new flask and adding complete PEM medium. In the case that the transferred cell suspension would exceed 20% of the total volume, the suspension was centrifuged at 150 x g for 5 minutes and the pellet was resuspended in fresh complete PEM medium. The volume of cell suspension per shaking flask was 20% of the total flask volume.

A minimum of three subcultures were performed after thawing before transfection experiments were performed.

### Protein expression in CAP-T cells by transient transfection

The CAP-T cells were transfected using the 4D-Nucleofector™ (Lonza, Basel, Switzerland). For each transfection 10x10⁶ CAP-T cells were centrifuged at 150 x g for 5 minutes in 15 ml conical tubes. The cells were resuspended in 95 µl supplemented SE Buffer, taking into account the volume of the pellet and the volume of the plasmid solution. Afterwards, 5 µg of the respective plasmid were added to the cell suspension followed by gentle mixing. The solution was transferred to 100 µl Nucleocuvettes. The used transfection program was ED-100. After the transfection, the cells from one Nucleocuvette were transferred to 125 ml shaker flasks, containing 12.5 ml complete PEM medium. The cells were cultivated for 4 days as described above. At day 4 the cells were harvested by centrifugation at 150 x g for 5 minutes. Larger protein amounts could be produced by combining 12.5 ml approaches as described above.

Supernatants were screened for FVIII activity and antigen directly after harvest.

The recombinant Factor VIII protein was further analyzed. FVIII activity was measured by chromogenic activity assay and clotting activity FSL assay. The antigen was estimated by FVIII antigen ELISA. As a further assay for biological activity, the cleavage of the recombinant proteins by thrombin was analyzed. Moreover, chain distribution and appearance was tested by Western Blots. Further, vWF-binding and albumin binding were tested.

### FVIII activity - chromogenic activity assay

The activity of FVIII was determined by a chromogenic assay. In this two-step assay, FIXa and FVIIIa activate FX in the first step. In the second step, the activated FX hydrolyses a chromogenic substrate, resulting in a color change, which can be measured at 405 nm. Due to the fact that calcium and phospholipids are present in optimal amounts and an excess of FIXa and FX is available, the activation rate of FX is only dependent on the amount of active FVIII in the sample.

The reagents for this chromogenic FVIII activity assay were taken from the Coatest® SP FVIII Kit. The kit contained phospholipids, calcium chloride (CaCl2), trace amounts of thrombin, the substrate S-2765, a mixture of FIXa and FX and the thrombin inhibitor I-2581. The inhibitor was added, in order to prevent hydrolysis of the substrate by thrombin, which was built during the reaction. All dilutions were performed in distilled water or Tris-BSA (TBSA) Buffer, containing 25 mM Tris, 150 mM sodium chloride (NaCl) and 1 % Bovine serum albumin (BSA), set to pH 7.4. Each sample was diluted at least 1:2 with FVIII-depleted plasma. Further dilutions were performed using the TBSA Buffer.

The assay was performed using the BCS XP (Siemens Healthcare, Erlangen, Germany), a fully automated hemostasis analyzer. All reagents including water, TBSA Buffer and the samples were inserted into the analyzer. For each sample the analyzer mixed 34 µl calcium chloride, 20 µl TBSA Buffer, 10 µl sample, 40 µl water, 11 µl phospholipids and 56 µl FIXa-FX-mixture. This mixture was incubated for 300 seconds. Afterwards, 50 µl of S-2765 + I-2581 were added to the reaction. Upon addition of the substrate, the absorption at 405 nm was measured for 200 seconds.

In order to calculate the amount of active FVIII, the software of the analyzer evaluated the slope of the measured kinetic between 30 seconds and 190 seconds after starting the reaction. This result was correlated to a calibration curve, generated with a biological reference preparation (BRP) of FVIII. The activity of the BRP is indicated in IU/ml. However, IU/ml can be assumed equivalent to U/ml. The results were indicated as "% of normal". These results were converted to U/ml, as 100 % of normal FVIII activity are equivalent to 1 U FVIII activity per ml.

### Clotting Activity FSL

In addition to the two-stage chromogenic assay (see above), a one-stage clotting assay was also performed in order to determine the amount of active FVIII. During this assay, FVIII-depleted plasma, CaCl₂, the activator Actin FSL and the FVIII-containing sample are mixed in one step. The activator leads to the generation of FXIa, which activates FIX. FVIIIa, FIXa and FX built the tenase complex and FX becomes activated. Further activation of prothrombin and fibrinogen finally leads to the formation of a fibrin clot. The time needed to form the clot, the activated partial thromboplastin time (aPTT), is measured. The aPTT varies, depending on the amount of FVIII.

The clotting assay was performed using the BCS XP. TBSA Buffer, FVIII-depleted plasma, Actin FSL, CaCl2 and the sample were inserted into the analyzer. The sample was diluted at least 1:2 with FVIII-depleted plasma. Further dilutions were performed using the TBSA Buffer. For each sample the analyzer mixed 45 µl TBSA Buffer, 5 µl sample, 50 µl FVIII-depleted plasma and 50 µl Actin FSL. The reaction was started by the addition of 50 µl CaCl₂. The analyzer measured the time needed for clot formation.

In order to calculate the amount of active FVIII, the software of the analyzer evaluated a baseline extinction at 405 nm at the beginning of the reaction. All of the following extinction values, within a time of 200 seconds, were analysed regarding their difference to the baseline extinction. The first time point exceeding a defined threshold was determined as the clotting time. This result was correlated to a calibration curve, generated with a BRP of FVIII.

### FVIII antigen ELISA

The amount of FVIII antigen was determined using the Asserachrom® VIII:Ag ELISA (Diagnostica Stago, Asnières sur Seine Cedex, France). In this sandwich ELISA, the applied FVIII is bound by mouse monoclonal anti-human FVIII F(ab')₂ fragments, which are coated to the plate by the manufacturer. The detection of the bound FVIII occurs via mouse monoclonal anti-human FVIII antibodies, which are coupled to a peroxidase. In the case that FVIII is present, the peroxidase-coupled antibody binds to FVIII and can be detected by the addition of a tetramethylbenzidine (TMB) solution. TMB turns from a clear to a blue-green solution upon reaction with peroxidase. After a short time, this reaction is stopped by the addition of sulfuric acid (H₂SO₄), which turns the solution yellow. The amount of bound FVIII correlates with the intensity of the yellow color, which can be measured at 450 nm. The final amounts of FVIII are calculated using a calibration curve generated by the measurement of at least five serial dilutions of a calibrator with a known antigen concentration.

The supplied calibrator and control were reconstituted with 500 µl of distilled water, 30 minutes before starting the ELISA. After this incubation time, the calibrator was diluted 1:10 in the supplied phosphate buffer. This represented the starting concentration. The calibrator was further serially diluted 1:2 up to a dilution of 1:64. As the concentration of the calibrator contained approximately 1 U/ml FVIII, depending on the batch, the starting concentration was equivalent to 0.1 U/ml FVIII whereas the last dilution contained approximately 0.0016 U/ml FVIII. The control was diluted 1:10 and 1:20 with the phosphate buffer. All samples were diluted with the phosphate buffer, depending on their previously determined activity (see above) with the aim to be in the middle of the calibration curve. After the dilution of FVIII samples, control and calibrator, 200 µl of each solution were applied per well in duplicates. In addition to that, two wells were filled with 200 µl of phosphate buffer as a blank control. The plate was incubated for 2 hours at room temperature covered with a film. During this time, the peroxidase-coupled anti-human FVIII antibodies were reconstituted with 8 ml phosphate buffer and incubated 30 minutes at room temperature. After the antigen immobilization, the wells were washed five times with the supplied washing solution, which was previously diluted 1:20 with distilled water. Immediately after the washing, 200 µl of the peroxidase-coupled anti-human FVIII antibodies were added to each well and incubated for 2 hours at room temperature covered by a film. Afterwards, the plate was washed five times as before. In order to reveal the amount of bound FVIII, 200 µl of TMB solution were added to each well and incubated for exactly 5 minutes at room temperature. This reaction was stopped by the addition of 50 µl 1 M H₂SO₄ to each well. After an incubation time of 15 minutes at room temperature, the absorbance of each well was measured at 450 nm using the POLARstar Omega plate reader (BMG LABTECH, Ortenberg, Germany).

The results of the ELISA were calculated using the MARS software (BMG Labtech). In a first step, all wells were blank corrected and the mean of the duplicates was calculated. Afterwards, a 4-parameter fit was applied, in order to calculate the concentrations from the calibration curve. According to this calibration curve the amount of FVIII antigen in each well was determined. In the last step, the values were corrected by the dilution factor, resulting in the FVIII antigen amount of each sample.

### Albumin binding capacity assay

20% human serum albumin (HSA) was diluted 1:4000 in PBS. 96-well ELISA plates were filled with 100 µl/well with diluted HSA solution and coated during a 2 h incubation at 37°C and 400 rpm on a thermoshaker. ELISA plates were washed 3-times with 300 µl/well washing buffer. Standard control and FVIII samples either with or without Albumin pre-incubation were diluted with Tris/NaCl pH 7.4 to a concentration of 0.5 U/ml chromogenic activity and 100 µl/well were added as 7-step 1:2 serial dilution. Incubation was performed for 1 h at 37°C covered on a thermoshaker. In the meantime, FIXa and FX were resolved together in 10 ml aqua dest., substrate (S-2765 and I-2581) was solved in 12 ml aqua dest.. After FVIII incubation, plates were washed again 3 times with 300 µl/well washing buffer. Phospholipides and the FIXa/FX solution were mixed 1:5 and subsequently 50 µl/well of this solution were added and incubated for 5 min at 37°C. Without any washing step 25 µl CaCl₂ was added to each well, followed by 5 min incubation at 37°C. Finally, 50 µl/well substrate were added and detection of activated FX-mediated substrate turnover was performed at 405 nm for 25 cycles followed by end point measurement using an ELISA reader.

### vWF binding capacity assay

1 U/ml of each FVIII molecule was either pre-incubated or not with 40 mg/ml albumin for 30 min at RT to promote ABD-albumin binding and an assay for determining the vWF binding capacity was performed as follows:
Plasma purified vWF (Biotest AG) was diluted with 0.9% NaCl solution to a concentration of 0.1 U/ml. Coating onto 96-well ELISA plates was done by transferring 100 µl of this solution to each well followed by an 2 h incubation at 37°C and 400 rpm. The wells were washed 3 times with 300 µl of washing buffer (8 mM sodium phosphate, 2 mM potassium phosphate, 0.14M NaCl, 10 mM KCI, 0.05% Tween-20, pH 7.4). FVIII standard (commercial rFVIII without vWF) and samples were pre-diluted with dilution buffer (25 mM Tris, 150 mM NaCl, pH 7.4) to a concentration of 0.25 U/ml according to chromogenic activity and transferred as a 7-step, serial 1:2 dilution into each plate well (100 µl/well). Incubation was carried out for 1 h at 37°C and 400 rpm. In the meantime, FIXa and FX were resolved together in 10 ml aqua dest., substrate (S-2765 and I-2581) was solved in 12 ml aqua dest.. After FVIII incubation, plates were washed again 3 times with 300 µl/well washing buffer. Phospholipides and the FIXa/FX solution were mixed 1:5 and subsequently 50 µl/well of this solution were added and incubated for 5 min at 37°C. Without any washing step 25 µl CaCl₂ was added to each well, followed by 5 min incubation at 37°C. Finally, 50 µl/well substrate were added and detection of activated FX-mediated substrate turnover was performed at 405 nm for 25 cycles followed by end point measurement using an ELISA reader.

### Western Blot

### Reducing sodium dodecyl sulfate polyacrylamide gel electrophoresis (SDS-PAGE)

Cell supernatants, cell lysates or purified material of FVIII variants were appropriately diluted with 1x NuPAGE LDS Sample Buffer (4x, Thermo Fisher Scientific, NP0007) and further diluted 1:2 with reducing sample buffer. Reducing sample buffer was produced by combining 2.5 parts of NuPAGE LDS Sample Buffer with 1 part of NuPAGE Sample Reducing Agent (10x, Thermo Fisher Scientific, NP0004). 20 µl of each sample were mixed with 20 µl of reducing sample buffer in a 1.5 ml vial and heated for 10 min at 70°C using a thermoshaker (Eppendorf). A NuPAGE 4-12% Bis-Tris Protein Gel (Thermo Fisher Scientific) was inserted into the XCell SureLock Mini-Cell Electrophoresis System (Thermo Fisher Scientific) and inner and outer chambers were filled with 1x NuPAGE MOPS SDS Running buffer (Thermo Fisher Scientific, NP0001). 500 µl of NuPAGE Antioxidant (Thermo Fisher Scientific) was added to the inner chamber. 10 µl of the each prepared sample and 4 µl of Precision Plus Protein All Blue Standard (Bio-Rad, 161-0373) diluted 1/10 in 1x LDS Sample Buffer were loaded onto the gel. The sample separation was achieved by running the gel at a constant voltage of 200 V for 50-60 min.

### Non-reducing SDS-PAGE

20 µl of cell supernatants, cell lysates or purified material of FVIII variants were, either pre-diluted or not, appropriately diluted with 10 µl of NuPAGE LDS Sample Buffer (4x, Thermo Fisher Scientific, NP0007) and 10 µl aqua dest.. Samples were heated for 10 min at 70°C using a thermoshaker (Eppendorf). A NuPAGE™ 3-8% Tris-Acetate Protein Gel (Thermo Fisher Scientific) was inserted into the XCell SureLock Mini-Cell Electrophoresis System (Thermo Fisher Scientific) and inner and outer chambers were filled with 1x NuPAGE™ Tris-Acetate SDS Running Buffer (Thermo Fisher Scientific, LA0041). 10 µl of the each prepared sample and 4 µl of Precision Plus Protein All Blue Standard (Bio-Rad, 161-0373) diluted 1/10 in 1x LDS Sample Buffer were loaded onto the gel. The sample separation was achieved by running the gel at a constant voltage of 150 V for 55-70 min.

### Western Blotting and Detection

To investigate the separated proteins by immunofluorescence detection, they were transferred onto an Odyssey nitrocellulose membrane (Li-Cor) or an Amersham Hybond Low Fluorescence 0.2 µm polyvinylidene fluoride (PVDF) membrane (GE Healthcare Life Sciences) by using the XCell II blot module (Thermo Fisher Scientific) for semi-wet protein transfer. The PVDF membrane was activated in methanol and then applied to the SDS gel, whereas the nitrocellulose membrane was directly applied to the SDS gel. The system was filled with NuPAGE transfer buffer (20X, Thermo Fisher Scientfic) according to the manufacturer's instructions. Protein blotting was performed for 1 h at 30 V. After protein transfer, the membrane was blocked over night at 4°C in Odyssey blocking buffer (Li-Cor). Afterwards the membrane was incubated for 1 h at room temperature simultaneously with either a rabbit anti-coagulation factor VIII monoclonal antibody (Sino Biological, 13909-R226, 1:1000) and a mouse anti-human factor VIII monoclonal antibody (Merck, MAB038, 1:2500) or with 0.0004 µg/µl sheep anti-human factor VIII:C polyclonal antibody (Cedarlane, CL20035AP, 1.5000), each diluted in Odyssey Blocking buffer containing 0.05% Tween 20. After incubation, the membrane was washed 4-times for 5 min in 0.1% PBST. For detection of FVIII heavy and light chain the membrane was incubated with 0.067 µg/ml IRDye 800CW donkey anti-mouse (Li-Cor, 926-32212, 1:15000) and 0.067 µg/ml IRDye 680RD donkey anti-rabbit (Li-Cor, 926-68073, 1:15000) diluted in Odyssey blocking buffer containing 0.05% Tween 20 for 1 h at room temperature. Alternatively, the CF680 donkey anti-sheep IgG (H&L) antibody (Biotium, 20062-1) was used in a 1:5000 dilution in Odyssey blocking buffer for binding the respective primary antibody. Finally, the membrane was washed 4 times for 5 min in 0.1% PBST, 2 times for 5 min in PBS and rinsed in water. The membrane was visualized using the Licor Odyssey Imager.

### Pharmacodynamic studies

Coagulation factors were administered by a single intravenous tail vein injection into female haemophilia A mice with doses of 200 U/kg body weight or respective amounts of a control solution. After 4 or 20 h post dosing, tail vein transection bleeding assay was performed as follows: The animals were anaesthetised with 5% isoflurane in 30% O2 and 70% N2O, and immediately placed in prone position on a heating pad at +37°C. Tail vein transection was performed as described by Johansen et al., 2016. Haemophilia 22(4):625-631.

Bleeding was monitored for 60 min and bleeding time was determined using a stop clock. Primary bleeding time was noted until first bleeding cessation. After the primary bleeding, the tail was put into a new centrifuge tube filled with pre-warmed saline. If the mouse was not bleeding at 15, 30 and 45 min post injury, the tail was lifted out of the saline and the wound was challenged by gently wiping it twice with a saline wetted gauze swab in the distal direction. Immediately after the challenge, the tail was re-submerged into the saline. The cumulative bleeding time of all following bleeds constitute the secondary bleeding time. The total bleeding time is defined as the sum of the primary and all secondary bleeding times.

Following the determination of bleeding time, the tubes were centrifuged at 4140 g at room temperature for 3 minutes. Apart from 1 mL, the supernatant was removed. The cell pellet was resuspended and hemoglobin content was determined by using a method similar to that described by Elm et al. (2012).

### Results and Discussion

Generation and prescreening of several different FVIII-ABD fusion proteins covering FVIII double chain and single chain constructs was very promising in initial experiments and similar in both single chain and double chain backbones.

The formation of single chain FVIII molecule was increased compared to double chain forms when ABD was fused in between heavy chain and light chain (data not shown).

The FVIII proteins further developed and produced shown herein are listed in Table 1.

**Table 1 Structure of relevant variants of FVIII with ABD fusion. A= FVIII A1+a1+A2+a2+truncated B domain, C= FVIII a3 (optionally truncated) +A3+C1+C2 domains, L= Thrombin cleavable linker, G= flexible glycine-serine linker 1 (G1), D= ABD2,**

| **Construct name** | **Structure** | **Protein of the invention?** |
|---|---|---|
| AD2CD2_SC | ALDGLGDLCLDGLGD_SC | Yes |
| ADLCLD_SC | ADLCLD_SC | Yes |
| AbD2CD2_SC | ALDGLGDLCLDGLGD_SC + Y1699F + Y1683F | Yes |
| AD2CD_SC | ALDGLGDLCLD_SC | Yes |
| ACLD_SC | ACLD_SC | No |
| ADLC_SC | ADLC_SC | No |
| AD2C_SC | AD2C_SC | No |
| ACLDGLGD_SC | ACLDGLGD_SC | No |
| AD2CD2woLG_SC | ADDCDD_SC | Yes |
| AD2CD2woL_SC | AGDGDGCGDGD_SC | Yes |
| ACD4woLG_SC | ACDDDD_SC | No |
| ACL(GD)4_SC | ACLGDGDGDGD_SC | No |

Six single chain FVIII-ABD fusion molecules were generated *in silico* and respective DNA constructs were tested for their expression in either HEK293 or CAP-T cells (cf. Table 3). As all of those FVIII-ABD variants were expressed, secreted and functional, based on results of the chromogenic FVIII activity measurement, all molecules were produced in midi-scale CAP-T cell culture and successfully purified in larger amounts as needed for further characterizations and PK (pharmacokinetic) analysis.

**Table 3 FVIII-ABD fusion proteins analyzed in supernatants of transfected HEK293 or CAP-T cells. (n/a: not available)**

| **Construct** | **Chromogenic activity [U/mL]** | **Clotting activity [U/mL]** | **FVIII:Ag [U/mL]** | **Specific chromogenic activity [%]** | **Expression Cell line** |
|---|---|---|---|---|---|
| ADLC_SC | 0,98 | 0,9 | 0,47 | 209 | HEK293 |
| ACLD_SC | 1,26 | 0,79 | 0,72 | 175 | HEK293 |
| ADLCLD_SC | 1 | 0,45 | n/a | n/a | HEK293 |
| AD2CD2_SC | 1,45 | 0,35 | 0,71 | 204 | CAP-T |
| AD2CD_SC | 1,3 | 0,5 | 0,48 | 271 | CAP-T |
| AD2C_SC | 2,0 | 1,0 | n/a | n/a | CAP-T |
| ADLC_SC | 0,9 | n/a | n/a | n/a | CAP-T |
| ADLCLD_SC | 1,9 | n/a | n/a | n/a | CAP-T |

All six purified FVIII-ABD fusion variants were extensively characterized by several methods including determination of FVIII antigen and chromogenic activity, Actin FSL clotting, heavy and light chain detection by western blotting (WB), thrombin-cleavage analysis and binding to vWF and albumin. Table 4 gives an overview of produced FVIII-ABD variants in terms of chromogenic and clotting activity as well as antigen levels in the final solutions. Measurement of these values indicated that FVIII-ABD fusion proteins are still capable of their biological function: Bridging factor IXa and factor X leading thereby to the activation of the latter one. Comparison of the specific chromogenic activity (chromogenic activity / antigen *100) demonstrates that ADLC_SC and ReFacto AF® are similar (109% vs 104%). However, the specific chromogenic activities of all other FVIII-ABDs are much better, ranging from 130% to 206%.

Interestingly, the results indicate that increasing numbers of ABD motifs within one FVIII molecule decrease the clotting activity and also the capability of vWF binding. The decrease in clotting activity may be caused by the setup of the assay, which is strictly time-dependent. This may not mirror *in vivo* clotting activity.

**Table 4 Measurements of chromogenic FVIII activity, FSL clotting activity, and antigen levels. The indicated specific activity was calculated by the ratio of chromogenic activity and antigen.**

| **FVIII-ABD variant** | **chromogenic activity [U/ml]** | **FSL clotting [U/ml]** | **Antigen [U/ml]** | **Specific activity [%]** |
|---|---|---|---|---|
| ADLC_SC | 147 | 101 | 135 | 109 |
| ACLD_SC | 150 | 100 | 101 | 149 |
| ADLCLD_SC | 128 | 80 | 62 | 206 |
| AD2CD_SC | 120 | 61 | 92 | 130 |
| AD2CD2_SC | 164 | 42 | 96 | 171 |
| AD2C_SC | 112 | 72 | 78 | 144 |
| ReFacto AF® (Control) | 253 | 242 | 243 | 104 |

Western blot was used to observe the heavy and light chain patterns of the different FVIII-ABD fusion molecules in comparison to ReFacto AF® (data not shown). The analysis demonstrated that, in contrast to ReFacto AF®, the FVIII-ABD variants were mostly expressed as single chain molecules.

Activation of FVIII-ABD variants was investigated by direct incubation with thrombin at 37°C for 8 min and subsequent provision for reducing SDS-PAGE followed by western blotting. Band patterns of thrombin-activated or untreated FVIII-ABD molecules show that all FVIII-ABD molecules were activated by Thrombin in a comparable manner as ReFacto AF® (data not shown).

Albumin binding of the ADLCLD_SC variant was tested by an assay in comparison to FVIII 6rs-Ref, demonstrating the capability of albumin binding (Fig 1). An excess of unbound soluble albumin inhibited the binding to plate-bound albumin.

The influence of ABD and linker modifications on the binding between FVIII and vWF were investigated in two settings: 1. directly, without the presence of albumin; 2. after a 30 min pre-incubation with physiological concentrations of albumin promoting the ABD-albumin binding. As demonstrated in Fig. 2, the vWF binding of FVIII-ABD fusion proteins directly decreases by an increasing number of ABD motifs. However, only one ABD motif per FVIII does not have an influence on the vWF binding in the absence of albumin, independent from its position within the molecule. When FVIII-ABDs were pre-incubated with albumin, a decreased vWF binding was observed for all FVIII-ABD variants. This reduction of vWF binding was higher the more ABD domains were incorporated into FVIII.

To investigate the impact of linkers on the production and functionality of FVIII-ABD variants, one preferred variant, AD2CD2_SC, was also produced (I) without any linkers between FVIII and ABD-Domains (AD2CD2woLG_SC) and (II) with G1 linkers but without thrombin-cleavable L linkers (AD2CD2woL_SC). These variants were compared to the double chain FVIII 6rs-Ref (ReFacto amino acid sequence), single chain FVIII backbone AC_SC and two FVIII-ABD variants having four C-terminal ABD domains either without any linkers (ACD4woLG_SC) or with one thrombin-cleavable linker followed by four ABD domains separated by G1 linkers (ACL(GD)4_SC).

Respective plasmids encoding the different FVIII variants were nucleofected into CAP-T cells and 4-day cell culture supernatants were tested for chromogenic FVIII activity, FVIII clotting activity and FVIII antigen levels according to the above-described methods. As shown in Fig. 3, AD2CD2woLG_SC, ACD4woLG_SC, and ACL(GD)4_SC were expressed in only low amounts and chromogenic activity was strongly decreased. AD2CD2woLG_SC was not expressed in high amounts, but had some specific chromogenic activity. No FVIII clotting activity could be detected for any of these variants. In comparison to all other controls, AD2CD2_SC and AD2CD2woL_SC demonstrated good FVIII antigen levels and great FVIII chromogenic and clotting activities, resulting in superior specific chromogenic activity values of approx. 200% or higher. AD2CD2_SC demonstrate an especially high specific clotting activity.

A western blot analysis based on a non-reduced SDS-PAGE separation of these variants is demonstrated in Fig. 4. Except for FVIII 6rs-Ref, all other variants are mainly present as single chain FVIII molecules. However, AD2CD2woLG_SC and AD2CD2woL_SC tend to form multimers or aggregates, which are not observed for the AD2CD2_SC variant.

### 3. Pharmacokinetic experiments

Purification of FVIII ABD variants was performed for in vivo experiments, based on supernatants of transfected CAP-T cells by strong anion exchange chromatography and affinity chromatorgraphy.

To investigate the half-life prolongation effect of ABD motifs introduced into the FVIII molecules, two pharmacokinetic (PK) studies were performed in hemophilia A mice. 12 mice per test item were used, 2 or 3 for each time point. All FVIII-ABD molecules were administered in a single dose of 200 U/kg body weight (6 ml/kg) into the tail vein by a single intravenous tail vein injection into female haemophilia A mice (B6, 129S4-F8<tm1Kaz>/J). Plasma samples taken 0.5, 4, 8, 12, and 20h (and 24h) post injection were analyzed regarding FVIII chromogenic activity and antigen levels in citrate plasma which was subsequently extracted by centrifugation. Plasma samples were stored at -80°C and analyzed for FVIII antigen and chromogenic activity. ReFacto AF® was tested as control beside the FVIII-ABD variants.

Results are shown in Table 5.

**Table 5 Calculated t1/2 of FVIII-ABD variants. *AD2C_SC data were highly variable. t_{1/2} is specified in hours**

| FVIII-ABD variant | Antigen: t_{1/2} | Chromogenic activity: t_{1/2} |
|---|---|---|
| Study 1 | | |
| ACLD_ SC | 4.8 | 4.3 |
| AD2CD_SC | 4.3 | 5.0 |
| AD2CD2_SC | 14.5 | 9.4 |
| ReFacto® (control) | 5.7 | 4.1 |

| Study 2 | | |
|---|---|---|
| ADLC_SC | 7.8 | 6.8 |
| ADLCLD_SC | 10.4 | 10.5 |
| AD2C_SC* | 13.0 | 6.1 |
| ReFacto® (control) | 7.0 | 6.8 |

Thus, by pharmacokinetic studies in hemophilia A mice, preferred FVIII proteins of the invention were identified which show a half-life prolonged up to 2.5x (e.g., ADLCLD_SC - about 1.5x; AD2CD2_SC - about 2.5x). Pharmacokinetics of AbD2CD2_SC were tested in a separate study and were similar to AD2CD2_SC.

It is noted that the hemophilia A mouse model may even underestimate half-life extension due to the discrepancy of murine and human albumin (murine albumin only has a half-life of about two days). Nevertheless, the observed relative extended half-life of the FVIII proteins of the invention already allows a potential reduction of intravenous FVIII injection in hemophilia patients from 2 - 3 days to a once weekly dosing.

Moreover, a pharmacokinetic proof of concept study in albumin-deficient Tg32 mice having a knock-out of murine albumin and expressing human FcRn a-chain instead of the murine one (B6.Cg-*Alb^{em12Mvw} Fcgrt^{tm1Dcr}* Tg(FCGRT)32Dcr/MvwJ) was performed. This mouse model (Alb⁻/mFcRn⁻/hFcRn⁺), compared to hemophilia A mice, reveals a situation closer to humans as injected human albumin has a half-life of approx. 20 days which is similar to the half-life in humans. Intraveneous FVIII injection (AD2CD2_SC; ReFacto AF®) was done with 200 U/kg (based on chromogenic activity) plus 1% human albumin.

The results, shown in Fig. 5 demonstrated a half-life extension of AD2CD2_SC in comparison to ReFacto AF® of about 4x, allowing a potential reduction in patients of i.v. FVIII injection from 2 - 3 days to a 8 - 12 days dosing.

In addition, a pharmacokinetic study was performed in Göttingen Minipigs. Three animals per group were injected with 30 U FVIII antigen/kg body weight with either (I) ReFacto AF + 1% human serum Albumin (HSA), (II) ReFacto AF + 10% HSA, (III) AD2CD2_SC + 1% HSA or (IV) AD2CD2_SC + 10% HSA via the ear vein. Blood samples were taken predose, 4, 12, 36, 48, and 120 h post administration and citrate plasma was isolated immediately by centrifugation. Bioanalytical sample measurement was performed by FVIII antigen ELISA, which is specific for human FVIII and does not detect any porcine FVIII. Evaluation by non-compartment analysis (Figure 6) obtained half-lives for (I) ReFacto AF + 1% HSA of 7.1 h, (II) ReFacto AF + 10% HSA of 6.4 h, (III) AD2CD2_SC + 1% HSA of 18.6 h, and (IV) AD2CD2_SC + 10% HSA of 20.7 h. Thus, an half-life extension of approx. 3-fold was observed for AD2CD2_SC compared to ReFacto AF in this model.

Moreover, pharmacodynamics studies have been performed. Hemophilia A mice (Jackson No. B6; 129S4-F8<tm1 Kaz>/J) and control mice (Jackson No. C57BL/6NCrl) were intravenously injected with 200 U/kg (based on chromogenic FVIII activity) of each FVIII variant (ReFacto®, Eloctate®, AD2CD2_SC, ADLCLD_SC) or control solutions (Vehicle Control, 0.9% NaCl) and weight loss through bleeding, bleeding time and Hb amount by OD550 have been analyzed. Additional plasma sampling (0.5 h p.a. by retro-orbital withdraw, after experiment) have been done for analysis of FVIII activity. As shown in Fig. 7, all FVIII proteins of the invention diminish total bleeding time and blood loss similar to that of control mice indicating in vivo functionality of AD2CD2_SC and ADLCLD_SC.

## Claims

1. A recombinant Factor VIII protein comprising a heavy chain portion and a light chain portion of Factor VIII and at least two albumin binding domains, wherein at least one albumin binding domain is C-terminal to the heavy chain portion and at least one albumin binding domain is C-terminal to the light chain portion,
wherein, if the protein is a single chain protein, the albumin binding domain(s) C-terminal to the heavy chain portion is/are N-terminal to the light chain portion.

2. The recombinant Factor VIII protein of claim 1 that is a single chain protein.

3. The recombinant Factor VIII protein of any one of the preceding claims, wherein two albumin binding domains are C-terminal to the heavy chain portion and two albumin binding domains are C-terminal to the light chain portion.

4. The recombinant Factor VIII protein of any one of the preceding claims, wherein albumin-binding domains are separated from the heavy chain portion and/or the light chain portion and/or other albumin-binding domains by a linker.

5. The recombinant Factor VIII protein of claim 4, wherein the linker comprises a Thrombin-cleavable linker that optionally has the sequence of SEQ ID NO: 39.

6. The recombinant Factor VIII protein of any one claims 4 or 5, wherein the linker comprises a glycine-serine linker that optionally has the sequence of SEQ ID NO: 40 or SEQ ID NO: 41.

7. The recombinant Factor VIII protein of any one claims 4 to 6, wherein said linker comprises a Thrombin-cleavable linker flanked on each side by a glycine-serine linker, wherein said linker optionally has the sequence of SEQ ID NO: 42 or SEQ ID NO: 43.

8. The recombinant Factor VIII protein of any one of the preceding claims, wherein the albumin binding domain comprises a sequence according to SEQ ID NO: 44, wherein preferably the sequence is SEQ ID NO: 46.

9. The recombinant Factor VIII protein of any one of the preceding claims that optionally is a single chain protein, wherein said protein comprises a heavy chain portion having at least 90% sequence identity to aa20-aa768 of SEQ ID NO: 16 and a light chain portion having at least 90% sequence identity to aa769-aa1445 of SEQ ID NO: 16.

10. The recombinant Factor VIII protein of any one of the preceding claims that is a single chain protein comprising at least two albumin binding domains between the heavy chain portion and the light chain portion and at least two albumin binding domain C-terminal to the light chain portion, wherein the protein has at least 80% sequence identity to any of SEQ ID NO: 48, 49 or 51,
wherein the protein preferably has at least 80% sequence identity to SEQ ID NO: 48.

11. The recombinant Factor VIII protein of any one of the preceding claims, wherein the protein is a de-immunized protein.

12. A nucleic acid encoding a recombinant Factor VIII protein of any one of the preceding claims, wherein said nucleic acid preferably is an expression vector suitable for expression of said recombinant Factor VIII protein in a mammalian cell, optionally, a human cell, such as a CAP cell.

13. A host cell comprising a nucleic acid of claim 12, wherein preferably the host cell is a mammalian cell comprising an expression vector suitable for expression of said recombinant Factor VIII protein in said cell.

14. A method of preparing a recombinant Factor VIII protein, comprising culturing the host cell of claim 13 under conditions suitable for expression of the Factor VIII protein and isolating the recombinant Factor VIII protein, wherein the method optionally comprises formulating the Factor VIII protein as a pharmaceutical composition.

15. A pharmaceutical composition comprising the recombinant Factor VIII protein of any of claims 1-11, the nucleic acid of claim 12 or the host cell of claim 13, optionally further comprising a biologically acceptable carrier and/or albumin,
wherein the pharmaceutical composition preferably is for use in treatment of hemophilia A.
